# EUROPEAN PATENT SPECIFICATION

(11) **EP 3 199 190 B1**
(45) Date of publication and mention of the grant of the patent: **04.12.2019**
(21) Application number: 16200040.0
(22) Date of filing: 30.09.2014
(51) Int. Cl.: A61M 5/32, A61M 5/142, A61M 5/20, A61M 5/24, A61M 5/315

(54) **STABILIZED PEN INJECTOR**
STABILISIERTER STIFTFÖRMIGER INJEKTOR
STYLO INJECTEUR STABILISÉ

(30) Priority: 30.09.2013 US 201361884597 P; 04.12.2013 US 201314096977; 21.02.2014 US 201414186403; 30.06.2014 US 201462019066 P
(43) Date of publication of application: 02.08.2017
(62) Divisional of application: 14789668.2
(73) Proprietor: Medimop Medical Projects Ltd., 43665 Ra'anana (IL)
(72) Inventor: CABIRI, Oz, 71799 Macabim-Reut (IL); HEZKIAHU, Ran, 4668346 Herzliya (IL)
(74) Representative: Lippert Stachow Patentanwälte Rechtsanwälte

(56) References cited:
- EP-A1- 2 468 340
- EP-A1- 2 468 342
- US-A1- 2003 229 308

## Description

### BACKGROUND OF THE INVENTION

The present invention, in some embodiments thereof, relates to a medical injector and, more particularly, but not exclusively, to an injector stabilized for an extended bolus delivery.

International Patent Application Publication No. WO/2013/104414 to SANOFI-AVENTIS DEUTSCHLAND GMBH Describes a guiding assembly for an injection device comprising a mount adapted to receive an injection device, a first gripping member rotatably coupled to the mount, a first lateral stop member coupled to the first gripping member, and a spring biasing the first gripping member in a first angular position.

U.S. Patent Application Publication No. 2011/0166509 to Gross and Cabiri discloses an apparatus for use with tissue of a subject, including a substance configured to be injected into the tissue, and first and second tissue-squeezing surfaces configured to be placed on first and second sides of the tissue, to exert pressure on the tissue by being moved toward each other in response to a squeezing force (F), and to facilitate injection of the substance into the tissue by releasing the substance in response to application of the squeezing force.

U.S. Patent Application Publication No. 2012/0130344 to Ebbett discloses a skin gripping means for use with an injector. In one embodiment the skin gripping means is a needle guard. An exterior surface of the skin gripping means is provided with a plurality of fingers adapted to engage a subject's skin when in use. A method of performing a subcutaneous injection is also disclosed with includes the steps of bringing a skin gripping means of an injector into contact with the skin of a subject, moving the skin gripping means substantially parallel to the skin to thereby form a fold in the skin, moving a needle of the injector into the fold to a suitable position for a subcutaneous injection and injecting a substance through the needle.

U.S. Patent No. 8,267,890 to Alchas discloses a medication delivery device, particularly an intradermal delivery device, having a needle cannula, with a sharpened distal end having a forward tip, and a limiter disposed about the needle cannula. The limiter has a distal end defining a skin engaging surface which is disposed transversely to, and at least partially about, the needle cannula. The skin engaging surface is generally non-flat with generally coplanar portions, and a recess being defined in the skin engaging surface which defines a void in or adjacent to the coplanar portions into which portions of a patient's skin can be deformed into when the skin engaging surface is pressed against the patient's skin. The forward tip of the needle cannula is spaced apart from a plane defined by the coplanar portions a distance ranging from about 0.5 mm to 3.0 mm such that the skin engaging surface limits penetration of the forward tip of the needle cannula to the dermis layer of the patient's skin.

EP 2468340 A discloses a drug delivery device having a preloaded syringe in a distal housing portion and a motor and transmission in a proximal housing portion, wherein when said syringe is inserted into a channel of the distal housing portion, joining a distal end of said proximal housing to a proximal end of said distal housing operationally connects said transmission to said plunger such that activating said motor brings about discharging of said contents of said barrel.

EP 2468342 A discloses relates to a front end for an auto-injector, the front end comprising a syringe with an injection needle and a stopper, further comprising an outer sleeve arranged to be attached to an auto-injector back end comprising a drive means for advancing the syringe and needle in a proximal direction for needle insertion and for advancing the stopper within the syringe for injection, wherein the syringe is slidably arranged within a transfer sleeve which is slidably arranged in the outer sleeve, wherein the transfer sleeve is biased in the proximal direction against the syringe, wherein a locking mechanism is arranged for limiting a proximal extension of the transfer sleeve from the outer sleeve to an initial position in an initial state, wherein the locking mechanism is arranged to allow the transfer sleeve to be extended into a needle safe position covering the advanced needle beyond the initial position after having been translated in the distal direction into a depressed position.

US 2003/0229308 discloses an injector adapter that permits an automatic injector to be releasably coupled to the skin of a living being without the need for a user to hold the injector against the skin during injection, especially in cases where the drug being delivered requires a slow rate of injection. In addition, another variation of injection adapter is described whereby the working end of the automatic injector includes an integrated injector adapter.

Additional background art includes U.S. Patent Application Publication No. 2009/093,792 to Gross and Cabiri, U.S. Patent No. 8,348,898 to Cabiri, U.S. Patent 7,530,964 to Cabiri and U.S. Patent Application Publication No. 2009/0012494 to Yeshurun.

### SUMMARY OF THE INVENTION

The scope of the invention is defined solely in the appended claims.

According to an aspect of some embodiments of the present invention there is provided a drug delivery device comprising: a preloaded syringe; including a sterile hollow needle, a barrel in fluid communication with the sterile hollow needle, a distal end of the barrel rigidly attached to the sterile hollow needle, a plunger glidingly sealing a proximal end of the barrel, the plunger sliding distally thereby discharging a contents of the barrel out the hollow needle and a flange at a proximal end of the barrel; a proximal housing portion including a motor assembly and a transmission converting a rotation motion of the motor assembly to a linear motion; a distal housing portion including a channel fitting the preloaded syringe, the channel including a proximal opening large enough for inserting the syringe into the channel, a distal opening large enough to allow the hollow needle to protrude from a distal end of the distal housing portion; and an adhesive base for fastening the distal portion to a skin of a patient, and wherein when the syringe is inserted into the channel, joining a distal end of the proximal housing to a proximal end of the distal housing operationally connects the transmission to the plunger such that activating the motor brings about discharging of the contents of the barrel wherein the drucg delivery device of the present invention comprises the further features of claim 1.

According to some embodiments of the invention, the drug delivery device further comprises a mechanical connector for fastening the distal housing portion to the proximal housing portion.

According to some embodiments of the invention, the motor assembly includes: a support structure connectable to the proximal housing portion, a motor rigidly attached to the support structure, a power source rigidly attached to the support structure and a syringe position sensor rigidly attached to the support structure; the syringe position sensor controlling an electrical connection between the power source and the motor.

According to the invention, the preloaded syringe includes a needle cover preserving sterility of the sterile needle and the distal housing portion includes an activation device including a handle accessible from outside the channel an attachment mechanism, exposed to the channel such that when the preloading syringe is fully inserted into the channel, the attachment mechanism attaches to the needle cover a coupler passing through a needle aperture in the adhesive base, the coupler connecting the handle to the attachment mechanism.

According to the invention, an adhesive cover for the adhesive base connected to the handle by an extender for peeling the adhesive cover from the base.

According to the invention, the adhesive base is located distal to all of the needle cover.

According to some embodiments of the invention, the distal housing portion further includes a biasing mechanism pushing the syringe distally to a disengaged position.

According to some embodiments of the invention, the adhesive base has an aspect ratio of a long axis to a short axis of at least 1.5 to 1.

According to some embodiments of the invention, the needle has a principle longitudinal axis substantially parallel to an axis of the barrel.

According to some embodiments of the invention, the drug delivery device further comprises a flexible adhesive skirt extending beyond at least one edge of the adhesive base and where the flexible adhesive skirt extends in a direction of a short axis of the adhesive base further from the at least one edge of the adhesive base than from a second edge of the adhesive base.

According to some embodiments of the invention, the drug delivery device further comprises a needle aperture in the adhesive base offset from the at least one edge toward the second edge.

According to some embodiments of the invention, the at least one edge and the second edge are substantially perpendicular to the short axis.

According to some embodiments of the invention, the needle is oriented toward the at least one edge at an angle between 85 and 99 degrees of the base.

According to an aspect of some embodiments of the present invention there is provided a method of manufacture of an autoinjector comprising; supplying a preloaded syringe, a preassembled distal housing portion including a channel for insertion of the preloaded syringe and a proximal housing member including a motor and a transmission; sliding the preloaded syringe longitudinally through an opening in a proximal end of the distal housing member into the channel; joining by an axial motion a distal end the proximal housing portion to a proximal end of the distal housing portion thereby connecting the transmission to a plunger of the preloaded syringe, and characterized by the further features of claim 12.

According to some embodiments of the invention, the preloaded syringe includes a sterile needle and needle cover and the distal housing member includes an adhesive base on a distal end thereof, and the method further comprises attaching a cover remover to the needle cover through an aperture in the adhesive base.

According to some embodiments of the invention, the needle cover is located entirely on one side of the adhesive base.

According to the invention, the adhesive base includes an adhesive cover, and the method further includes connecting the cover remover to the adhesive cover such that removing the needle cover also peels the adhesive cover from the adhesive base.

According to a further aspect of some embodiments which do not form a part of the invention, there is provided an autoinjector comprising: a syringe including: a drug container, a hollow needle rigidly connected to a distal end of the drug container, the hollow needle in fluid communication with an interior of the drug container and a plunger sealing a proximal opening of the drug container; a driver for driving the plunger distally along an axis of the drug container thereby discharging a contents of the drug container through the needle into a patient; and an adhesive base having an aspect ratio of a long axis to a short axis of at least 1.5 to 1; the adhesive base being attached movably to the hollow needle for moving hollow needle axially through a needle hole of the adhesive base and orienting an axis of the drug container at an angle between 60 to 120 to the base during operation of the autoinjector.

According to some embodiments, a height of the autoinjector is at least the length of the short axis of the base.

According to some embodiments, an adhesive strength the adhesive base is small enough to be pivoted off a skin of a patient with ripping the skin when the autoinjector is pivoted around a pivoting axis perpendicular to a short axis of the base.

According to some embodiments, the hollow needle has a principle longitudinal axis substantially parallel to the axis of the drug container.

According to some embodiments, the autoinjector further comprises a flexible adhesive skirt extending beyond at least one edge of the adhesive base and where the flexible adhesive skirt extends in a direction of a short axis of the adhesive base further from the at least one edge of the adhesive base than from a second edge of the adhesive base.

According to some embodiments, the autoinjector further comprises a needle aperture in the adhesive base offset from the at least one edge toward the second edge.

According to some embodiments, the at least one edge and the second edge are substantially perpendicular to the short axis.

According to some embodiments, the hollow needle is oriented toward the at least one edge at an angle between 85 and 99 degrees of the base.

According to some embodiments, the autoinjector further comprises a needle cover for the hollow needle; and a handle connected the needle cover such that a linear movement of the handle pulls the needle over out a needle aperture of the base.

According to some embodiments, the autoinjector further comprises an adhesive cover for the adhesive base connected to the handle by an extender for peeling the adhesive cover from the base upon the linear movement of the handle.

According to some embodiments, the adhesive base is located distal to all of the needle cover.

According to some embodiments, the autoinjector further comprises a needle protection sheath configured to protrude distally from the autoinjector surrounding the hollow needle.

According to an aspect of some embodiments which does not form a part of the invention, there is provided a method of injecting a substance into a patient comprising: fastening a base of the injector to a skin of the patient; moving a syringe axially with respect to the base to insert a needle of the syringe through a needle aperture in the base into the patient; discharging the substance from the syringe through the needle into the patient while the injector remains fastened to the patient; pivoting the injector around an axis along the skin to unfasten the base from the patient.

According to some embodiments, base has an aspect ratio of a long axis to a short axis of at least 1.5 to 1 and wherein the pivoting is around the axis substantially perpendicular to a short axis of the base.

According to some embodiments, the pivoting is around the axis is along an edge of the base.

According to some embodiments, the base includes a flexible adhesive skirt extending beyond at least one edge of the base and wherein the pivoting is around the axis opposite an opposite edge of the base and wherein the skirt extends less beyond the opposite edge than and extent of the base beyond the at least one edge.

According to an aspect of some embodiments there is provided a method of manufacture of a stabilized autoinjector comprising: installing a syringe rigidly attached to a sterile needle and needle cover into a pen injector having an adhesive base; and attaching a cover remover to the needle cover through an aperture in the adhesive base.

According to some embodiments, the method further comprises packaging the stabilized autoinjector for shipping to a patient after the installing and attaching.

According to some embodiments, the needle cover is located entirely on one side of the adhesive base.

According to some embodiments, the syringe includes a plunger sealing a proximal opening thereof and wherein the installing includes inserting the syringe into a channel in a distal assembly of the autoinjector, the distal assembly including the adhesive base on a distal end thereof and further comprising: joining the plunger to a transmission and motor for discharging medicine from the syringe by driving the plunger distally into the syringe, the joining by attaching a proximal assembly to the distal assembly.

According to some embodiments, the attaching includes uniting the distal assembly and the proximal assembly by an axial motion.

According to an aspect of some embodiments there is provided a stabilizer for an autoinjector including housing including a distal surface and a side surface, the autoinjector also including a protective needle sleeve extending distally from the autoinjector, the stabilizer comprising: a connector for attaching to the side surface of the autoinjector; and a fastener for attaching a base of the stabilizer to a skin of a patient the connector configured for holding the housing of the autoinjector in a fixed spatial relation to the fastener; a needle aperture in the base, the needle aperture being large enough for the sleeve to extend through the aperture.

According to some embodiments, the base has an aspect ratio of a long axis to a short axis of at least 1.5 to 1.

According to some embodiments, the stabilizer further comprises a flexible adhesive skirt extending beyond at least one edge of the base and wherein the flexible adhesive skirt extends in a direction of a short axis of the base further from the at least one edge of the base than from a second edge of the base.

According to some embodiments, the stabilizer further comprises a needle aperture in the base offset from the at least one edge toward the second edge.

According to some embodiments, the at least one edge and the second edge are substantially perpendicular to the short axis.

According to some embodiments, the stabilizer further comprises a needle cover for preserving a sterility of a hollow needle of the autoinjector; and a handle connected the needle cover such that a linear movement of the handle pulls the needle cover out the needle aperture.

According to some embodiments, the stabilizer further comprises a cover for the base connected to the handle by an extender for peeling the cover from the base upon the linear movement of the handle.

According to some embodiments, the base is located distal to all of the needle cover.

According to an aspect of some embodiments there is provided an article of manufacture for stabilizing an autoinjector comprising: a fastener for attachment to an injection site of a patient; and a connector for axially reversible attaching to a housing of the autoinjector, the connector configured for contacting an area of less than 4 cm² of the housing and wherein the connector is configured to reversibly hold the housing in a fixed orientation to the fastener.

According to some embodiments, the area of the house includes at least one location at least 1 mm distant from a distal surface of the autoinjector.

According to some embodiments, the connector includes a cavity for enclosing a portion of the autoinjector.

According to some embodiments, the fastener includes an adhesive surface.

According to some embodiments, the adhesive surface has a surface area on at least 2 cm².

According to some embodiments, the contacting area is on a side of the autoinjector.

According to an aspect of some embodiments there is provided a method of retrofitting for stabilized operation an autoinjector configured for discharging of medicine to a skin of a patient contacting a distal surface of the autoinjector comprising: connecting a surface of an external housing of the autoinjector to a stabilizing adapter, the surface including at least one location not on a side surface of the housing; and supplying the autoinjector to a patient after the connecting before fastening the stabilizing adapter to patient.

According to some embodiments, the method further comprises packaging the autoinjector for shipment after the connecting.

According to some embodiments, the method further comprises enclosing a portion of the autoinjector in the adaptor.

According to some embodiments, the autoinjector includes a syringe rigidly attached to a sterile needle and needle cover and wherein the adaptor includes an adhesive base the method further comprising: attaching a cover remover to the needle cover through an aperture in the adhesive base.

According to some embodiments, the needle cover is located entirely on one side of the adhesive base.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart illustrating a method of stabilizing a needle in a patient in accordance with an embodiment of the present invention;
FIG. 2 is a flowchart illustrating a method of injecting a substance in accordance with an embodiment of the present invention;
FIG. 3 is a state diagram of an injector in accordance with an embodiment of the present invention;
FIGs. 4A-C are schematic illustrations of a stabilized injector in accordance with an embodiment of the present invention;
FIG. 5 is a schematic illustration of a needle stabilizer in accordance with an embodiment of the present invention;
FIGs. 6A-F are detailed illustrations of a stabilized injector in accordance with an embodiment of the present invention;
FIGs. 7A-K are detailed illustrations of an alternative stabilized injector in accordance with an embodiment of the present invention;
FIG. 8 is a flow chart illustration of a method of manufacture of a stabilized pen injector in accordance with an embodiment of the present invention;
FIG. 9 illustrates an external view of a stabilized injector in accordance with an embodiment of the current invention;
FIG. 10A-D are crossectional views illustrating a microswitch activator for an autoinjector in accordance with some embodiments of the current invention;
FIG. 11 is a flowchart illustration of a method of stabilizing an autoinjector in accordance with some embodiments of the current invention;
FIG. 12 is a state diagram of an autoinjector and extending adaptor in accordance with some embodiments of the current invention;
FIG. 13 is a block diagram of an autoinjector and a stabilizing adaptor in accordance with some embodiments of the current invention;
FIG. 14A-F is a perspective view and schematic cross sectional views of various states of an adhesive and ring stabilizing adaptor and autoinjector in accordance with some embodiments of the current invention;
FIG. 15 is a schematic cross sectional illustration of a friction fitting for stabilizing an autoinjector in accordance with some embodiments of the current invention;
FIG 16 is a perspective view of an adjustable angled stabilizing adaptor in accordance with some embodiments of the current invention;
FIG 17 is a perspective view of a stabilizing adaptor and autoinjector with a strap fastener in accordance with some embodiments of the current invention;
FIG. 18 is an illustration of a stabilizing adaptor with threaded connector in accordance with some embodiments of the current invention;
FIGS. 19A,B are a schematic cross sectional illustrations and a perspective illustration of a stabilizing adapter in accordance with some embodiments of the current invention;
FIGS 20 A,B are schematic cross sectional illustrations of a stabilizing adapter in accordance with some embodiments of the current invention;
FIG 21 is a schematic cross sectional of a multipronged adapter and autoinjector in accordance with an embodiment of the current invention;
FIG. 22 is a flowchart illustrating a method of manufacture of a compound device in accordance with an embodiment of the present invention;
FIGs. 23A-D illustrate a needle cover and an injector safety cover including a needle cover remover in accordance with the present invention;
FIGs. 24A-G illustrate a modular stabilized injector in accordance with an embodiment of the present invention;
FIG. 25 illustrates a modular stabilized injector in accordance with an embodiment of the present invention;
FIG 26 is a flow chart illustrating a method of manufacture of a modular preloaded stabilized drug delivery device in accordance with an embodiment of the current invention;
FIG 27 is a flow chart illustrating a method of retrofitting a pen injector in accordance with an embodiment of the current invention;
FIGs. 28A-C illustrate removing a stabilized pen injector in accordance with an embodiment of the current invention;
FIGs. 29A-B illustrate a stabilizing adaptor having a large needle aperture in accordance with an embodiment of the current invention
FIGs. 30A-B illustrate a stabilizing adaptor which allows an injector to be removed from the adapter axially in accordance with an embodiment of the current invention; and
FIG. 31 illustrates pen injector **1400** retrofit with stabilizing adaptor **1627** packaged for distribution in accordance with an embodiment of the current invention.

### DESCRIPTION OF SPECIFIC EMBODIMENTS OF THE INVENTION

The present invention, in some embodiments thereof, relates to a medical injector, namely a drug delivery device, and, more particularly, but not exclusively, to an injector for an extended bolus delivery.

### Overview

### 1 Pen injector with stabilizer

An aspect of some embodiments of the present invention relates to an autoinjector and/or pen injector with stabilization. Stabilization may include for example fastening and/or adhering the injector to the skin of a patient. In some embodiments, a stabilized injector may perform injections of larger volumes and/or larger time lengths than a conventional pen injector.

In some embodiments, a pen injector may include a pre-sterilized fluid path. Optionally the fluid path may be inserted into the injector in a sterile and/or protected condition. Optionally a protective cover for the fluid path may be removed by an end user. Optionally the fluid path may be very simple. For example, the fluid path may include a straight needle directly and/or rigidly connected to a drug container. In some embodiments, the needle may be in fluid communication with the drug container, for example as in a standard hypodermic syringe. In some embodiments the needle may have a sterile cover. The needle cover may for example include a standard needle cover. Optionally, the needle may be coaxial to the drug container. Alternatively or additionally the needle may be mounted of center of the drug container. Some embodiments of the invention may include a standard straight needle. Alternatively or additionally, the needle may be bent and/or curved. Optionally the needle cover may not protect the sterility of the needle. For example the entire injector or a portion thereof may be sterilized.

In some cases, it may be difficult for a user to manually hold an autoinjector stable. For example the user may find it difficult to hole the injector immobile enough and/or for long enough to complete injection. A stabilized auto injector may include a skin fastener, for example an adhesive, to increase stability of the injector. The fastener may hold and/or assist the user to hold the activation zone of the injector and/or the injection zone and/or a needle aperture and/or a needle stable with respect to the skin of the patient. The pen injector may include and/or perform some or all of the functions of a syringe stabilizer and/or a needle positioner, for example as mentioned herein below.

In accordance with some embodiments of the current invention the payload of the syringe may include, for example between 0.5 and 2ml and/or between 2 and 4ml and/or between 4 and 5 ml of a drug and/or more. In some embodiments, the injector may discharge the entire payload as a single dose. For example, a pen injector may include an internally powered driver to drive the plunger and/or discharge the payload. For the sake of this application an internally powered injector driver may be defined as a drive mechanism powered by energy stored at least temporarily within the injector. Power may be stored in a power supply, for instance as chemical potential (for example a chemical that produces an expanding gas and/or a battery) and/or mechanical potential (for example stored in an elastic member and/or a spring and/or a pressurized gas). For example the driver may be designed to discharge the payload over a time period ranging between 20 and 120 seconds and/or between 120 and 600 seconds and/or longer. In some embodiments, discharge may be driven by a driver. An internally powered driver may be powered by various mechanisms including for example a motor (including for example a DC motor, an actuator, a brushless motor) and/or a transmission including for example a telescoping assembly and/or a threaded element and/or a gear and/or a coupling and/or an elastic mechanism (for example a spring and/or a rubber band) and/or an expanding gas and/or a hydraulic actuator).

A pen injector in accordance with some embodiments of the current invention may include a medicine container. For example a medicine container may be a standard type syringe. Optionally a standard type syringe may be preloaded with medicine using standard equipment and/or in an aseptic room. A preloaded standard type syringe may optionally include a proximal opening. A plunger may optionally seal the proximal opening and/or protect the sterility of the contents of the syringe. A sterile needle (for example a hollow needle) may optionally be connected to the syringe barrel. For example, the hollow of the needle may be in fluid communication with the interior of the barrel. The needle may optionally be rigidly attached to the distal end of the barrel. The sterility of all and/or part of the needle may for example be protected by a sterile cover. The sterile cover may remain on the needle when the syringe is supplied and/or installed into an injector. For example, the medicine container may optionally include a cylindrical barrel rigidly attached to a needle. Optionally, the long axes of the needle and barrel of the syringe may be parallel and/or coaxial. Optionally, the needle may be mounted on the distal end of the barrel. Optionally the needle point may be pointing in the distal direction. In some embodiments a plunger may slide axially along the inside of the barrel to discharge a medicine payload. For example, the medicine may be discharged through the hollow needle.

An aspect ratio of the base may be defined as the ratio of the length of the longest axis of the base to the shortest axis. Optionally the axis ratio may range between 1.5 to 2 and/or between 2 to 3 and/or greater than 3. In some embodiments, the height of the injector may range between half the length of the short axis of the base to the length of the short axis of the base and/or between the length of the short axis of the base to twice the length of the short axis of the base and/or greater than the twice length of the short axis of the base. The height of the injector may supply leverage for pivoting the adhesive off the skin of a patient after use.

### 2 Syringe stabilizer

An aspect of some embodiments of the present invention relates to a stabilization device for a hypodermic syringe. In some cases, it may be difficult for a user to manually hold a conventional syringe in a proper location and/or immobile enough and/or for long enough to complete injection. This problem may be particularly acute in home treatments where the caretaker may not be highly skilled. This problem may be particularly acute when injecting large volumes of liquid and/or for long periods of time, for example longer than 20 sec.

In some embodiments a syringe may be held stable with respect a fastener, fastened to the skin of a patient. The fastener may stabilize the syringe with respect to the skin of a patient. For example, with stabilization, a non-skilled person may be able to hold a syringe in its proper place for a time ranging between 20 to 600 seconds and/or the syringe may be held substantially immobile with respect to the skin of a patient for a time ranging for example between 60 to 180 seconds. For example the injector may inject of volume of drug ranging between 0.5 to 5ml. Optionally, the injection may be a bolus delivery and/or extended bolus delivery and/or continuous delivery.

For example, holding the injector immobile may include preventing straining the needle and/or bending the needle and/or occluding the needle and/or injury the patient in the injection site and/or moving the needle from the intended injection site for example due to shearing forces along the patient's skin and/or forces perpendicular to the patient's skin. In some embodiments, the fastener may be strong enough to prevent lateral movement of the injector (sliding along the skin of the patient) under designated design stresses. In some embodiments, the fastener may be strong enough to hold the injector to the body of the patient without additional support (for example preventing lateral movement and/or longitudinal movement (perpendicular to the skin of the patent) and/or rotational movement with respect to the patient's skin under designated design stresses. Alternatively or additionally, the fastener may assist a user to manually hold the injector to the patient's body.

### 3 Needle positioner

An aspect of some embodiments of the present invention relates to a device for positioning a needle in an intended tissue for medical injection. For example, it is sometimes desired to inject a drug into subcutaneous tissue. In some cases locating a conventional needle in the proper tissue can be difficult. For example skin can fold, wrinkle, stretch and/or deform in such a way that it is difficult to locate the correct tissue. In some embodiments of the current invention, an adhesive base is used to hold the skin of a patient in a fixed position. A syringe may be held in a predetermined position with respect to the base such that a needle connected to the syringe pierces the skin and enters a desired tissue.

In some embodiments, a syringe may be held substantially perpendicular to a surface contacting the user's skin. For example, the syringe may be held at an angle ranging between 60° to 120° to the user's skin during injection. In some embodiments an injection may be into subcutaneous tissue. For example the needle length and/or needle insertion depth may range for between 3 to 12 mm. In some embodiments an injection may be into muscle. For example the needle length and/or needle insertion depth may range for between 16 to 25 mm.

### 4 Modular autoinjector

An aspect of some embodiments of the present invention relates to a modular autoinjector and/or methods of construction and assembly of a parenteral drug delivery system. Optionally, final assembly of the autoinjector is by bringing together an Upper (proximal) Sub-assembly and/or a Lower (distal) Subassembly, and/or a pre-loaded syringe including for example a sterile needle and/or a sterile needle cover. The syringe optionally contains an aseptic medicament solution. For example the final assembly may consist of the pre-filled syringe being first installed in the Lower Subassembly in a motion along single axis. The Upper Sub-assembly and Lower Sub-assembly are optionally coupled together, e.g. by mechanical snap fit assembly, in a single axial motion with the pre-filled syringe captured within. The Upper Sub-assembly optionally comprises a housing, a transmission including for example coupling (for example a gear) and/or a telescoping screw assembly. Optionally a motor drive and/or some and/or all associated electrical components, e.g. switch, batteries and allied components, accessories, etc. are installed on an integral support structure (for example printed circuit board PCB). The electrical components and/or the support structure may constitute for example a pre-fabricated Motor Drive Sub-assembly. The prefabricated Motor Drive Sub-assembly can optionally be tested independently from the drug delivery system. The Motor Drive Sub-assembly is optionally configured to be installed into the Upper and Lower Subassemblies co-axially, for example by means of mechanical snap fit relationships.

### 5 Flexible cover to peel adhesive protector

An aspect of some embodiments of the present invention relates to a mechanism to peel an adhesive protector when a safety cover is pulled off of an autoinjector. For example the safety cover may be flexible and/or a hinged and/or may be anchored to an edge of the adhesive cover. For example the safety cover may include a needle cover remover connected to a folded adhesive protector. As the safety cover is pulled away the pulling force may be transferred to a peeling force, peeling back the adhesive at one or more edges of the adhesive protector.

### 6 Rotary needle retraction mechanism

An aspect of some embodiments of the present invention relates to a needle safety mechanism for an autoinjector. Optionally, an unshielded needle may be safeguarded in response to resistance and/or a change of resistance to discharging of the injector payload.

In some embodiments a change in resistance to discharging may activate a rotary needle protector. For example, a resistance to discharging may engage a pair of threaded elements. Optionally, a drive may power discharging of a drug. The drive may drive a relative rotation between the pair of threaded elements. Relative rotation of the threaded elements may, in some embodiments, activate needle protection. For example, relative rotation of the pair of threaded elements may cause retraction of the needle.

In some embodiments, a lock may hold a needle in an unshielded position. The lock may optionally act as a support for a discharge driver. Increasing resistance to discharge may increase the stress on the driver. When the stress on the driver increases beyond a threshold and/or decreases beyond a threshold the lock may be released and/or the needle retracted to a safe location. For the sake of the current disclosure retraction of a needle may include pulling a needle point back into a shielded location without changing the length and/or shape of the shielding; and/or retracting may include extending the housing of the injector and/or a shield ahead of a needle point such that the needle is left in a shielded location.

In some embodiments, a support for a needle assembly may include a telescoping assembly. Optionally, the telescoping assembly may retract the needle (for example by contracting) in response to a stress from a driver.

In some embodiments, the safeguarding mechanism may include a sensor sensitive to a linear force from the driver. For example, a pushing force passes a threshold; a lock may be released moving the needle to the retracted configuration.

In some embodiments, the force to insert the needle to the skin of a patient may range for example between 0.02 to 0.2 N and/or between 0.2 and 0.5 N. Optionally, the force required to inject the drug (for example the force on a syringe plunger) may range for example between 5 to 60 N. For example the force required to inject the drug may depend on the injection rate and/or the viscosity of the drug and/or the syringe geometry and/or the needle dimensions.

In some embodiments a needle protection mechanism may be triggered by a linear force greater than, for example, between 10 to 60 N.

For example, an autoinjector may be activated by manually pushing with enough force to insert the needle. The device may then apply an injection force to inject a drug. Once the entire drug is injected and/or when there is an obstruction and/or occlusion, the injection force may rise until it passes a threshold triggering safeguarding of the needle and/or ending injection.

For example in the event of an occlusion and/or at the end of delivery, the linear force generated by the device may increase to the level of up to 60 N. A needle safeguarding mechanism may include a sensor that is sensitive to the force. For example the sensor may include a snap that gives way at 40 N returning the needle to the retracted position.

In some embodiments, the stress to inject a medicine and/or to trigger safeguarding of a needle may include a torque. For example, injection of medicine may be driven by a plunger. The plunger may optionally be driven by a threaded assembly, for example a threaded screw and/or teeth and/or a telescoping assembly. Optionally the pitch of the teeth and/or an associated screw may range for example between 0.5 and 2 mm. The diameter of the screw may range for example between 3 and 15 mm. The torque to power injection may range for example between 0.2 and 1.0 N*cm. The trigger torque (the torque at which the needle safeguarding is triggered) may range for example between to 0.5 to 2 and/or from 2 to 4 and/or from 4 to 10N*cm.

In some embodiments a safety mechanism may include linear movement of the ranging between 5 to 15 mm. For example movement of the safety mechanism may include extension of a needle during insertion and/or retraction of the needle and/or extensions of a safety shield and/or retraction of a safety shield. Optionally a needle insertion length (for example the length of needle inserted into a patient) may range for example between 3 to 12mm.

During injection, the linear movement of a plunger may range for example between 10-50mm. The length of movement of the plunger may vary for example with the volume of medicine to be injected that may range for example between 0.5 to 3ml.

In some embodiments, a sensor of a safeguarding mechanism may be sensitive to a torque. For example, the needle may be retracted when the mechanism is exposed to a twisting moment. Optionally, discharge may be driven by a torque. For example the driver may apply torque to threaded element pushing a plunger. When the torque on the driver reaches a threshold value, the needle may be released and/or retracted and/or a needle shield may be deployed. Alternatively or additionally the trigger mechanism may require both a torque and a linear force. For example, requiring both a torque and a linear stress may prevent premature activation due to momentary friction.

In some embodiments an interference element (for example a snap) may provide resistance to retraction. For example, an annular ring may impede contraction of a telescoping assembly. Alternatively or additionally a rib may impede twisting of a support structure. When stress from the driver passes a threshold, the stress may optionally overcome the interference element. Overcoming the interference element may for example reverts the support to a retracted configuration.

In some embodiments, a stress resulting from resistance to discharge may trigger deployment of a needle shield. The needle shield may optionally move to shield the needle in reaction to the increased stress.

In some embodiments, the retraction mechanism may include a rotary drive. For example, threaded element may raise the needle. For example a motor may drive a plunger injecting a drug (for example by means of a threaded plunger driving assembly). Upon triggering of the release mechanism the same motor may optionally rotate a second threaded assembly retracting the needle.

### 7 Stabilizing adapter for an autoinjector

An aspect of some embodiments of the present invention relates to an adaptor for extending the use of a drug delivery device, for example a single use autoinjector. Optionally an adapter may stabilize an existing injector. For example an adaptor may be configured to fasten an existing pen injector to an injection site of a patient and/or at a fixed orientation to the injection site. In some embodiments, stabilization may extend the use of an existing subcutaneous pen injector. For example stabilizing a pen injector may extend use of the injector to longer injection times and/or to larger injection volumes and/or to more viscous medicines and/or to lower temperatures and/or to use by movement limited patients and/or to patients for whom it is difficult to stabilize the injector and/or hold the injector to the skin (for example extremely fat and/or flabby patients). For example, a non-stabilized pen injector may be licensed for autonomous use (without a stabilizing adapter) with a certain medicament. Under certain conditions (for example when the medicament is highly viscous for example because it cold or highly concentrated) the injection time may extend beyond the licensed maximum injection time for the unstabilized injector. Optionally, in some embodiments adding a stabilizing adaptor will extend the permissible injection time of the injector. Extending the permissible injection time may, for example, allow use of the injector under the new conditions. Alternatively or additional, stabilization may extend use of a stabilized injector to a patient population that is limited in its ability to hold the non-stabilized injector in place.

In some embodiments, stabilization will have minimal or no affect on the workings of the injector. For example, a stabilizing adaptor may be designed to allow unchanged functioning and/or operation of an existing injector. Minimizing changes in the function and/or operation of the injector may reduce the amount of testing necessary for licensing and/or marking of the stabilized injector and/or adaptor.

Optionally the injector may be held offset from the base of the stabilizer and/or the needle of the injector may be at an angle to the base of the stabilizer. For example, the needle location and/or the injector may be located away from an axis of pivoting for removing the stabilizer. For example the injector and/or the needle may be pointed inward with respect to the axis of pivoting. Alternatively or additionally the adhesive may be peeled from the skin manually, for example like a band aid. For example, the adhesive may have a tab and/or an area of reduced tackiness for easy peeling.

In some embodiments, an adaptor (for example a stabilizer) may extend use of an injector to new conditions. For example a stabilized injector may be used for adapting the injector to longer injection times, higher viscosity medicine and/or larger volumes of medicine. For example, the time of injection with the adaptor may range between 15 to 20 seconds (for example for between 0.8 and 1.2ml of fluid having a viscosity ranging between 7 to 9cp) and/or between 20 to 30 seconds (for example for between 1.2 and 1.7 ml of fluid having a viscosity ranging between 7 to 9cp) and/or between 30 to 60 seconds (for example for between 1.7 and 2.3ml of fluid having a viscosity between 8 to 12cp) and/or between 60 to 120 seconds (for example for 2.0 to 2.5 ml of fluid having a viscosity of between 16 and 40cp) and/or between 120 seconds and 3 minutes for larger volumes and/or viscosities

In some embodiments, an adaptor may include an adjustable element. For example an adjustable element may include an elastic element and/or a spring and/or a threaded element and/or a clamp. In some embodiments an adaptor may include a friction fitting and/or an adhesive and/or a tightenable fitting. For example, a friction fitting may include one way teeth. For example it may require less force to insert an injector into an adapter then to remove the injector from the adapter. For example the force for removal may range between 1.5 and 3 times the force for insertion and/or the force for removal may range between 3and 6 times the force for insertion and/or the force for removal may be more than 6 times the force for insertion. Alternatively or additionally a friction fitting may have substantially the same resistance to insertion and/or removal of the injector.

In some embodiments, an adaptor may be configured to attach to any one of a group of multiple injectors. Alternatively or additionally, an adaptor may be configured to be attached to a specific injector, for example, HUMIRA® pen autoinjection device for subcutaneous injection of the fully human monoclonal antibody adalimumab from Abbot lab and/or Enbrel® of Amgen - injected with auto injector for treatment of moderate to severe rheumatoid arthritis, adult chronic moderate to severe plaque psoriasis and/or other pen injectors like Ember™ and/or Molly™ and/or DAI™ and/or PSDI™ or other models from SHL and other producers

In some embodiments, an extending adaptor and/or a portion thereof may be supplied with an injector. For example, an adaptor may be supplied with the injector in a kit. Alternatively or additionally an adaptor may be attached to an injector and supplied as a single unit. For example the adaptor will be attached to the injector by a supplier. Alternatively or additionally the adaptor may be attached to an injector by a doctor, a nurse, a pharmacist and/or another medical professional before being supplied to a patient. In some embodiments, the adaptor may be permanently attached to the injector. Alternatively or additionally, the adaptor may be removed from the injector, for example after use. For example, the injector may be removed from the patient while the adaptor remains fastened to the patient. Alternatively or additionally, the adaptor and/or the injector may be removed together from the patient. In some embodiments, a connector may be supplied with and/or come attached to the injector. A fastener for fastening to a patient may mate with a coupling. Optionally, a single fastener may be configured for use with different couplers (for example to connect to different injectors).

In some embodiments an adapter may be used to stabilize a subcutaneous injector. For example a subcutaneous injector may include a patch injector and/or a pen injector. For example, a pen injector may have a skin contact area on a base of the device, for example on a distal surface of the device. Optionally a pen injector has a long axis substantially perpendicular to the surface of an injection site on the patient. Alternatively or additionally a pen injector has a long axis that is substantially parallel to the long axis of the injection needle. A pen injector is optionally operated and/or configured to be operated with a long axis of the injector at an angle ranging between 80 to 100 degrees and/or between 60 and 120 degrees and/or between 30 and 150 degrees and/or ranging between 10 to 170 degrees to the surface of an injection site on the patient. The distal surface of a pen injector may have an area ranging between 1 mm² to 25 mm² and/or between 25 mm² to 1 cm² and/or between 1 cm² to 2 cm² and/or between 2 cm² to 4 cm² and/or between 4 cm² to 9 cm². For example the skin contact area may surround a needle aperture on the distal surface of the device. For example a patch injector may have a long axis parallel to the distal surface of the injector and/or parallel to an injection zone on the patient. The long axis of a patch injector may for example be at an angle ranging between 80 to 100 degrees and/or between 60 and 120 degrees and/or between 30 and 150 degrees and/or ranging between 10 to 170 degrees to the long axis of an injection needle. The distal surface of a patch injector may include the base of the device and/or a skin contact zone. The distal surface may, for example, have an area ranging between 4 cm² to 50 cm² or more. Subcutaneous injection sites may include for example an outer area of the upper arm and/or Just above and/or below the waist and/or an upper area of the buttock and/or a front of the thigh. In some embodiments a stabilizing adaptor may surround and/or increase the skin contact area of an injector and/or a distal surface of the injector. Alternatively or additionally an adaptor and/or a connector may be attached to a housing of an injector at a location distanced from the distal surface of the injector. For example the attachment location may range between 1 to 5 mm from the distal surface and/or between 5 mm and 2 cm from the distal surface and/or between 2 cm and 5 cm from the distal surface and/or more than 5 cm from the distal surface. For example the adapter may be attached to the side walls of the injector. The adaptor may fasten to the skin at the injection site and/or in an area ranging between 1 mm and 1 cm from the injection site and/or in an area ranging between 1 cm and 5 cm from the injection site and/or in an area ranging between 5 cm to 10 cm from the injection site.

In some embodiments, the connector may include a cavity for enclosing a portion of the injector. For example, a distal portion of the injector may be held in the cavity. In some embodiments the injector may be held inside the cavity by an adhesive and/or a friction element and/or an interference element and/or a magnet. For example the cavity may be cylindrical and/or conical and/or elliptical. Optionally the cavity may be fit for the shape of the outer housing of the autoinjector. For example the cross section of the opening of the cavity may range for example between 1 mm² to 25 mm² and/or between 25 mm² to 1 cm² and/or between 1 cm² to 2 cm² and/or between 2 cm² to 4 cm² and/or between 4 cm² to 9 cm². The length of the cavity may range for example between 1 mm to 5 mm and/or between 5 mm to 20 mm and/or between 20 mm to 50 mm and/or between 50 mm to 100 mm. The surface of contact between the injector and the connector may be less than 1 mm² (for example for a pressure fit screw or pin) and/or may range between 1 mm² to 4mm² and/or may range between 4 mm² to 10 mm² and/or may range between 10 mm² to 1 cm² and/or may range between 1 cm² to 10 cm² and/or may be greater than 10 cm².

In some embodiments an adapter may be used to stabilize an intramuscular injector. For example an intramuscular injection site may include an area over a Deltoid Muscle and/or an area over a Vastus Lateralis Muscle and/or an area over a Ventrogluteal Muscle and/or an area over a Dorsogluteal Muscle.

In some embodiments, an extending adaptor may be supplied separately from the injector. For example, in some embodiments, the adaptor may be fastened to the patient without the injector. Optionally the injector is attached to the adaptor while the adapter is still on the patient. Alternatively or additionally, the patient and/or a medical aid may attach the adapter to the injector before fastening the adaptor to the patient.

In some embodiments, an adaptor extending use of an injector may make minimal changes in the injector. For example, the adaptor may be attached to the injector in a manner that permits access to the controls of the injector. Optionally, the adaptor is attached to the outer housing of the injector without changing the internal operation of the injector. For example, an autoinjector that is activated by the skin contact trigger in the autonomous mode may also be activated by a skin contract trigger in the extended mode. For example, an autoinjector that is activated by push button trigger in the autonomous mode may also be activated by the push button trigger in the extended mode.

In some embodiments, an adaptor may be configured to permit operation of a stabilized injector with minimal change from unstabilized autonomous operation. For example an adaptor may permit user access to the controls of the injector in the same manner as for autonomous operation. For example, the positioning and/or operation of the injector may be within the prescribed operation and/or positioning of the injector for unstabilized operation. For example, when stabilized the injector may be orientated to the patient as in autonomous operation. For example, the distal surface of the injector and/or a contact surface of the injector in autonomous operation may also contact the patient in stabilized operation. Alternatively or additionally, distal surface and/or the contact surface may contact the adaptor in stabilized operation and/or the adaptor may contact the skin of the patient. In some embodiments, in stabilized operation the distance between the injection site of the patient and a distal surface of the adaptor may range between 1 mm and 0.5 mm and/or between 0.5 mm and 0.1 mm and/or less than 0.1 mm. An angle between the injector and the injection site of the patient in stabilized operation may within the recommended range of angles during unstabilized operation.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary embodiments

### 1 Method of positioning and/or stabilizing a needle

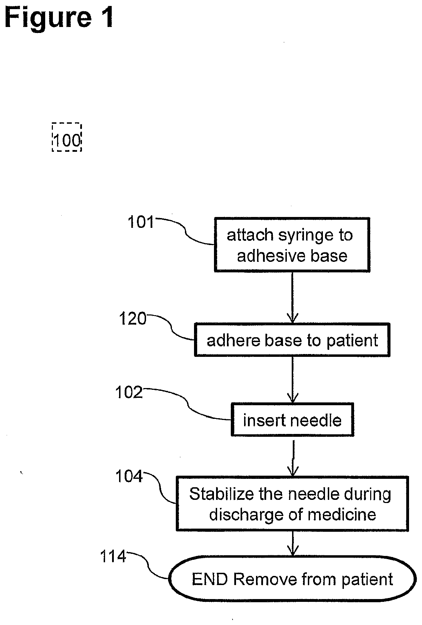

Referring now to the drawings, fig. 1 illustrates a method of positioning and/or stabilizing a hypodermic needle in accordance with an embodiment **100** of the present invention. In the method an adhesive base is used to retain the skin and/or hypodermic tissue of a patient in a predicable geometry. A syringe retainer holds a syringe in a predetermined position with respect to the base such that a needle mounted to the syringe penetrates the skin to reach a desired tissue. A medicament may optionally be discharged to the desired tissue. The syringe and/or needle may optionally be held in place for an extended period, for example, for a long injection ranging between 30 seconds and 500 seconds. Optionally, an injection could include a large dose for example between 0.5 and 5 ml.

In some embodiments a syringe may be attached **101** to an adhesive base. The attachment may have fixed position and/or the syringe may be movable attached to the base. For example, the attached syringe may move longitudinally between a storage position wherein a needle is shielded by the base and an exposed position wherein a portion of the needle protrudes past the base.

In some embodiments, the base may be adhered **120** to the skin of a patient. Optionally the needle may be attached to the base either before or after adhesion **120** to the skin.

In some embodiments, a needle may be inserted **102** into the skin of the patient. For example, the needle may be inserted **102** by means of a longitudinal movement pushing a portion of the needle into a deployed position point past the base (for example through a hole in the base) into the patent. Optionally, the syringe may be fixedly attached to the base with the needle protruding beyond the adhesive such that placing the adhesive onto the patient also inserts the needle into the patient.

Optionally the device may stabilize **104** in a desired position during discharge of a medicine. When discharge of the medicine finishes, the needle may be removed **114** from the patient and/or the adhesive base may be peeled and/or pivoted from the skin. For example, the entire device may be twisted such that one side of the adhesive is lifted and/or pivoted and/or peeled from the skin while the far edge of the base of the injector remains in contact with the skin and serves as a fulcrum.

### 2 Method of injecting a drug

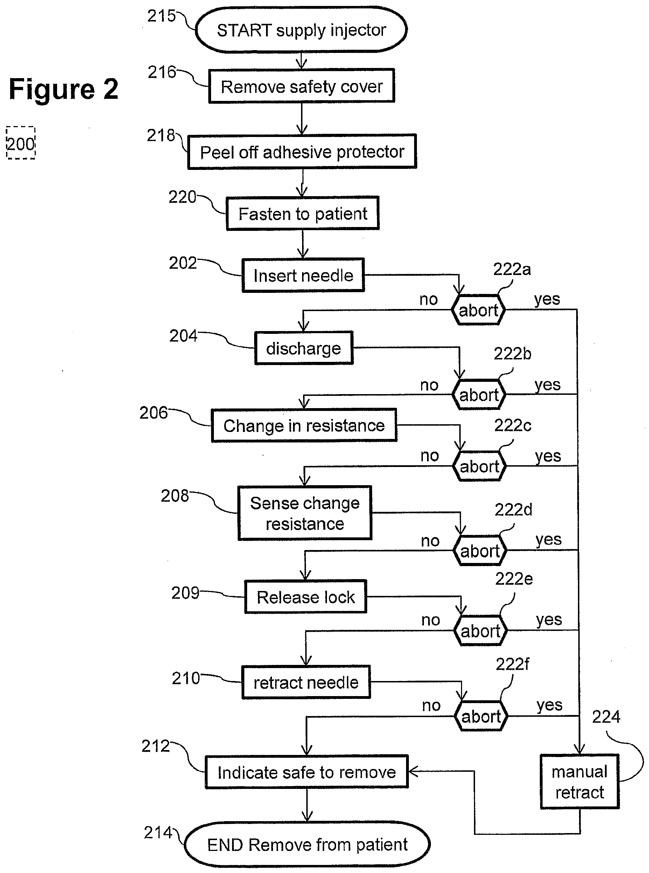

Referring now to the drawings, fig. 2 illustrates a method of injecting a drug in accordance with an embodiment **200** of the present invention. In the exemplary method, a needle is optionally held stable while a drug is dispensed to a patient (dispensing a drug may include discharging a drug from the injector).

In exemplary embodiment **200** a user (for example a patient and/or a medical aid in home care) may be supplied **215** with an autoinjector ready to administer a medicine.

The user may optionally remove **216** a safety cover from the injector. Removing **216** the cover may optionally include removing a sterile cover from a needle. Removing **216** the cover may optionally automatically peel **218** an adhesive protector from an adhesive. For example, the adhesive may be supplied to stabilize the injector on the skin of a patient during injection. The injector may optionally be fastened **220** to a patient (who may be the user). In some embodiments a user may hold the injector to the skin of a patient; the fastening **220** may stabilize the injector for example from shifts and/or movements of the patient and/or the user.

In some embodiments, the user may set off an activation mechanism. The activation mechanism may for example insert **202** the needle into the patient, for example by extending the needle outward. For example, a syringe may be moveably attached to the base. A syringe may optionally be rigidly attached to the syringe. For example the syringe may slide linearly along its axis. Sliding the syringe towards the base may cause the needle rigidly to protrude beyond the base. For example, part of the needle may pass through a hole in the base and pierce the skin of a patient. The adhesive of the base may hold the skin of the patient steady while the needle pierces the skin. The combination of an adhesive holding the skin and moving the needle to a predetermined position past the base may facilitate the inserting **202** of the needle into the skin to the desired depth.

The needle may optionally be locked in the extended position. Optionally, the needle may be biased to a protected position (for example to retract into a housing of the injector). Alternatively or additionally, the needle may be biased to the unshielded position. Alternatively or additionally, the autoinjector may be supplied with the needle in an extended mode and/or protected by a cover.

At a point during the injection process, an optional manual retraction **224** mechanism may be used to place the injector in a safeguarded mode. For example, when the user decides to abort **222a-f** at a point in the process (for example when he detects some sort of malfunction and/or feels a negative reaction to the medicine) the user may manually retract **224** the needle. Optionally there may be an indicator to indicate **212** whether the needle was automatically retracted **210** and/or whether needle was manually retracted **224.** Alternatively or additionally there may be an indicator whether a full dose was administered and/or how much medicine was administered.

Once the needle is inserted into the patient, the injector may optionally begin discharging **204** medicine. For example the medicine may be injected through the needle into the patient. Optionally, discharge may continue until a full dose of the medicine is administered.

In some embodiments, after administration of a full dose of the medicine, there may be a change **206** in resistance to further discharging. For example in a syringe based injector, a plunger may reach the end of the syringe and cease to move increasing resistance. Alternatively or additionally, after discharging the entire dose a transmission may be disconnected (for example a threaded element may pass the end of its threading) reducing resistance. Alternatively or additionally, the change **206** in resistance may result from another cause for example increased resistance due to a full or partial occlusion of a fluid pathway and/or jamming of a mechanical component (for example cross threading of a screw). The change of resistance may optionally be sensed **208** triggering retracting **210** of the needle.

In some embodiments, the needle may be locked in an unshielded state by a force sensitive lock. When the lock senses **208** the change **206** in resistance, it may release **209** the needle which may be retracted **210** to a shielded position.

In some embodiments, a flag may be supplied (for example a LED and/or a changing color indicator) to indicate **212** to the user that the needle has been retracted **210** and/or that the injector can safely be removed **214** from the patient and/or that a fastener has been released. For example, if the injector is adhered to the patient, it may be peeled off and/or a fastener may be released.

### 3 States of an autoinjector

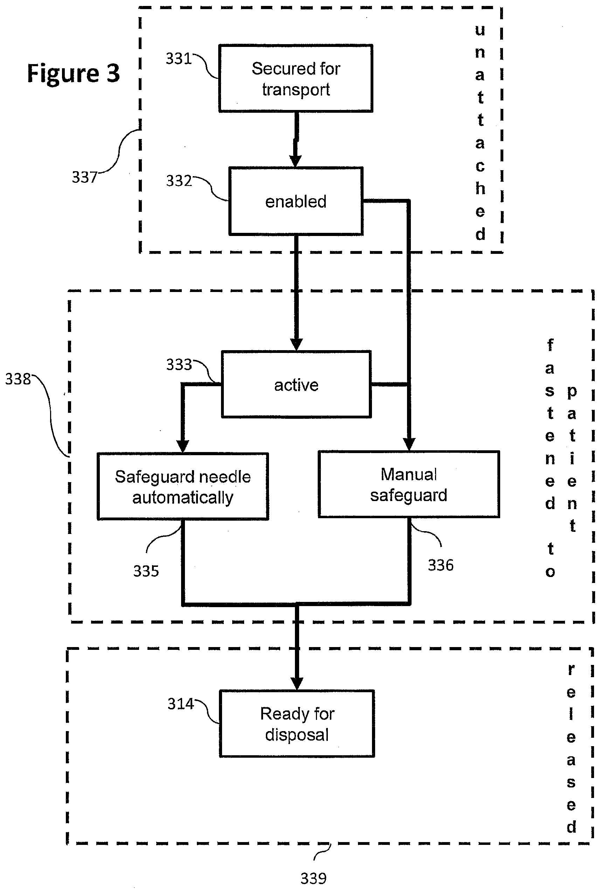

Fig. 3 is a state diagram of an autoinjector in accordance with an embodiment of the present invention. In general, may be supplied in an unattached **337** state. An unattached **337** autoinjector may have a secured **331** state. For example in the secured **331** state the injector may be safe to handle and/or transport. Optionally the injector may have an enabled **332** state. For example, in the enabled **332** state, the injector may be unstable and/or easily activated. For example, an injector may be switched from the secured **331** state to the enabled **332** state by removing a needle protector and/or an adhesive cover.

Once activated the injector may optionally be fastened to a patient. In the fastened **338** state the injected may optionally be activated. For example, while the injector is in the active **333** state, a needle may project from the injector. In some embodiments the injector may be hazardous to handle in the enabled **332** and/or active **333** state.

In some embodiments, after use (optionally whether or not administration of the full dose was successful) the user may want to remove and/or dispose of the autoinjector. In some embodiments, it may be difficult and/or dangerous to remove an injector in the enabled and/or active state. For example, when an injector is fastened to a patient by an adhesive, it may be difficult to remove the needle by pulling the injector away from the skin. Optionally, first a needle may be retracted from the skin into the injector. Subsequently the adhesive may be removal by pivoting and/or pulling and/or peeling from the skin. In some embodiments, the injector may automatically be safeguarded **335** for example by retraction of a needle upon completion of injection. Alternatively or additionally, the user may have the option to manually secure the injector into a safeguarded **336** state. For example, the optionally of manually needle retraction may avoid the situation where a patient may not be able to properly remove the injector due to a malfunction that leaves the injector fastened to the skin with the needle inserted into the patient. During and/or after safeguarding **335, 336** the injector may be removed from the patient.

Optionally, the injector may have a final released state **339,** for example wherein the needle is retracted back into the injector and/or the needle tip is shielded and/or the injector has been unfastened from the patient. Optionally one or more indicators may be supplied to indicate the state of the injector and/or the quantity of medicine discharged. Once released, the injector may be in final **314** state (protected from hazards and/or ready for disposal, for example in a municipal waste).

### 4 Fastening via clasping

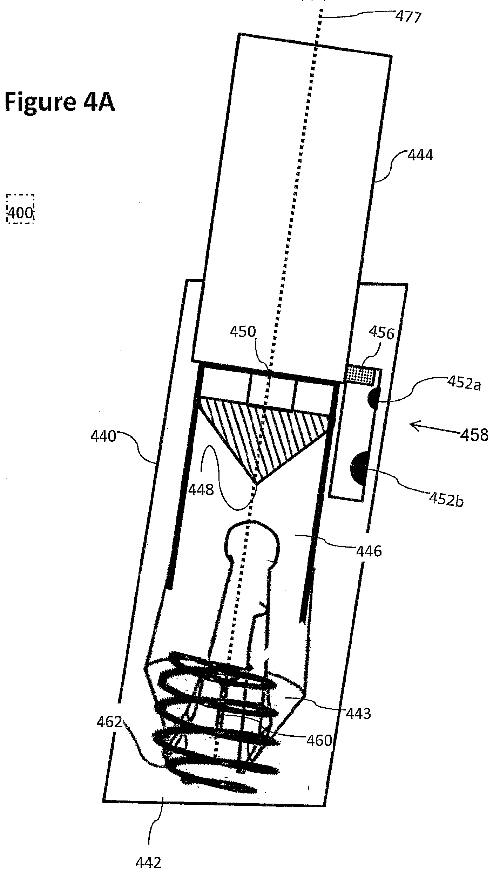

Figs. 4A-C are schematic illustrations of a drug delivery device including a needle stabilizing mechanism that clasps the skin of a patient in accordance with an exemplary embodiment **400** of the present invention. In exemplary embodiment **400,** a needle **460** is biased to a retracted state for example by a spring **462** (see for example Fig. 4A). In the retracted state, an optional clamp **443** may be biased in an open state.

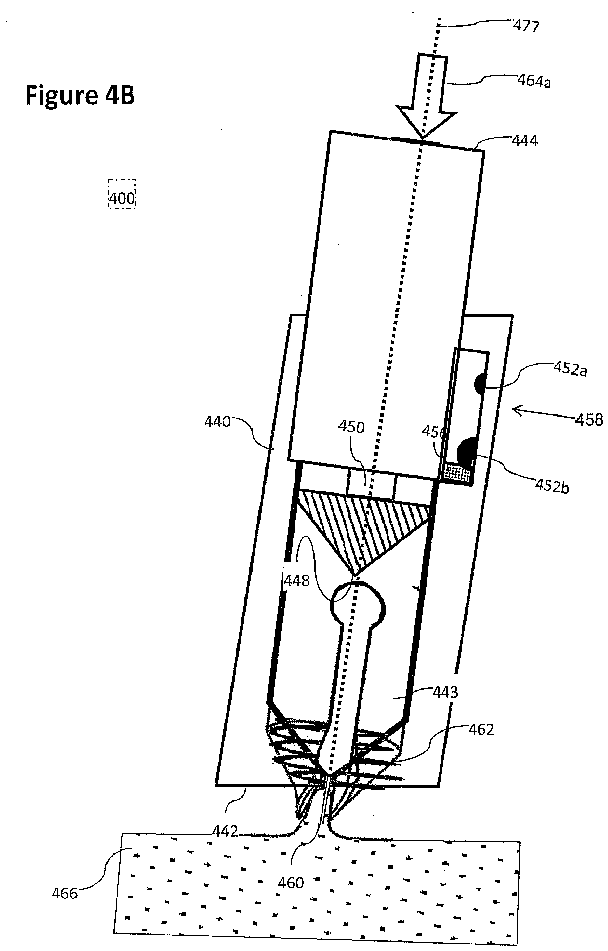

During injection (for example as illustrated in Fig. 4B) needle **460** is optionally held in an extended state by a driver **444** and/or a medicine container **446.** In the extended state, clamp **443** may close onto the skin of a patient holding the injector steady. For example needle **460** may be in fluid communication with medicine container **446.**

In some embodiments, at the end of injection driver **444** may retract and/or release medicine container **446** and/or the needle **460.** For example driver **444** may be unlocked. Once unlocked, driver **444** may optionally revert to a retracted state and/or clamp **443** may optionally be released and/or needle **460** may optionally retract back into the housing (for example as illustrated in Fig. 4D).

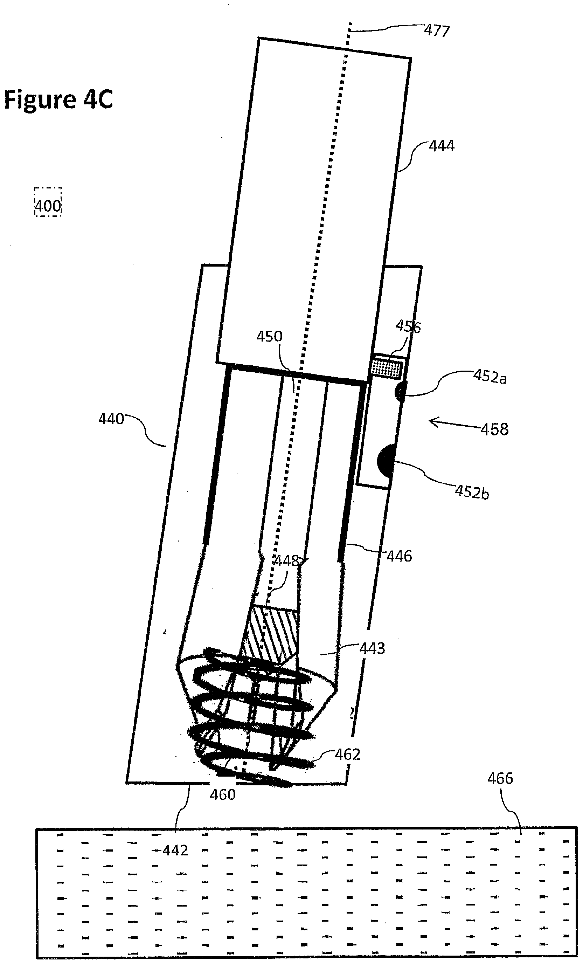

In some embodiments locking mechanism may include for example interference elements **452a,b** and/or a locking pin **456** to retain driver **444** in a retracted and/or extended position until a force is applied. For example pin **456** may be rigidly connected to driver **444.** In order to move driver **444** from the retracted position (for example as illustrated in Figs. 4A and 4C) to the extended position (for example as illustrated in Fig. 4B) or back, a force may be applied to push pin **456** past interference elements **452a,b.**

Figure 4A illustrates exemplary embodiment **400** in an enabled state prior to activation. For example, in the enabled state an optional safety cover and/or a sterile cover may and/or an adhesive protector may have been removed from the injector. In the enabled state a needle **460** is shielded by an activation zone on a base **442** of a housing **440.** Needle **460** is safeguarded by a retraction assembly including for example a spring **462** biasing the needle into housing **440** and a needle locking mechanism **458** and a driver **444** which hold needle **460** and its supporting medicine container **446** inside housing **440.**

Fig. 4B illustrates exemplary embodiment **400** in an activated state, for example right before and/or at the beginning of discharge of a medication. For example, embodiment **400** is activated by pushing driver **444** with a sufficient force **464a.** Pushing driver **444** optionally pushes needle **460,** medicine container **446,** and/or clamp **443** from the retracted position (illustrated in Fig. 4A) to the extended position (illustrated in Fig. 4B). Movement may, for example, be along the primary longitudinal axis **477** of medicine container **446.** Optionally, needle **460** is shown coaxial to medicine container **446.** Alternatively or additional a needle may be mounted off center of a syringe. As clamp **443** moves from the retracted to the extended position, it closes, grasping a skin **466** of the patient.

In some embodiment, once activated, driver **444** may apply a force on plunger rod **450** and/or plunger **448** to discharge medicine. Optionally driver **444** may be configured to drive discharge of the medicine over a relatively long period of time, for example between 30 to 120 seconds and/or between 120 to 600 seconds.

Figure 4C illustrates exemplary embodiment **400** at the end of discharge of a medicament. Plunger **448** has optionally reached the end of its path. For example, a locking mechanism **458** has been released. Unlocking locking mechanism **458** may optionally trigger releasing clamp **443** and/or safeguarding needle **460.**

### 5 Adhesive syringe stabilizer

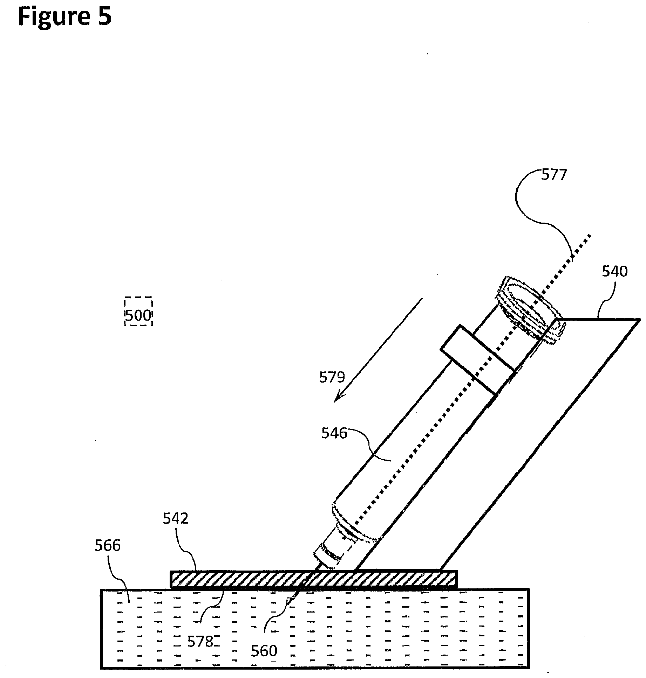

Fig. 5 illustrates a drug delivery device including an adhesive syringe stabilizer **500** in accordance with an embodiment of the current invention. Syringe stabilizer **500** includes a base **542** adhering to a skin of a patient. A syringe **546** is attached to base **542** by means of a housing **540.** Syringe **546** is rigidly connected to a needle **560** which protrudes from syringe **546** past base **542** into a skin **566** of a patient. In operation, needle **560** may be in fluid communication with syringe **546** and/or the patient. Needle **560** is shown in embodiment **500** mounted coaxially to syringe **546** (along the principal longitudinal axis **577** of syringe **546**). Optionally, needle **560** may be mounted to the syringe off center. In the present application, the term principle longitudinal axis of a drug container may be used to refer to the longest axis of symmetry of the drug container.

In some embodiments, an adhesive **578** may be attached to a distal surface of a base **542.** A housing **540** attached to a proximal side of base **542** may optionally hold syringe **546** with a needle **560** protruding from syringe **546** across base **542** (for example through a hole in base **542**).

In some embodiments, syringe **546** may be attached to base **542** prior to adhering base **542** to a patient. The entire assembly may be attached to the patent (piercing the skin **566** with needle **560** and inserting needle **560** into the skin until adhesive **578** contacts skin **566**).

In some embodiments, syringe **546** may be movably attached to housing **540.** For example, syringe **540** may slide longitudinally along housing **540.** Optionally, syringe **546** may be attached to housing **540** with needle **560** held proximal to base **542.** Then the distal surface of base **542** may be adhered to skin **566.** Subsequently, syringe **546** and needle **560** may be slide longitudinally in the distal direction **579** until needle **560** protrudes through base **542** into skin **566.** Once needle **560** has been inserted into skin **566** adhesive **578** may assist a user to steady syringe **546** as he discharges medicine into the patient (for example by pushing on a plunger).

In some embodiments, base **542** may first be attached to the patient. Then a user may hold syringe **546** in his hand and insert needle **560** through a hole in base **542** into the skin of the patent. A possible advantage of inserting needle **560** into skin **566** after attaching base **542** to skin **566** is that adhesive **578** may inhibit deformation of skin **566** during needle insertion. This may make it easier to control the precise depth of insertion. After needle **560** has been inserted syringe **546** may be attached to housing **540.** Adhesive **578** may assist a user to steady syringe **546** as he discharges medicine into the patient (for example by pushing on a plunger).

### 6 Detailed illustration of states of an injector

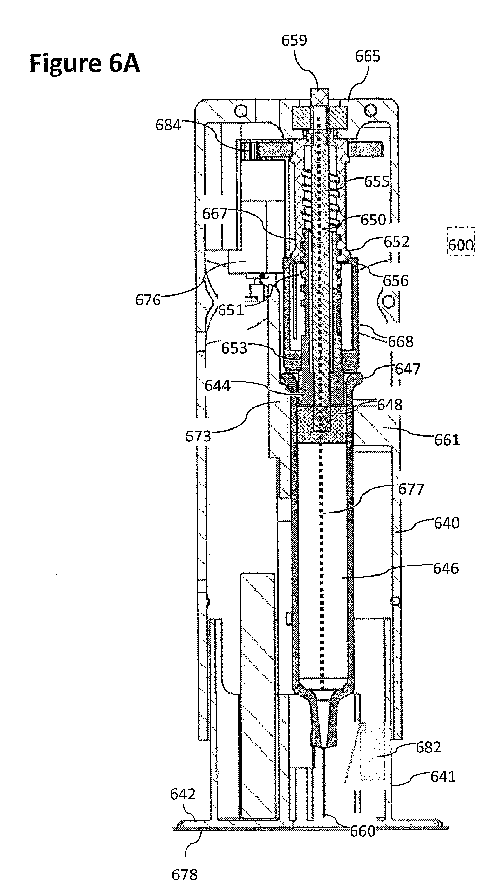

Figs. 6A-D include detailed cross sectional side views illustrating four states of an autoinjector drug delivery device in accordance with an embodiment of the present invention. In some embodiments, an injector **600** is an automated self injection device. For example the self injecting device may in some ways be similar to a pen injector. Optionally injector **600** may be loaded with a standard type syringe **646** and/or hypodermic needle **660.** For example, needle **660** may be rigidly connected and/or project from a distal end of syringe **646.** Needle **660** may be coaxial with syringe **646.** Alternatively or additionally the axis of needle **660** may be parallel to the primary longitudinal axis **677** of syringe **646** but offset therefrom. Syringe may be loaded into injector **600** with needle **660** in a sterile state and/or covered by a sterile cover.

In some embodiments, an injector may include for example an adhesive **678** base **642.** For example, adhesive **678** base **642** may assist a user to hold injector **600** steady on the skin of a patient for an extended period. For example, injector **600** may be used to give injections of volume ranging between 0.5 and 3.0 ml over a time period ranging between 30 sec to 180 sec.

In some embodiments Injector **600** may include a transmission. For example, the transmission may include a rotary screw converting rotational motion of a motor **676** to linear motion for sliding a plunger to discharge a drug and/or for retracting a needle. Alternatively or additionally, in some embodiments, linear motion of the transmission may also be used to insert a needle. For example, in injector **600,** the transmission includes for example an annular snap resistance element **652** paired to an annular driver support **656.** When a linear stress increases past a threshold, the annular snap gives way and a needle **660** may optionally be retracted to a protected location.

Fig. 6A is a schematic cross sectional side view illustrating injector **600** in an enabled state (ready for activation). For example, in the enabled state an optional safety cover and/or a sterile cover may and/or an adhesive protector may have been removed from the injector. In the enabled state needle **660** is in a protected location, created by a shield **641** which extends the distal end of a housing **640** of the injector. Needle **660** and/or shield **641** may optionally be retained in position, for example by a snap and/or held in position by a biasing device, for example a spring.

In some embodiments, needle **660** may optionally be supported by a syringe **646;** which is in turn supported for example by a cylindrical outer sleeve **668.** Outer sleeve **668** may optionally be supported by an annular support **656** resting on an annular snap resistance element **652.** For example annular snap resistance element **652** may extend radially outward from a cylindrical inner sleeve **667.** Optionally, inner sleeve **667** and/or outer sleeve **668** and/or a driver **644** may be operationally linked to a coupling **684** (for example including a gear) such that rotating coupling **684** rotates one or more of inner sleeve **667** and/or outer sleeve **668** and/or a driver **644.**

In some embodiments, syringe **646** position sensor, for example a motor switch **682** may be located in shield **641.** In the enabled state (before activation), switch **682** is optionally switched off.

In injector **600,** syringe **646** is held to outer housing **640** by a socket **661.** Socket **661** allows syringe **646** to slide axially with respect to housing **640** but not to move laterally. In injector **600,** coupling **684** is held rotatably fixed to housing **640** by bearing **659** in a hub **665.**

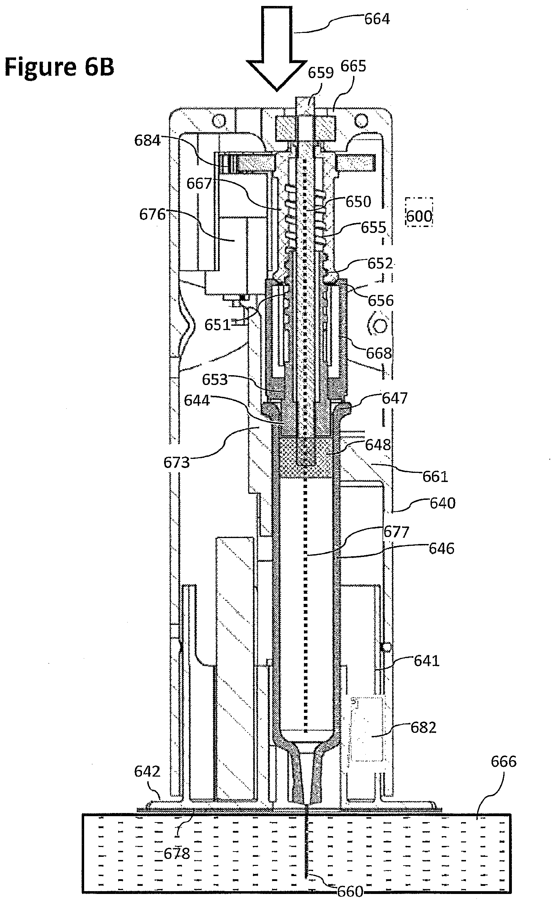

Fig. 6B is a schematic cross sectional side view illustrating injector **600** immediately after activation. For example, to activate the injector, a user may place the distal end of the injector (including for example an adhesive **678** and/or an activation zone on base **642**) against the skin **666** of a patient and/or push **664** on the proximal end of the injector until shield **641** collapses into housing **640** in a direction parallel to the longitudinal axis of needle **660.** Collapse of shield **641** may optionally unshield needle **660** tip which may for example be pushed into the skin **666** of the patient. For example, in operation, needle **660** may protrude from injector **600** into a patient. Optionally, in operation, needle **660** may be in fluid communication with syringe **646** and/or the patient. For example needle **660** may supply a fluid pathway for discharging medicine directly from syringe **646** through needle **660** into the patient.

In some embodiments, upon collapse of shield **641** switch **682** senses movement of syringe **646** to the active position and/or activates motor **676.** For example in injector **600** switch **682** is depressed by being pushed against syringe **646.** Depressing switch **682** may activate motor **676** to power a transmission and/or linear movement of a plunger **648** for discharging a drug. For example, in injector **600** motor **676** turns a coupling **684.** Coupling **684** may include for example a gear. Coupling **684** may optionally rotate inner sleeve **667** and/or driver **644.** In exemplary injector **600,** driver **644** includes teeth and/or threads which engage a screw thread **653** on a plunger rod **650.** Rotating driver **644** may optionally drive plunger rod **650** and/or plunger **648** in the distal direction, discharging the medicine. Optionally, plunger **648** continues to move distally until it is stopped by for example a blockage in the fluid path (preventing further discharge) and/or until plunger **648** reaches the distal end of syringe **646.** Optionally, when needle **660** is in the extended position, a flange **647** of syringe **646** seats against a bracket **673,** which holds syringe **646** and/or prevents further longitudinal movement.

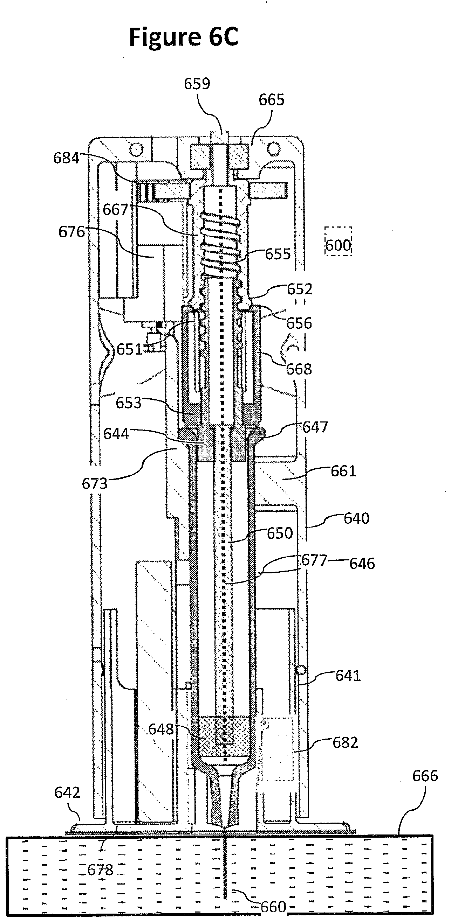

Fig. 6C is a schematic cross sectional side view illustrating injector **600** at the end of discharge of the payload. For example plunger **648** has discharged all of the medicine out of syringe **646** and/or has reached the distal end of syringe **646.** Optionally, further rotation of driver **644** increases the stress pushing driver **644** proximally. Interference element **652** may serve as stress sensor. For example, motor **676** may supply enough torque to create a force which overcomes interference element **652.**

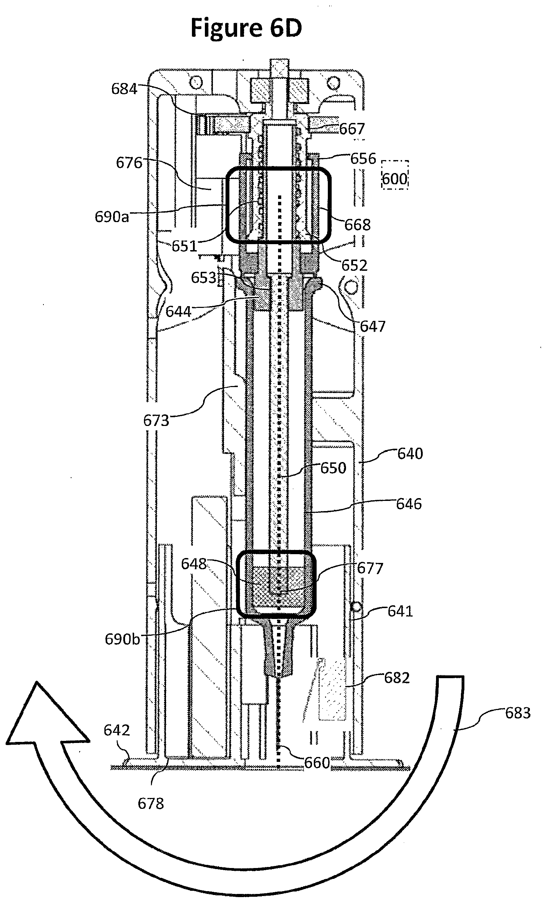

In some embodiments, once interference element **652** is overcome a course inner threading **655** in sleeve **667** rotates with respect to a course outer thread **651** of driver **644** drawing driver **644** and/or plunger **648** and/or syringe **646** and/or needle **660** proximally into a retracted state. For example, the course thread **651** has an opposite threading from the threading **653** between driver **644** and plunger rod **650.** The same direction of rotation that drives plunger **648** distally before overcoming interference element **652** also draws back plunger **648** and/or syringe **646** and/or needle **660** proximally after overcoming interference element **652.** Optionally needle **660** is retracted into a protected location inside housing **640** for example as illustrated in Fig. 6D. Alternatively or additionally, course thread **651** may have an the same direction of threading as threading **653** between driver **644** and plunger rod **650** and optionally rotation may be reversed to retract needle **660.**

Fig. 6D is a schematic cross sectional side view illustrating injector **600** in a safe state after finishing injection. Needle **660** point has optionally been retracted into a protected location within housing **640.** Syringe **646** has optionally been retracted. In exemplary injector **600,** switch **682** senses that syringe **646** is retracted, as syringe **646** no longer depresses switch **682.** Switch **682** may be biased off and/or raising syringe **646** may shut off motor **676.**

In some embodiments one or more windows may be supplied. A user may be able to determine a status of the device by viewing for the windows. For example in Fig. 6D, injector **600** has been supplied with two windows **690a,b.** For example window **690a** is located such that during injection, the user views inner sleeve **667** through window **690a.** When outer sleeve **668** has been retracted, it may optionally slide over inner sleeve **667.** After outer sleeve **668** has been retracted, the user views outer sleeve **668** through window **690a.** Optionally window **690a** may serve as an indicator whether it is safe to remove the injector. For example, outer sleeve **668** may be colored green and/or driver **644** and/or inner sleeve **667** may be colored red. For example, as long as the user sees red in window **690a** needle **660** tip has not been retracted and/or it is unsafe to remove the injector from the patient's skin; and/or when the user views green through window **690a** needle **660** has been retracted and/or discharge has ceased and/or it is safe to remove the injector from the skin of the patient. Optionally, window **690b** may be used to indicate whether an entire payload of medicine has been administered. For example, syringe **646** may be made of a transparent material. For example, during injection, the user can see the medicine through window **690b;** after syringe **646** is retracted if the payload has been fully discharged then the user will view plunger **648** through window **690b.** Optionally, if the user sees plunger **648** through both window **690b** and outer sleeve **668** through window **690a** then the user can ascertain that it is safe to remove the injector and/or that the drug was fully discharged.

In injector **600,** for example, after retraction of the needle the device may be twisted such that one side of the adhesive is lifted and/or pivoted and/or peeled (as illustrated by arrow **683** in Fig. 6D) from the skin while the far edge of the base of the injector remains in contact with the skin and serves as a fulcrum.

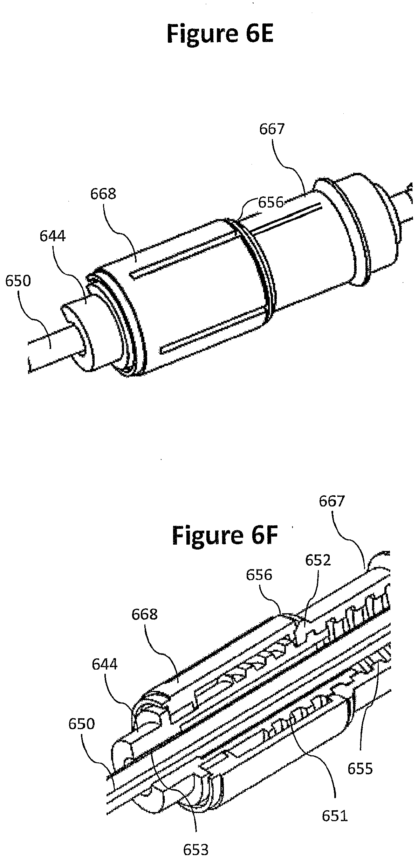

Figs. 6E and 6F illustrate an external view and cut away view respectively of a transmission for driving discharge and retracting a needle in accordance with exemplary embodiment (for example injector **600**) of the current invention. Injector **600** may optionally be designed such that, under sufficient linear stress, support **656** of external sleeve **668** deforms and/or opens to pass over resistance element **652.** Optionally, coupling **684** and/or inner sleeve **667** may be formed of one or more pieces of molded plastic. Optionally outer sleeve **668** and/or driver **644** may be formed of one or more pieces of molded plastic.

Fig. 6F, illustrates details of a rotary needle retractor in accordance with an embodiment of the current invention. Fig. 6F illustrates driver **644** before needle retraction (for example in a secured state, an enabled state and/or an active state). In the exemplary embodiment, driver **644** is engaged by a set of fine screw threads **653** to rod **650.** In the exemplary embodiment, driver **644** is engaged by a set of course screw threads **651, 655** to inner sleeve **667.** Optionally, course screw threads **651, 655** are threaded in an opposite sense from fine screw threads **653.**

In the exemplary embodiment, prior to needle retraction, sleeve **667, 668** and driver **644** are prevented from sliding longitudinally with respect one another. While sleeves **667, 668** and driver **644** are prevented from relative longitudinal movement, threads **651** and **655** prevent inner sleeve **667** and driver **644** from rotating with respect to one another.

In some embodiments, motor **676** drives coupling **684** to rotate inner sleeve **667.** Optionally, before needle retraction, rotating inner sleeve **667** rotates driver **644.** The sense of screw threads **653** and the rotating direction of motor **676** are optionally chosen such that rotating driver **644** relative to rod **650** pushes rod **650** and/or plunger **648** distally, optionally discharging a drug.

When plunger **648** has reached the distal end of syringe **646,** rod **650** is prevent from further distal movement. Torque applied to driver **644** produces a strong proximal stress on driver **644** and/or outer sleeve **668.** The strong proximal stress overcomes and/or releases interference element **652.** Once interference element **652,** is released outer sleeve **668** and/or driver **644** can move longitudinally with respect to inner sleeve **667.** Further rotation of inner sleeve **667** rotates sleeve **667** with respect to driver **644.** The sense of screw threads **655** and **651** and the rotating direction of motor **676** are optionally chosen such rotating driver **644** relative to sleeve **667** draws driver **644** and/or rod **650** and/or plunger **648** and/or syringe **646** and/or needle **660** proximally, optionally retracting needle **660.** Optionally the pitch of screw threads **651, 653** and/or **655** can be tuned to achieve a desired rate of medicine discharge and/or needle retraction for a given rotation rate of the motor. In some embodiments, as rod **650** and/or plunger **648** are drawn proximally, friction between plunger **648** and syringe **646** draws syringe **646** and/or needle **660** proximally. Alternatively or additionally, outer sleeve **668** may be attached to syringe **646.** Drawing back on driver **644** may draw outer sleeve **668** and syringe **646** back with it. In some embodiments additional threaded elements may be added to produce a multi-part telescoping assembly for extending plunger **648** to discharge medicine and/or for retracting needle **660.** In some embodiments some or all of rod **650,** inner sleeve **667,** and/or outer sleeve **668** and/or coupling **684** may be formed of molded plastic and or other materials.

### 7 Stabilized pen injector

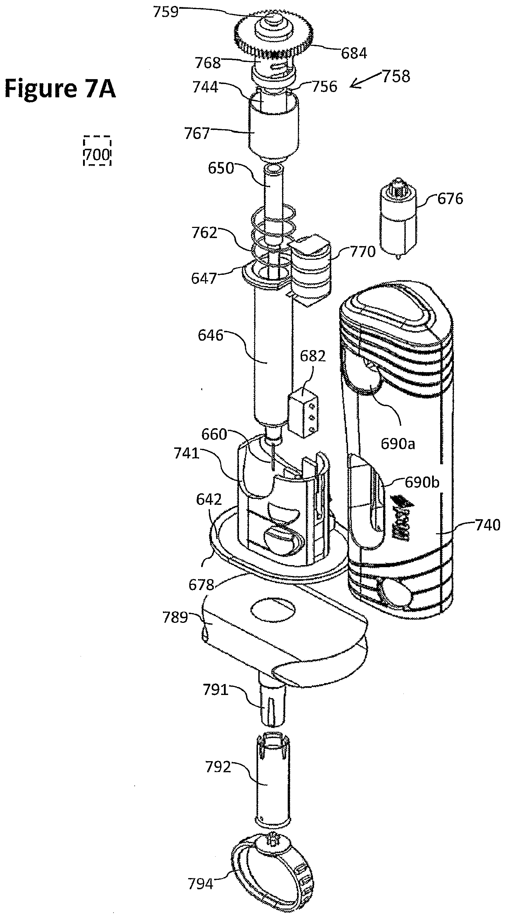

Figs. 7A-H illustrate a drug delivery device including a stabilized injector **700** in accordance with some embodiments of the present invention. Exemplary injector **700** is an automated injection device in some ways similar to a pen injector. Optionally injector **700** may be loaded with a standard type syringe **646** and/or hypodermic needle **660.** Optionally syringe **646** may be supplied loaded with medicine and/or covered with a sterile needle cover **791.** Syringe **646** may be loaded into injector **700** with in a sterile state with needle cover **791** in place. Injector **700** may include for example an adhesive **678** base **642.** In some embodiments, adhesive **678** base **642** may assist a user to hold injector **700** steady on the skin of a patient for an extended period. For example, injector **700** may be used to give injections of volume ranging between 0.5 and 3.0 ml over a time period ranging between 30 sec to 180 sec.

Fig. 7A illustrates an exploded view of injector **700.** Some components of the exemplary embodiment of injector **700** which are similar to corresponding parts of the exemplary embodiment of injector **600** are marked with the same number as the corresponding parts of the exemplary embodiment of injector **600.**

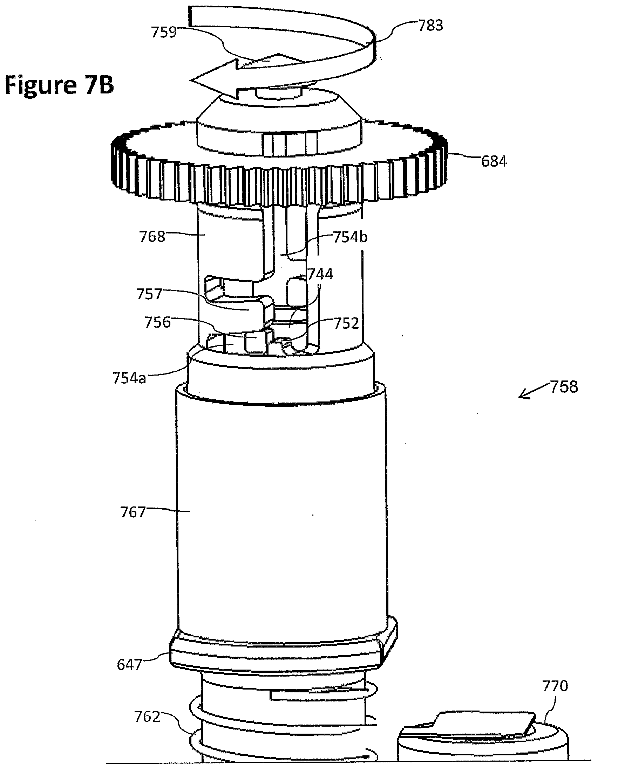

In the exemplary embodiment of injector **700** a power supply (for example batteries **770**) may optionally supply power to gear motor **676.** Figs. 7A,B illustrate flange **647** of syringe **646.** Optionally flange **647** has at least one non-rounded edge which may be held inside an autoinjector (for example autoinjectors **400, 600** and/or **700**) preventing rotation of syringe **646.** Outer housing **740** and/or shield **741** of injector **700** are similar to outer shell **640** and/or shield **641** of injector **600.**

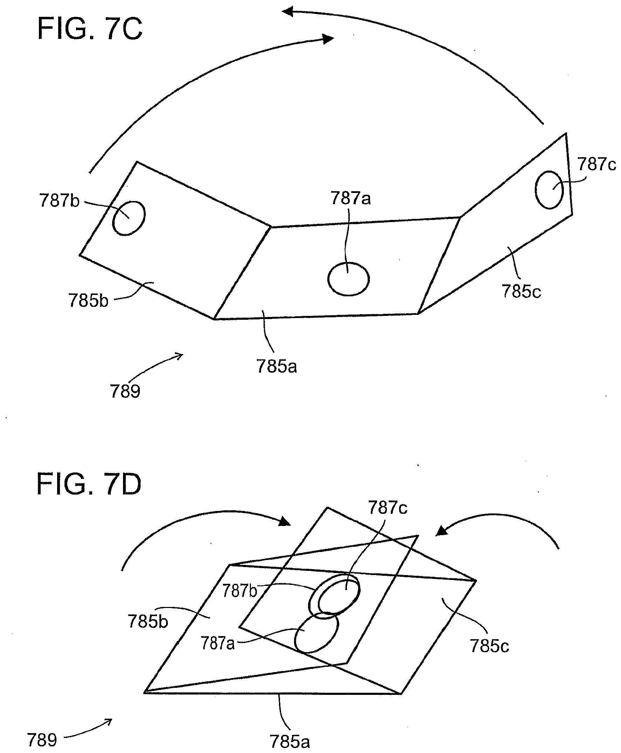

Some embodiments of a stabilized autoinjector (for example as illustrated in injector **700** but optionally included in injectors **600** and/or embodiments **400** and/or **200** and/or **100**) may include a safety cover and/or an adhesive protector and/or a handle. Details of an exemplary embodiment of an adhesive protector **789** and/or a handle **794** and/or a needle cover remover, for example a safety cover **792** are illustrated in Figs. 7C-H.

Fig. 7B illustrates an exemplary transmission including a retraction mechanism **758.** Retraction mechanism **758** is optionally activated by a combination of torque and linear stress. For example retraction mechanism **758** is optionally activated may when plunger **648** is blocked for example when it reaches the end of injection (for example as described in regards to Figs. 6A-D and/or due to an occlusion of needle **660**).

In some embodiments, during drug discharge a motor (for example motor **676**) rotates coupling **684** in the direction of arrow **783.** Coupling **684** may optionally be rigidly connected to and/or integrally molded with inner sleeve **768.** Rotating coupling **684** may also rotate inner sleeve **768.** A pin **756** protrudes from driver **744** into a nearly lateral slot **754a** in sleeve **768.** While pin **756** is in slot **754a,** driver **744** is prevented from moving longitudinally with respect to inner sleeve **768.** In some embodiments syringe **646** is supported (from moving proximally) by driver **744.**

In some embodiments, when there is a strong linear force on driver **744** in the proximal direction and/or there is a strong torque on sleeve **768** in the direction of arrow **783,** arm **757** is deflected upward and pin **756** slides past an interference element **752** into a longitudinal slot **754b.** In slot **754b** pin **756** may slide longitudinally (in the proximal direction). A geometry of pin **756** and/or interference element **752** may be chosen to achieve a desired resistance to movement. For example, pin **756** and/or interference element **752** may have a squared side, a flat side, a rounded side etc.

In some embodiments, a spring (for example spring **762**) biases syringe **646** in the proximal direction. For example spring **762** may apply a proximal force to flange **647.** Optionally another biasing element may be used in place of spring **762.** For example, a biasing element may include a stretched element (for example a rubber band and/or a twisted elements and/or a deflected plastic element).

Optionally when pin **756** enters longitudinal slot **754b,** spring **762** pushes syringe **646** and/or outer sleeve **767** and/or needle **660** and/or driver **744** and/or pin **756** proximally, retracting needle **660.** Optionally, needle **660** may be held in the retracted position by spring **762.** Alternatively or additionally a locking mechanism may be included to lock needle **660** in the retracted position, for example, a one way catch and/or an interference element may lock against syringe **646** as it is retracted and/or against pin **756** in slot **754b.** Optionally, in injector **700** driver **744** includes two molded plastic telescoping pieces. One piece is optionally integrally molded with outer sleeve **767.** Optionally, sleeve **767** and/or driver **744** may be made as a single piece and/or multiple parts. They may be formed of plastic and/or another material and/or they may be molded and/or formed by another process.

Figs. 7C and 7D illustrate an exemplary method of folding adhesive protector **789.** For example, adhesive protector may include three folded portions **785a, 785b,** and **785c.** For example, adhesive protector may include three holes **787a, 787b,** and **787c.** When folded, the side portions **785b,c** may fold over the center portion **785a.** Side holes **787c,b** may line up with center hole **787a.** Alternatively or additionally, an adhesive protector may have only one fold and/or one side portion and/or peeling may be from one side only.

In some embodiments, portion **785a** will be adhered to base **642** with the needle opening accessible through holes **787a-c.** A safety cover (for example cover **792**) may protrude through holes **787a-c.** The safety cover may optionally be connected to portions **785b,c.** The safety cover may pass freely through hole **787a** and/or not be connected to portion **785a** of adhesive protector **789.**

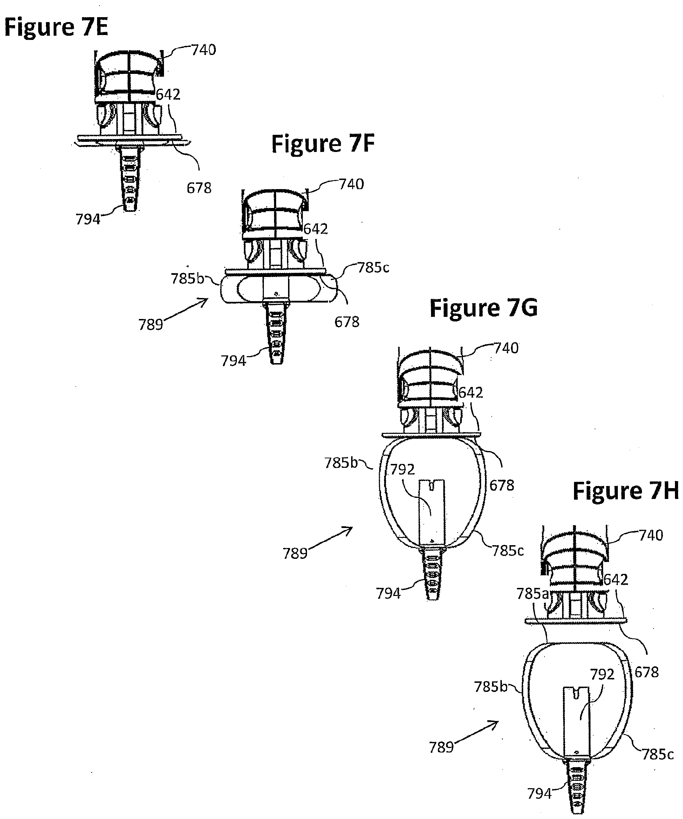

Figs. 7E-H illustrate removal of an exemplary safety cover **792,** needle cover **791** and/or adhesive protector **789.** For example, while safety cover **792** is mounted to needle cover **791,** safety cover may prevent deployment and/or activation of the injector. For example, safety cover **792** and handle **794** may supply a convenient means of removing needle cover **791** and/or adhesive protector **789.**

Fig. 7E illustrates injector **700** in a safe state for storage and/or transport. Needle cover **791,** safety cover **792** and adhesive protector **789** are in place. Adhesive protector **789** is folded for example as illustrated in Figs. 7C,D. Needle cover **791** (not seen in the drawing) may optionally preserve the sterility of needle **660.** Safety cover **792** surrounds and grasps needle cover **791.** Safety cover **792** may prevent inadvertent activation of the injector and/or protect users from a needle stick hazard.

Fig. 7F illustrates the beginning of removal of safety cover **792.** A user pulls handle **794** away from needle **660.** Handle **794** pulls needle cover **791** out the needle hole and/or needle aperture of injector **700** and through hole **787a.** As cover **792** is' pulled away from base **642,** portions **785b,c** of adhesive protector unfold while portion **785a** remains adhered to base **642.**

Fig. 7G illustrates that as safety cover is pulled further away from base **742** portions **785b,c** of adhesive protector **789** pull and/or pivot and/or peel portion **785a** away from base **742.**

Fig. 7H illustrates cover **792** and protector **789** fully removed from injector **700,** such that injector **700** is enabled and/or ready to adhere to a patient and/or ready for activation.

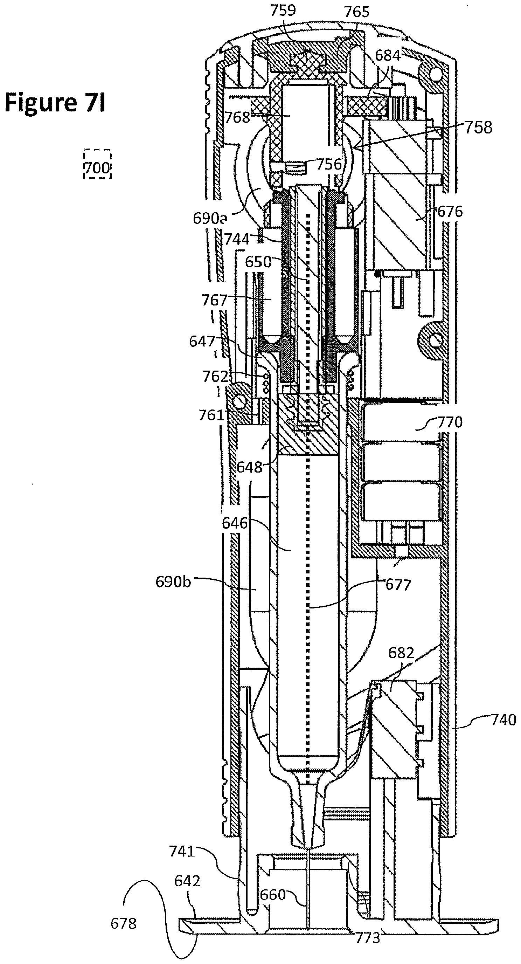

Fig. 7I illustrates exemplary embodiment 7I assembled and/or in an enabled state before insertion of needle **660** into a patient. Figure 7I illustrates various optional details and/or supporting structures for syringe **646** and/or plunger.

In injector **700,** syringe **646** is held to outer housing **740** by a socket **761.** Socket **761** allows syringe **646** to slide axially with respect to housing **740** but not to move laterally. In injector **700,** coupling **684** is held rotatably fixed to housing **740** by bearing **759** in a hub **765.**

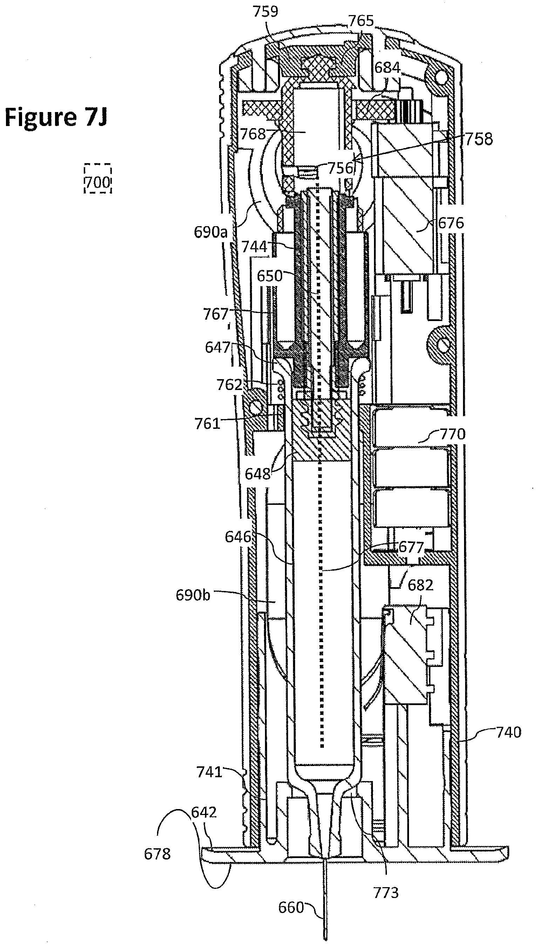

Fig. 7J illustrates exemplary injector **700** in an active state. For example when the injector is in the enabled state, a user may place adhesive against the skin of a patient and push downward (distally) on housing **740.** Housing **740** and its contents (for example syringe **646,** coupling **684,** locking assembly **758** etc.) along with needle **660** are all pushed distally along the axis of needle **660.** As needle **660** moves distally, the needle tip passes through a hole in shield **741.** For example, in operation, needle **660** may protrude from injector **600** into a patient. Optionally, in operation, needle **660** may be in fluid communication with syringe **646** and/or the patient. For example needle **660** may supply a fluid pathway for discharging medicine directly from syringe **646** through needle **660** into the patient.

In some embodiments, when needle **660** is in the extended position, the front end of syringe **646** seats into a bracket **773.** Bracket **773** may optionally hold syringe **646** steady and/or prevents further longitudinal movement and/or prevent lateral movement with respect to housing **740.**

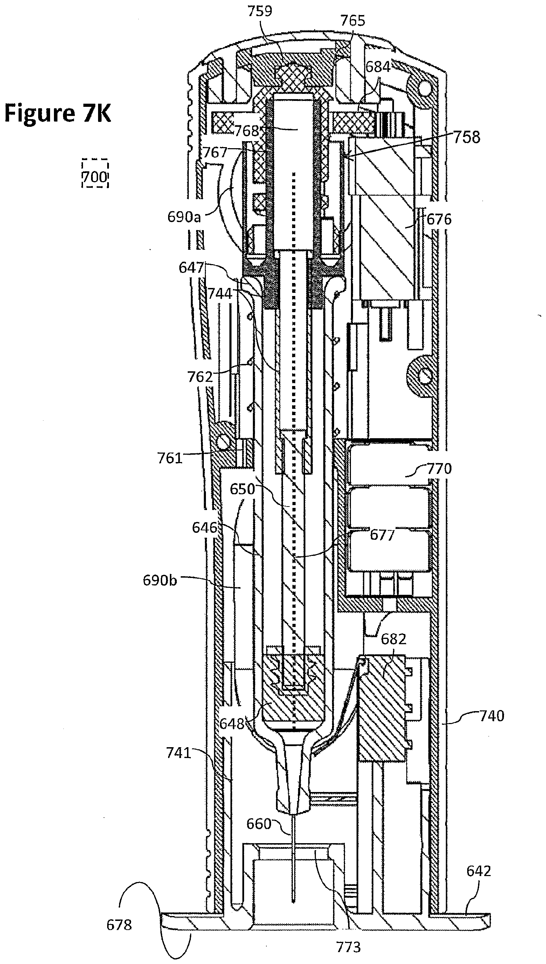

Fig. 7K illustrates injector **700** after needle retraction. Optionally driver **644** includes a telescopic assembly, which is shown in an extended state in Fig. 7K. Optionally, after retraction of needle **660,** the entire device may be twisted to pivot and/or peel adhesive **678** from the skin.

Various aspects or features illustrated herein with respect to a particular embodiment may be combined with other embodiments. For example, needle **460** and **560** of embodiment **400** and **500** are shown mounted at an angle to base **442** or **542.** Alternatively or additionally they may be perpendicular to the base. For example, needles **660** of embodiments **600** and **700** are shown perpendicular to base **642.** Alternatively or additionally they may be at an angle to the base. Needle covers and/or protective covers illustrated in one embodiment may be used with another embodiment. Retraction mechanisms illustrated in one embodiment may be used with another embodiment. A clip, an interference element, a catch and/or another locking mechanism may hold an injector in one or another state. For example an interference element may hold a needle in a retracted position and/or in the extended position.

### 8 Method of manufacture of a stabilized pen injector

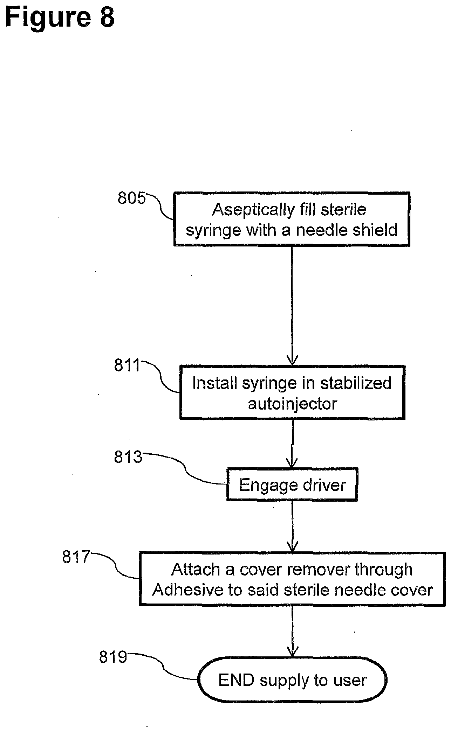

Fig. 8 is a flow chart illustration of a method of manufacture of a stabilized pen injector in accordance with an embodiment of the present invention. In the method a preloaded sterile syringe and needle may optionally be installed into an autoinjector for direct injection into a patient. For example the method of manufacture illustrated in Fig. 8 may optionally be used in manufacturing one, some and/or any of the embodiments of an injector illustrated herein above and/or below.

In some embodiments a syringe is preloaded **805** with a medicine. For example the volume of preloading medicine may range between 0.5 and 1ml and/or between 1 and 5 ml and/or greater than 5 ml of medicine. Preloading **805** may optionally be performed on standard syringe equipment and using standard filling procedures. Optionally the syringe may be a standard type syringe. For example preloading may be done in an aseptic environment. Optionally, a sterile needle and/or needle cover may be attached before filling the syringe. For example, the syringe may be filled while attached to a sterile needle and/or a sterile needle cover. Alternatively or additionally, the syringe may be attached to a sterile needle and/or a sterile needle cover while being filled and/or after being filled. Sterility of the needle may optionally be protected by a needle cover (for example cover **791**).

In some embodiments, the preloaded syringe with the sterile, protected needle may be installed **811** in an autoinjector including an adhesive stabilizer (for example one of the autoinjectors described herein above). Optionally, installing **811** the syringe may include engaging **813** a driver to a power source, for example a battery (for example directly and/or via a motor and/or coupling and/or a transmission) and/or a mechanical power source for example a spring.

In some embodiments, the fluid path of the injector may include the medicine container and/or the needle. For example, in operation, medicine stored in the container may pass directly from the container to the needle and/or from the needle directly to the patient. Optionally, the entire fluid path may be in a complete and/or sterile and/or assembled and/or protected state prior to and/or during filling of the container. For example, a syringe medicine container and/or needle may be filled and sealed under aseptic and/or sterile conditions, for example in an aseptic room. For example the syringe may be sealed by a needle cover and/or a plunger. Optionally, the syringe, with the fluid path in a sealed and/or protected state may be taken from the aseptic filling room and installed into an injector. Optionally, the fluid path may not require sterilization after being removed from the filling room and/or after installation into the injector.

In some embodiments, a cover remover (for example safety cover **792**) may be attached **817** to the needle cover through a hole in the adhesive. In some embodiments, the hole in the adhesive may be surrounded by the adhesive. Alternatively or additionally, the hole may be surrounded by the adhesive on two sides and/or on three sides. For example, the adhesive may be made of two pieces, one piece on one side of the hole and another piece on another side of the hole. The autoinjector may then be ready to be supplied **819** to a user. For example the user may use the cover remover to remove the needle cover and/or to enable the injector.

### 9 Stabilized injector dimensions

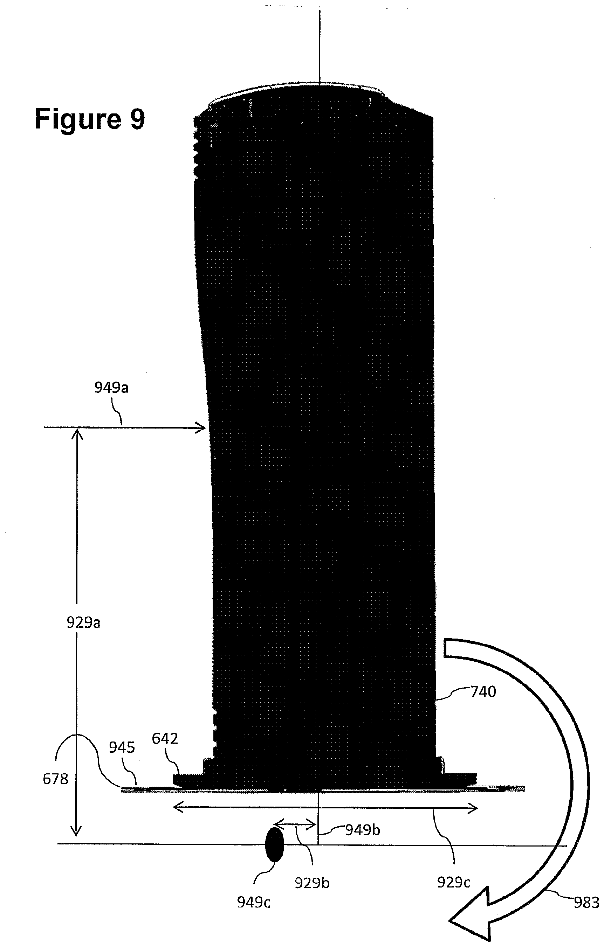

Fig. 9 illustrates an external view of a stabilized injector drug delivery device in accordance with an embodiment of the current invention. In Fig. 9 exemplary dimensions are shown to help understand the relationship between the size and weight of the injector and the strength and geometry of the adhesive. For example, any, some and/or all of the features and/or dimensions described herein below may apply to any, some and/or all of the embodiments described herein above.

In some embodiments, the height of an autoinjector (perpendicular to the adhesive layer) may be greater than then width of the adhesive layer (for example the height may be greater than the greatest length between any two points on the adhesive layer). For example, the distance **929a** from the longitudinal center of mass **949a** of the injector and the adhesive surface **678** may range for example between 50 ± 10 mm and/or the longitudinal center of mass may range for example between 60 and 80 mm from the adhesive. In some embodiments, the longitudinal center of mass may be greater than for example between 80 mm from the adhesive. The distance **929b** between the lateral center of mass **949b** of the injector and the center of adhesion **949c** on the base of the apparatus (when the weighted center of force on the adhesive when the injector is twisted off the skin in the direction of the arrow **983** in Fig. 9) may range, for example between 12.5 ± 4 mm. The width **929c** of base **642** may range, for example, between 60 ± 15 mm. There may optionally be a semi-stiff skirt **945** extending beyond the edge of base **642** for example between 0 to 2 mm and/or embodiments skirt **945** may extend for example between 4 to 10 mm beyond base **642.** In some embodiments, the width of skirt **945** may vary at various points around base **645.** (For example the skirt may be made of plastic, for example Polyethylene terephthalate (PET) and/or Polycarbonate and/or ABS. The thickness may range for example between 0.1 to 0.8mm.). The thickness of adhesive layer **678** may range between 0.1 and 1mm. An injector may weigh for example 50 ± 20 g. Then the resting torque adhesive when the injector is adhered to a vertical object will be approximately 50 mm × 50 g = 2500 g×mm. The strength of adhesion necessary to hold the injector to the patient will be approximately 2500 g×mm/12.5mm = 200 g. In some embodiments, movements of the user may place a considerably stress on the injector than the static stress. For example an adhesive may be provided to give a total adhesive strength ranging between 500 to 1500 g.

In some embodiments, the adhesive will be less strong and/or maybe easier to remove. For example the strength of the adhesive may be less than 500 g (for example the user may have to hold the injector with his hand to prevent it from falling, especially when the user is moving). Alternatively or additionally the adhesive may not include semi-stiff skirt **945.**

### 10 Microswitch activator for an autoinjector

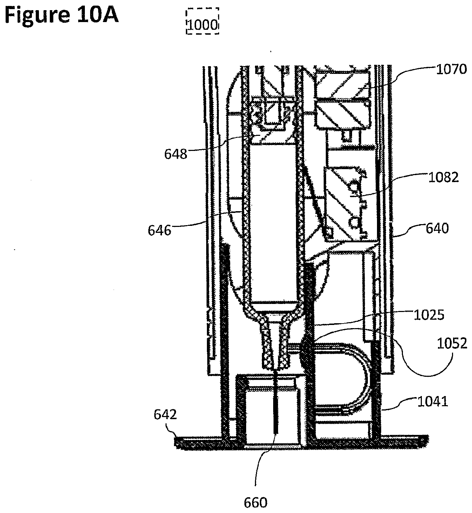

Figures 10A-D is are cutaway illustrations of an exemplary embodiment of an autoinjector **1000** drug delivery device including a motor switch located in the main body of the injector in accordance with some embodiments of the current invention. Optionally the motor switch remains stationary with respect to the housing of the autoinjector, for example housing **640,** and/or with respect to and/or a motor and/or a power source, for example batteries **1070.** When an activator of the injector is triggered (for example by collapsing a shield **1041**), movement of the activator may activate the switch. When the needle **660** is retracted and/or shielded (for example after injection), the switch is optionally returned to the inactivated state. Keeping a switch stationary with respect to a motor and/or power source may simplify assembly of the auto injector.

FIG 10A illustrates injector **1000,** prior to activation in accordance with an embodiment of the current invention. Optionally many structures are the same as other embodiments listed herein above or below, for example, injector **1000** may include the advancement and/or retraction mechanism of injector **600.** Optionally, prior to activation, a syringe position sensor, switch **1082** senses that shield **1041** is in a deactivated position. When shield **1041** is in a deactivated position, switch **1082** optionally deactivates the motor of injector **1000.**

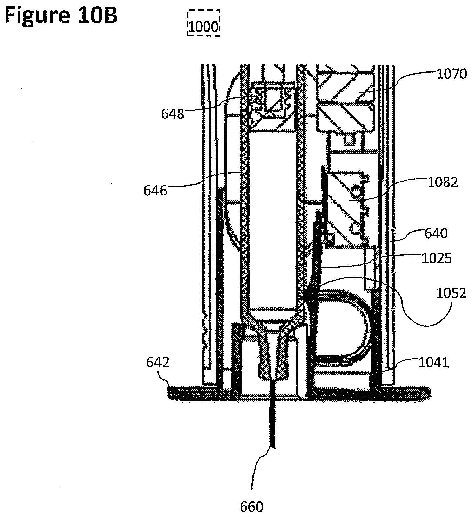

FIG 10B illustrates injector **1000,** immediately after activation in accordance with an embodiment of the current invention. In some embodiments, an injector may be activated by collapsing a needle shield **1041.** Collapsing shield **1041** optionally exposes needle **660.** Optionally the syringe position sensor (switch **1082**) senses the new position of shield **1041.** For example, shield **1041** pushes an element **1052** against a side of a syringe and/or pushes an extension **1025** of shield **1041** into switch **1082** and/or activates switch **1082** and/or activates a motor (for example similar to motor **676** of injector **600**).

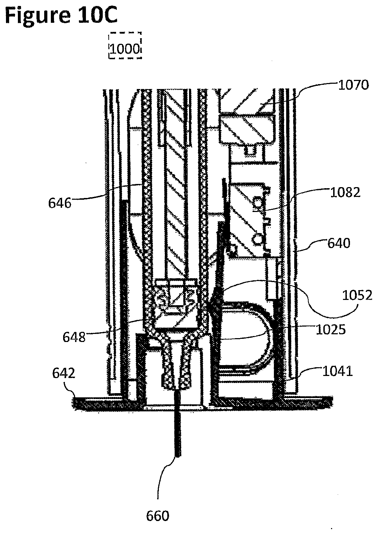

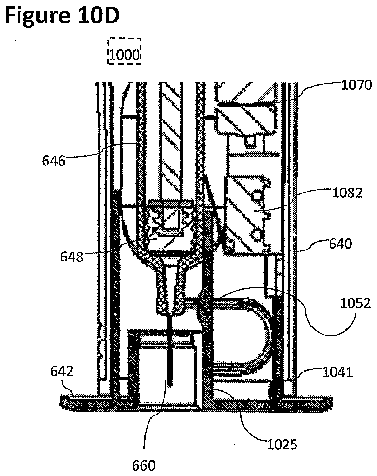

FIG 10C illustrates injector **1000**, immediately at the end of injection in accordance with an embodiment of the current invention. Optionally for as long as shield **1041** remains in a collapsed state and/or needle **660** remains in an extended state (protruding from the injector), extension **1025** continues to hold switch **1082** in an activated state and/or a motor continues to drive plunger **648.** Optionally, when plunger **648** reaches the end of syringe **646** a retraction mechanism (for example retraction mechanism **758**) retracts syringe **646** and/or needle **660** to a retracted state (as illustrated for example in FIG. 10D).

FIG. 10D illustrates injector **1000** in a retracted state in accordance with an embodiment of the current invention. Optionally, when needle **660** and/or syringe **646** is retracted, element **1052** is released and/or extension **1025** moves away from switch **1082** and/or switch **1082** moves into a deactivated state and/or the motor is switched off.

### 11 Method of stabilizing an autoinjector

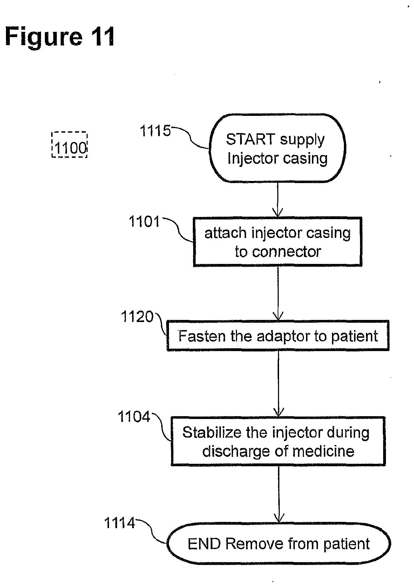

Figure 11 is a flow chart illustration of a method **1100** of adapting an injector for extended use in accordance with an embodiment of the present invention. For example an injector may be attached to a stabilizing adaptor. Stabilization may extend use of the injector to longer injection time and/or higher viscosity medicines and/or colder conditions.

In some embodiments, an injector housing may be supplied **1115.** Optionally the housing is attached **1101** to a connector. Optionally, the housing may be attached **1101** to the connector prior to assembly of autoinjector and/or prior to loading the autoinjector with medicine. For example, the autoinjector may be attached to the connector by a drug company and/or by a medical device supplier. Alternatively or additionally the injector housing may be supplied as part of an assembled autoinjector unattached to the connector. For example patient and/or a medical professional and/or a patient aid may attach **1101** the housing to the connector before use.

In some embodiments, a stabilizing adaptor may be fastened **1120** to a patient. Optionally, after attaching **1101** the injector to the adaptor, the entire assembly is fastened **1120** to the patient. For example, a patient and/or a medical professional and/or a patient aid may be supplied with a fully assembled injector and adaptor. For example the patient and/or the medical professional and/or the patient aid may fasten **1120** the assembled injector and adaptor to the patient and perform the injection. Alternatively or additionally, the adaptor may be fastened **1120** to the patient without the injector and then the injector may be attached **1101** to the adaptor while the adaptor is already fastened **1120** to the patient. For example, the adaptor may be fastened **1120** to the patient by a medical professional and/or patient aid and/or in a clinic and/or in a hospital. Later on the injector is optionally attached to the adaptor and/or enabled and/or activated, for example by the patient himself and/or a medical aid for example at home or elsewhere away from medical supervision.

In some embodiments, during discharge of the medicine, the autoinjector may be stabilized **1104** on the injection site of the patient by the adaptor.

In some embodiments, after injection ends, the injector and/or the adaptor may be removed **1114** from the patient. For example, the injector and the adapter may be removed **1114** together. Alternatively or additionally the injector may be disconnected from the adapter and removed from the patient while the adapter remains fastened to the patient, to be used for another injection and/or removed **1114** later.

### 12 States of an autoinjector and extending adaptor

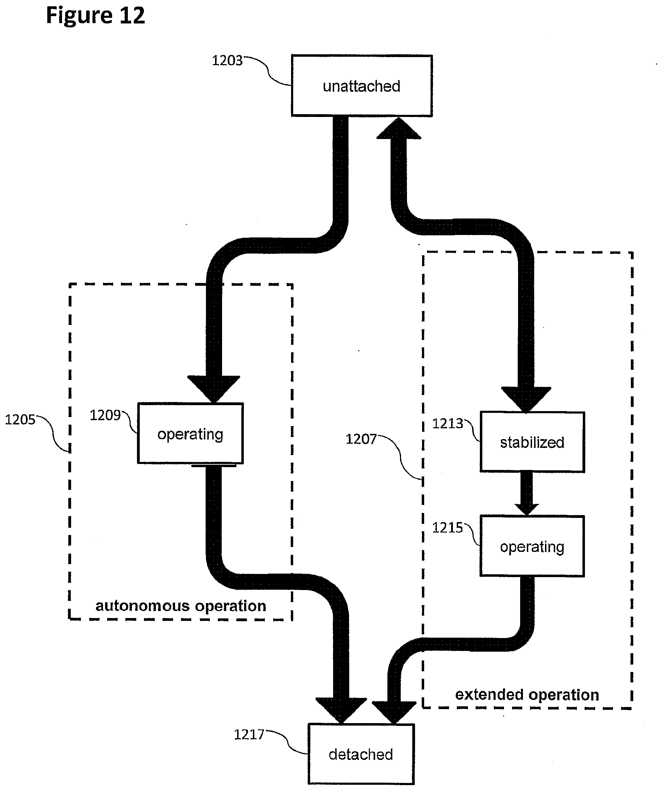

Figure 12 is a state diagram of an autoinjector and extending adaptor in accordance with an embodiment of the current invention. An autoinjector may have two or more optional operating states. For example the injector may operate in an autonomous state **1205** and/or in an operationally extended state **1207.** Operationally extended state **1207,** optionally includes operation of the injector extended to longer times and/or more viscous medicines and/or larger injection volumes. In some embodiments, operational extended state **1207** may include extension of the length of the injector. Alternatively or additionally, operational extended state **1207** may not include extension of the length of the injector. Prior to use the injector may have an unattached **1203** state and/or after use the injector may have a detached **1217** state.

In some embodiments, an injector may operate **1209** in an autonomous state **1205.** For example in the autonomous state the autoinjector may inject a medicine without an adaptor. Optionally in the autonomous state **1205** the injector may have a set of controls that are activated by a user in order. In the autonomous state **1205** the injector may inject a medicine in accordance with a prescribed protocol.

In some embodiments, the injector may operate **1215** in an extended state **1207.** In extended state **1207,** for example, an adaptor may be used to stabilize **1213** the injector on a patient. For example, the stabilized configuration may allow injection for longer periods of time and/or for a more dexterity limited patient. In the extended state **1207,** operation **1215** is optionally activated by the same set of controls and/or in accordance with the same protocol as in operation **1209** in the autonomous state **1205.** For example, for a higher viscosity medicament (for example a medicament at a lower temperature) using the same controls and protocol, the injector may require a longer time to discharge its payload. For example, extended operation **1215** may be very useful when an injector has been tested and/or approved for operation **1209** in the autonomous state **1205,** but under some conditions (for example when the medicine is cold) the injector does not meet certain requirements. For example, the injector requires more than the maximum approved time to discharge its payload. Adding the adaptor optionally makes minimal changes in the user interface and/or operation protocol of the injector, while allowing extended operation **1215.** Thus, for example with the adaptor may allow use of the injector in extended operation **1215** under extended conditions with reduced testing and/or approval while taking advantage of previous testing and/or regulatory approval of autonomous operation **1209** under more limited conditions.

### 13 Block diagram of an autoinjector and a stabilizing adaptor

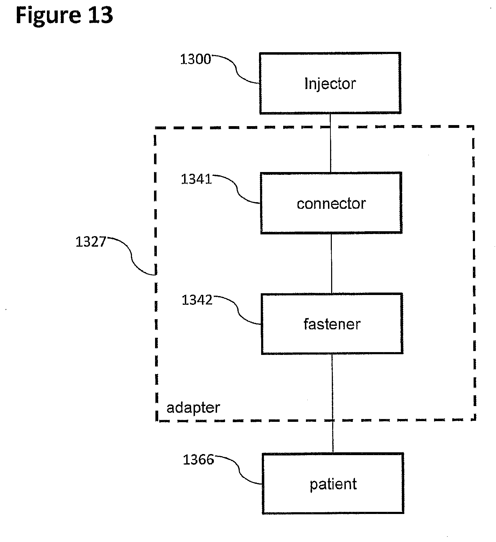

Figure 13 is a block diagram of an autoinjector **1300** and adapter **1327** in accordance with an embodiment of the present invention. Optionally an adaptor **1327** may extend operation of injector **1300.** For example, adaptor **1327** may stabilize injector **1300** on a patient allowing for longer injection times than for injector **1300** operating in an autonomous mode.

In some embodiments, adaptor **1327** may include a connector **1341** for attaching the adaptor to the injector and/or a fastener **1342** for fastening to a patient. For example, connector **1341** and fastener **1342** may be a single integrated part (for example molded out of a single piece of plastic). Alternatively or additionally, connector **1341** and fastener **1342** may be formed as two separate parts. Optionally, adapter **1327** including connector **1341** and fastener **1342** are first attached to injector **1300** and the complete assembly fastened to patient **1366** as a unit. Alternatively or additionally, adapter **1327** including connector **1341** and fastener **1342** may be fastened to the patient and injector **1300** stabilized as it is placed onto the patient. Alternatively or additionally, connector **1341** may be attached to the injector and fastener **1342** may be fastened to the patient and/or as injector **1300** is placed on the patient, connector **1341** is mated with fastener **1342** stabilizing the injector. In some embodiments there may be multiple fasteners **1342** and/or connectors **1341.**

In some embodiments, connector **1341** may attach to an outer housing of injector **1300.** For example, connector **1341** may be attached by a clamp and/or a friction fitting that grasps the walls of injector **1300.** For example the injector **1300** may have a patient contact surface on a distal end thereof and connector **1341** may be attached to the side walls of the injector **1300.** Alternatively, connector **1341** may attach to injector **1300** via an adhesive. In some embodiments, connector **1341** may be adjustable and/or adapted to attach any of a plurality to different injectors. In some embodiment, connector may be configured to attach to a specific injector. Alternatively or additionally other connection methods like magnetic force or Velcro are possible.

In some embodiments, connector **1341** may mate to fastener **1342** by a friction fitting. Alternatively or additionally connector **1341** may mate to fastener **1342** by a threaded fitting and/or a clamp and/or an interference element and/or a snap. The connection between fastener **1342** and connector **1341** may be reversible and/or may be permanent and/or irreversible (for example using a tooth and ratchet fitting). For example a ratchet and thread fittings may enable process of fixing and linkage. With a magnetic fitting linkage may occur alone. Adhesive, friction and/or Velcro® fittings may include passive actions that user should do properly. With thread, magnet, friction and/or Velcro it may be simple to disengage the device from the adaptor for example for re-use.

### 14 Adhesive and ring for stabilizing an autoinjector

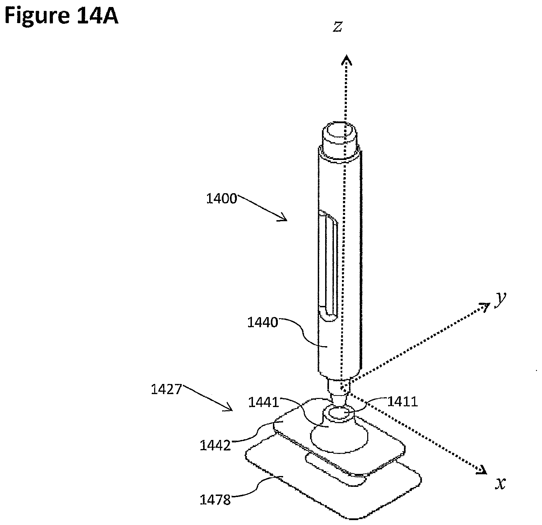

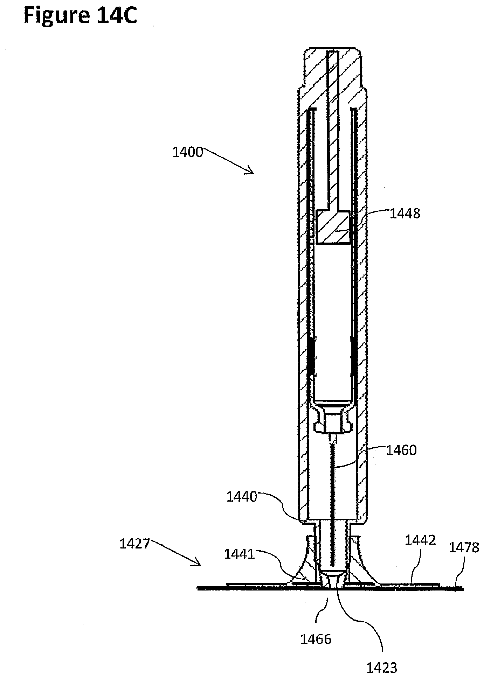

Figures 14A-F illustrate use of a drug delivery device, for example an injector **1400** and a stabilizing adapter **1427** in accordance with an embodiment of the current invention. In an exemplary embodiment, adapter **1427** includes an optional connector **1441** in the form of a ring. Optionally, the distal end of injector **1400** is inserted into a cavity **1411** of adaptor **1427.** Optionally, connector **1441** is permanently fixed to a fastener **1442** including a base and an adhesive **1478** for adhering to skin in the vicinity of an injection site **1466** of a patient (for example see Figure 14C). For example the adhesive may surround the injection site and/or may be in an area within 1 cm of the injection site and/or within 5 cm of the injection site. Connector **1441** is optionally configured to surround and/or limit movement of a distal surface of an outer housing **1440** of injector **1400.**

In some embodiments, injector **1400** may be configured for operation in an autonomous mode and/or without adapter **1427** and/or without stabilization. For example, injector **1400** may include for example a pen injector and/or for example one of the many pen injectors known in the art. Control and/or operation protocols of the stabilized injector **1400** in the extended mode (for example with adaptor **1427**) may be unchanged over operation in the autonomous mode.

Figure 14 A is an exploded perspective view of injector **1400** and adapter **1427** in accordance with some embodiments of the current invention. In some embodiments, connector **1441** may limit lateral movement (in the *x-y* plane parallel to injection site **1466** of the patient) of the distal end of injector **1400.** Additionally and/or alternatively, connector **1441** may limit rotational movement of injector **1400.** For example, connector may closely surround injector **1400** along its length, preventing rotation around the *x* and/or *y*-axis. For example, connector **1441** may attach itself to the side walls of injector **1400** over a length ranging between 0.5 cm and 2.0 cm and/or 2.0 cm to 5.0 cm and/or longer. Optionally, friction between housing **1440** and connector **1441** may prevent rotation of injector **1400** around its main axis (the z-axis). Alternatively or additionally, connector **1441** may limit movement of injector **1400** perpendicular to the injection site of the patient. For example, friction between connector **1441** and housing **1440** may prevent movement of injector **1400** in the axial direction (along the *z*-axis).

In some embodiments, adaptor **1427** may be fastened to a patient before being attached to injector **1400.** For example, a doctor may give a patient an injector **1400** including a medicine to be taken at a certain time after the patient is discharged from the hospital. A member of the hospital staff may fasten fastener **1442** and/or connector **1441** to the patient at a preferred injection location. When the time comes for the injection, the patient optionally inserts the distal end of injector **1400** into connector **1441** and activates injector **1400.** Alternatively or additionally a member of the hospital staff may fasten fastener **1442** to the patient at a preferred injection location. When the time comes for the injection, the patient optionally mates connector **1441** to fastener **1442** and/or inserts the distal end of injector **1400** into connector **1441** and/or activates injector **1400.** Alternatively or additionally, when the time comes for the injection, the patient may fasten fastener **1442** and connector **1441** to his injection site **1466** at a preferred injection location. The patient optionally then inserts the distal end of injector **1400** into connector **1441** and activates injector **1400.** Optionally, connector **1441** may be configured to make it easy for a limited dexterity patient to position injector **1400.** For example, the opening to connector **1441** may be beveled, for example the opening of connector **1441** may be beveled, for example like a funnel, to help direct the distal end of injector **1400** to the injection site.

In some embodiments, injector **1400** may be attached to connector **1441** before fastening to the patient. The patient optionally, fastens the entire assembly (injector **1400** and adapter **1427**) together to the injection location and/or activates the injector.

In some embodiments cavity **1411** may be cylindrical. The cross section of cavity **1411** optionally has a shape to fit the cross section of the distal end (the end nearest to the patient) of the injector. For example, the cross section of the distal end of the injector and/or of cavity **1411** may be circular and/or elliptical and/or rectangular and/or may have rounded corners. Alternatively or additionally cavity **1411** may be conical and/or pyramidal, for example with a large opening. The large opening may make it easier to insert the injector. The surface of contact between injector **1400** and adaptor **1427** may include, for example, the surface of the inner walls of adapter **1427** which contact, for example, the outer surface of the distal end of injector **1427.**

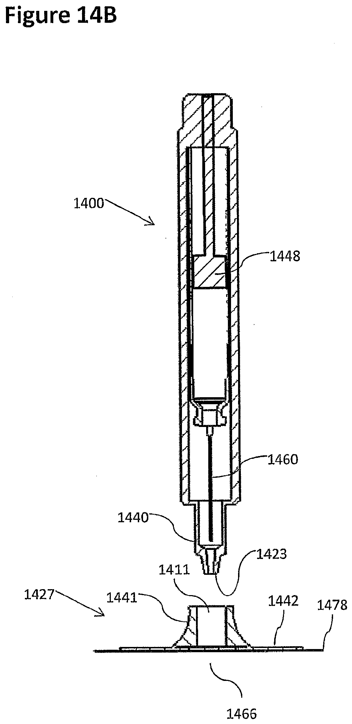

FIG. 14B is a crossectional view of injector **1400** and adapter **1427** prior to positioning on a patient and/or prior to injection in accordance with an embodiment of the current invention. A distal surface **1423** including a skin contact area is illustrated above an injection zone **1466** of the patient.

FIG. 14C is a crossectional view of injector **1400** and adapter **1427** positioned on a patient prior to injection in accordance with an embodiment of the current invention. Optionally injector **1400** includes a needle **1460** and/or a plunger **1448.** Optionally, injector **1400** includes a drive mechanism and/or a needle extension/retraction mechanism, for example, similar to embodiment **400** and/or injector **600** and/or **700** above. In some embodiments connector **1441** may attach to the side walls of injector **1400.** For example connector **1441** may be attached to a length of the side walls of the injector.

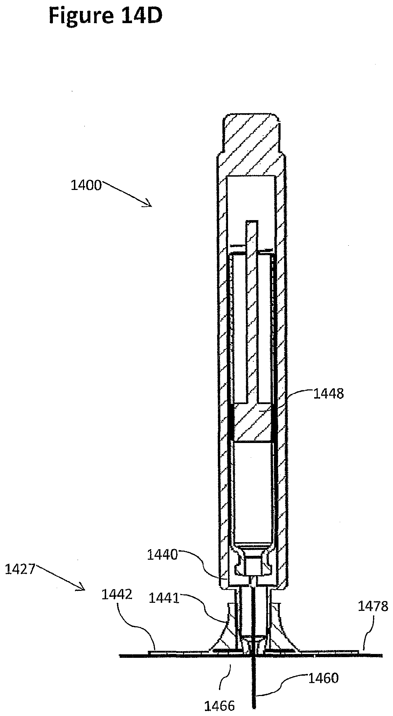

FIG. 14D is a crossectional view of injector **1400** and adapter **1427** positioned on a patient after activation and/or extension of needle **1460** into injection site **1466** and/or during injection as plunger moves downward discharging medicine into the patient in accordance with an embodiment of the current invention.

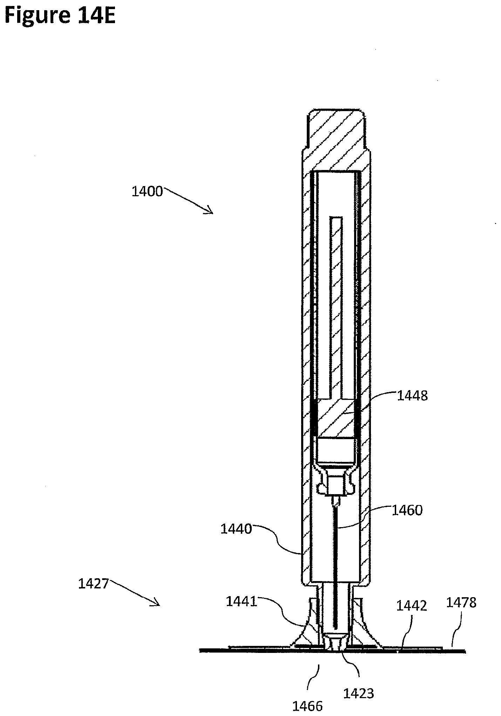

FIG. 14E is a crossectional view of injector **1400** and adapter **1427** positioned on a patient after injection in accordance with an embodiment of the current invention. Optionally, after injection, plunger **1448** has reached the end of its movement, discharging a full dose of the medicine and/or needle **1460** has retracted.

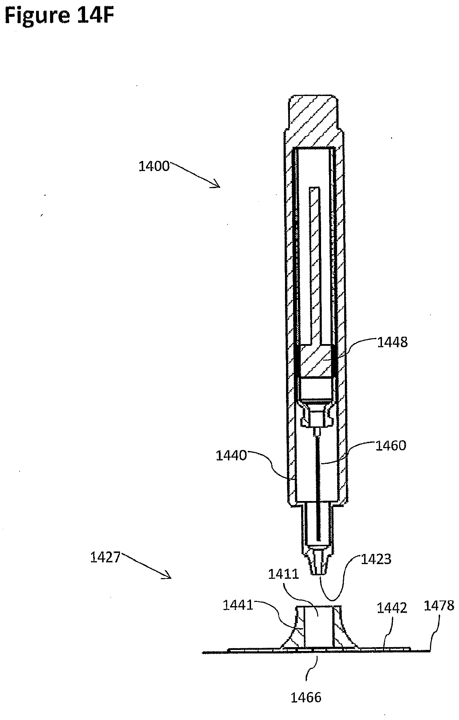

FIG. 14F illustrates removal of an injector from a stabilizing adapter and/or patient in accordance with an embodiment of the current invention. In the embodiment of Figure 14F, injector **1400** is optionally removed from injection site **1466** while adapter **1427** optionally remains on injection site **1466.** For example, adapter **1427** may be used to stabilize an injector in a subsequent injection. Alternatively or additionally adapter **1427** may be removed from injection site **1466** after removal of injector **1400.** Optionally, injector **1400** and/or adaptor **1427** are disposed of and/or reused and/or recycled separately and/or together. Alternatively or additionally, injector **1400** and/or connector **1441** and/or fastener **1442** may be removed from injection site **1466** together. For example, injector **1400** may be rotated to pivot off fastener **1442,** for example in a manner similar to that illustrated in Figure 6D. For example, using injector **1400** to pivot off fastener **1442** may be easier for patients with limited small motor skills that trying to pull and/or pivot and/or peel off fastener **1442** on its own.

### 15 Friction fitting for stabilizing an autoinjector

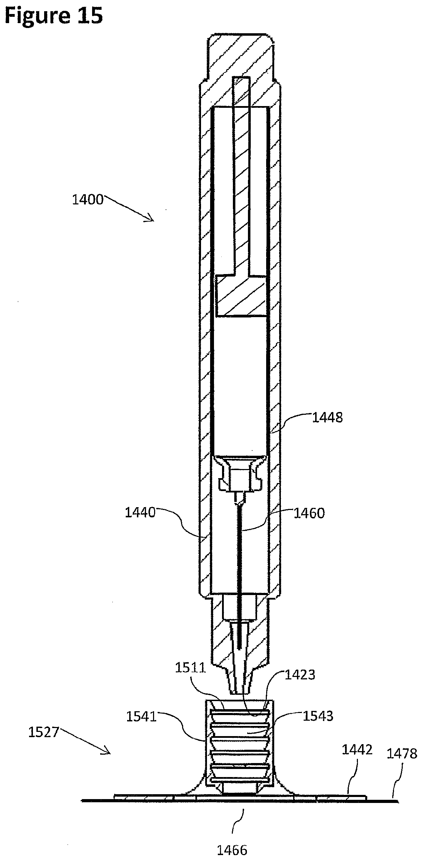

Figure 15 is a cross sectional illustration of injector **1400** and a stabilizing adapter **1527** including a friction fitting **1543** prior to positioning on a patient and/or prior to injection in accordance with an embodiment of the current invention. For example friction fitting **1543** may include a friction coating on the surface of contact between adapter **1527** and injector **1400.** For example friction fitting **1543** may be on the inner surface of a cavity **1511** in a connector **1541.** For example the fitting may include slanted teeth that increase the friction in one direction. For example it may require less force to insert injector **1400** into adapter **1527** then to remove injector **1400** from adapter **1527.** Operation of adapter **1527** may for example be as described with respect to adapter **1427.**

### 16 Adjustable angled stabilizing adaptor

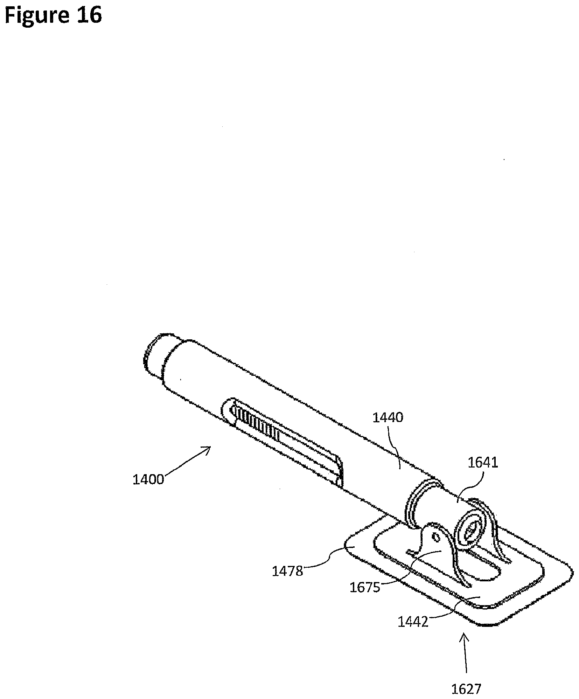

Figure 16 is a perspective view of injector **1400** and a stabilizing adapter **1627** having an adjustable angle between injection site and the injector in accordance with some embodiments of the current invention. For example the angle during operation of the injector between the skin surface at the injection site and the principle axis of the injector, the principle axis of the drug reservoir and/or the principle axis of the needle may be adjusted between 80 to 100 degrees and/or between 60 and 120 degrees and/or between 30 and 150 degrees.

In some embodiments, the connection between injector **1400** and/or adaptor **1627** and/or fastener **1442** may include a pivot **1675** and/or an axel (for example as illustrated on connector **1641**). Optionally a lock may fix the angle of injection. For example a lock may include a ratchet and teeth and/or a clamp.

In some embodiments an angle of the injection may be set prior to distribution of the injector and/or adaptor to a patient. For example, the angle may be set by a staff member at a hospital and/or clinic and/or by a pharmacist. Alternatively or additionally, the angle of the injection may be set by the patient and/or a medical aid.

### 17 Stabilizing adapter with strap fastener

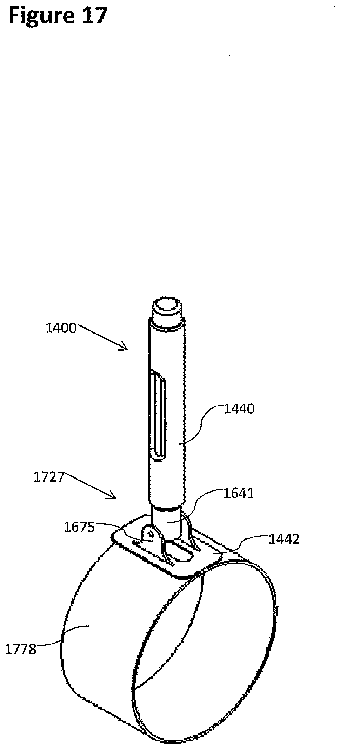

Figure 17 is a perspective view of injector **1400** and a stabilizing adapter **1727** having a strap **1778** for fastening to a patient in accordance with some embodiments of the current invention.

### 18 Stabilizing adaptor with a separate coupling

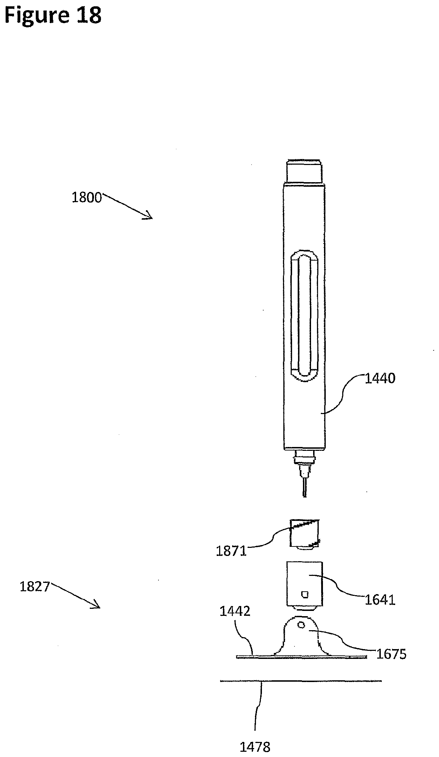

Figure 18 illustrates an exploded view of an autoinjector drug delivery device and a stabilizing adaptor **1827** having a separate coupler in accordance with an embodiment of the current invention. A separate coupler may be used to connect different models of autoinjectors to one model adaptor. For example, one model coupler **1871** may attach to injector **1800** and mate to connector **1641** and/or fastener **1442.** An alternative coupler may connect an alternative model injector to connecter **1641** and/or fastener **1442.** Optionally, coupler **1871** mates with connecter **1641** by means of a threaded connection. For example, connector **1641** may be reversibly mated to coupler **1871** and/or injector **1400.** Alternatively or additionally, connector **1641** may mate to coupler **1871** with another connection, for example an irreversible connection and/or a friction contact and/or a tooth, and/or an interference element and/or an adhesive.

### 19 Stabilizing adapter with injector clamp

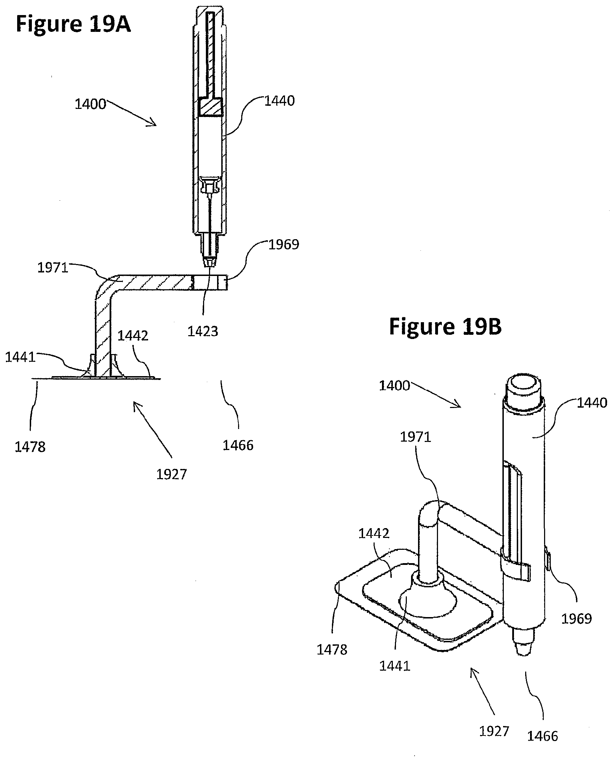

Figures 19A,B illustrate a stabilizing adaptor for an injector in accordance with an embodiment of the present invention. Optionally, adaptor **1927** includes a coupler **1971** having a clamp **1969** that clamps to the side walls of an injector. For example, clamp **1969** may be adjustable to hold onto injector **1400** and/or a different model injector. For example, claim may hold onto injector **1400** at a distance ranging between 1 to 10 cm from the distal end of the injector (and/or the skin contact area on the distal end of the injector). Optionally coupler **1971** may be fastened to the patient along with connector **1441** and/or fastener **1442.** Once adapter **1927** is fastened to a patient, injector **1400** may for example be clamped into place and activated. In some embodiments, fastener **1442** may be fastened to the patent at a distance from injection zone **1466.** For example fastener **1442** may be fastened to the patent at a distance ranging from 0.1 to 3.0 cm on one side of injection zone **1466.** Alternatively or additionally, injector **1400** may be help to coupler **1971** by another kind of fitting (for example a friction fitting and/or a threaded fitting etc.)

### 20 Stabilizing adapter with separate coupler

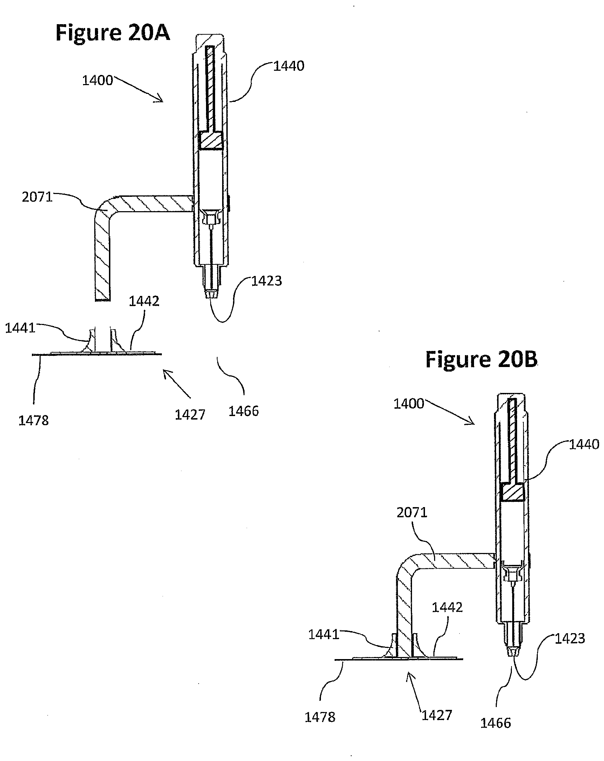

Figures 20A,B illustrate a stabilizing adapter with a mating coupler in accordance with some embodiments of the present invention. Coupler **2071** of may for example be separated from connecter **1441** and/or fastener **1442.** For example, injector **1400** may be attached to coupler **2071** while coupler **2071** is disconnected from connector **1441**. Fastener **1442** is optionally attached independently to the patient in the vicinity of injection site **1466.** For example, in the embodiment of figures 20A,B fastener **1442** may be fastened to the patient by a medical staff member while the patient is in a clinic and/or by the patient and/or a medical aid before injection. Coupler **2071** may optionally be mated to connector while injector **1400** is attached to coupler **2071.**

### 21. Adaptor with multiple fasteners and/or couplers

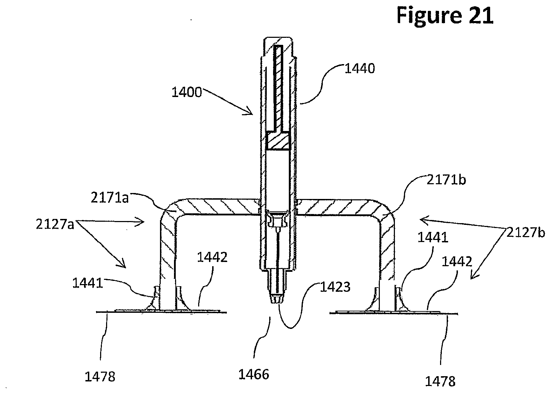

Figure 21 illustrates a multipart adapter for stabilizing an autoinjector in accordance with an embodiment of the current invention. For example, multiple adaptors **2127a,b** may be distributed in an area around injection site **1466.** Each adaptor **2127a,b** may stabilize injector **1400** from one or another side. Skin between adaptors **2127a** and **2127b,** may be stretched, for example making it easier to locate injection zone **1466** for a patient having flabby skin. In some embodiments multiple adaptors **2127a,b** may first be attached to the patient and then injector **1400** may be attached to adapters **2127a,b** (for example as in the embodiment of Figure 19 for a single coupler). Alternatively or additionally, first injector may be attached to couplers **2171a,b** and then couplers **2171a,b** may be mated to couplers **1441,** for example similar to the embodiment of figure 20 for a single coupler.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

In some embodiments, the adhesive may include a semi stiff skirt. The skirt may make the injector more stable. Alternatively or additionally, the adhesive may be connected to a stiff base (for example the base of the injector) without a semi-stiff skirt. For example, an embodiment without a semi stiff skirt may be easier to remove after the end of injection.

### 22 Method of manufacture of an compound device

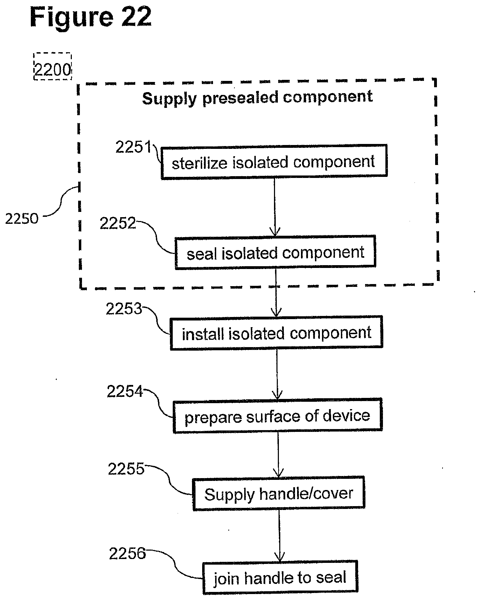

Fig. 22 is a flow chart illustration of a method of manufacture of a compound device in accordance with an embodiment of the present invention. In some embodiments, a protected component may be installed into a device in a sealed state. The device may optionally include an active surface and/or a handle and/or a surface cover and/or an activation mechanism. Optionally, the handle and/or the surface cover and/or the activation mechanism may be joined to a seal of the protected component, for example by a coupler. Optionally, the coupler may synchronize unsealing of the protected component and activation of the active surface. For example the method of manufacture illustrated in embodiment **2200** of Fig. 22 may optionally be used in manufacturing one, some and/or any of the embodiments of an injector illustrated in the embodiments illustrated herein above and/or below. In some embodiments, a sterile adhesive may be sealed and/or covered by a protective packaging for example a cover. The adhesive may for example be attached to a surface in a sterile and/or protected state. Optionally the coupler may be connected to the protective packaging and/or cover of the adhesive and/or may synchronize opening of the packaging with another act.

In some embodiments an isolated component may be supplied **2250** in a presealed state. For example, the presealed component may include a fluid path of a preloaded syringe. Optionally the syringe and/or fluid path may be sterilized **2251** and/or preloaded with for example medicine and/or sealed **2252** in an aseptic room.

In some embodiments, a protected component may be installed **2253** into a device in a sealed state. For example, in some embodiments, a preloaded syringe with a sealed sterile fluid path may optionally be installed **2253** into an autoinjector.

In some embodiments, the device may optionally include an active surface with an activation mechanism. The active surface may be prepared **2254.** For example an autoinjector may include an active surface including an adhesive for attaching to a patient. Preparing **2254** the surface may include for example applying the adhesive to the surface.

In some embodiments, the active surface may be supplied **2255** with an activation mechanism and/or protective cover. For example, in some embodiments, an adhesive surface may be covered by an adhesive cover and/or enabled by removing the adhesive cover.

In some embodiments, the surface cover and/or activation mechanism and/or handle may be joined **2256** to the seal of the protected component, for example by a coupler. Optionally, the coupler may synchronize unsealing of the protected component and activation of the active surface. For example removing the surface cover and/or activating the surface may trigger unsealing of the protected component. Alternatively or additionally, unsealing of the protected component may trigger removal of the surface cover and/or activating of the active surface.

### 23 Safety cover

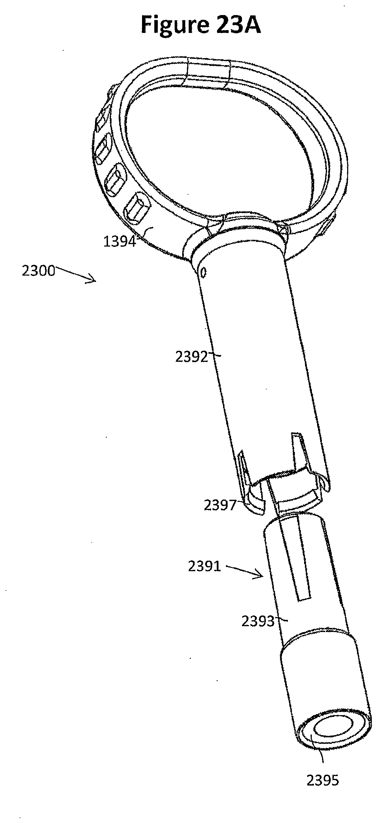

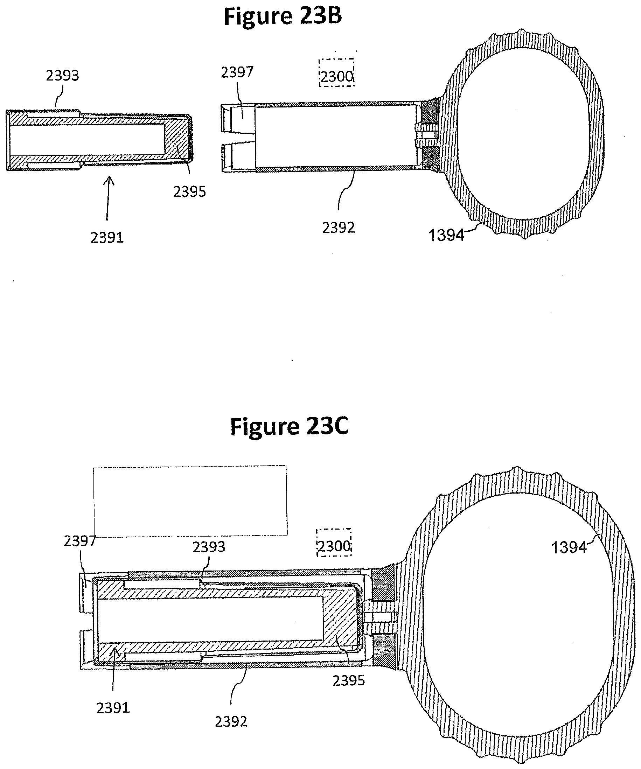

Figs. 23A-D illustrate a system **2300** designed to remove a needle cover **2391** in accordance with an embodiment of the current invention. In some embodiments a needle may be installed into an autoinjector while covered with a needle cover. A safety cover may be installed to the injector and/or attached to the needle cover. Removal of the safety cover may also bring about removal of the needle cover.

Fig. 23A is a perspective view of system **2300** and associated needle cover **2391.** Optionally, needle cover **2391** includes a rigid outer shell **2393** and a sterile rubber core **2395.** When needle cover **2391** is installed over a sterilized needle, rubber core **2395** may protect the sterility of the needle and/or shell **2393** may protect the needle from causing a stick hazard. Alternatively or additionally, a needle may be covered with a single cover (for example either rubber or rigid) that may preserve sterility and/or prevent a stick hazard.

For example, a prefilled syringe may be installed into an autoinjector (for example injector **1400**) with a needle already covered with needle cover **2391.** Before shipping the injector, system **2300** may be installed onto the injector. A needle cover remover, for example a needle protector **2392** is optionally pushed over shell **2393** of the needle cover. Needle protector **2392** may optionally have the form of a sleeve and/or a clamp that is inserted through an aperture in base **1442.** Optionally, needle protector **2392** is inserted into the injector housing and/or is attached to needle cover **2391** after assembly of the injector. Protector **2392** may include clasps (for example clasps **2397**) which engage shell **2393.** When protector **2392** is removed from the injector, clasps **2397** may pull off needle cover **2391.**

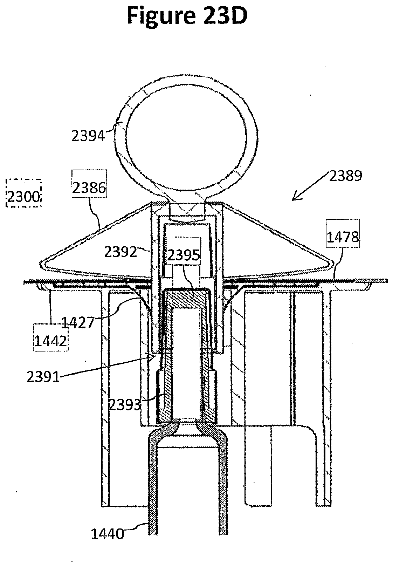

Figs 23D illustrate installation of needle protector **2392** and adhesive cover for example adhesive protector **2389** in accordance with exemplary system **2300** of the present invention. The safety cover may include for example a handle **2394** and/or a needle protector **2392** and/or an adhesive protector **2389** for covering an adhesive **1478.** For example, handle **2394** may be attached to extenders **2386** for attaching to adhesive protector **2389** and/or for peeling adhesive protector **2389** from adhesive **1478** when handle **2394** and/or a needle protector **2392** and/or the needle cover (for example shell **2393** and or rubber core **2395**) are pulled off.

In some embodiments, a stabilizer (for example adaptor **1427**) may be attached to an autoinjector pen (for example Rita pen injector planned for production by Unilife corperation150 South Warner Road, King of Prussia, PA 19406 USA and/or Metoject/Metex pen injector available from Medac GmbH Scion House Stirling University Innovation Park Stirling FK9 4NF England). Optionally, protector **2392** and/or handle **2394** are installed through the open front end of the injector (for example in place of the front cap of the injector). The injector can then be used in a supported and/or stabilized mode without no and/or minimal changes in the manufacture and/or operation of the device. For example, the user pulls off handle **2394** and/or protector **2392.** Optionally, protector **2392** pulls off sterile needle cover **2391** that may for example have been mounted on the syringe during preloading of medicine in a clean room. The injector and/or stabilizer is optionally then placed on the skin and/or used exactly as the unstabilized original injector.

### 24 Modular drug delivery device

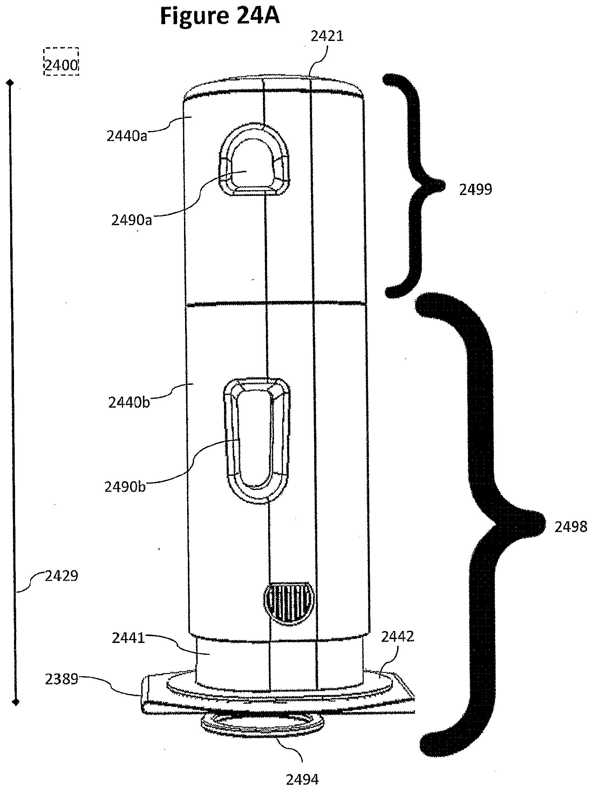

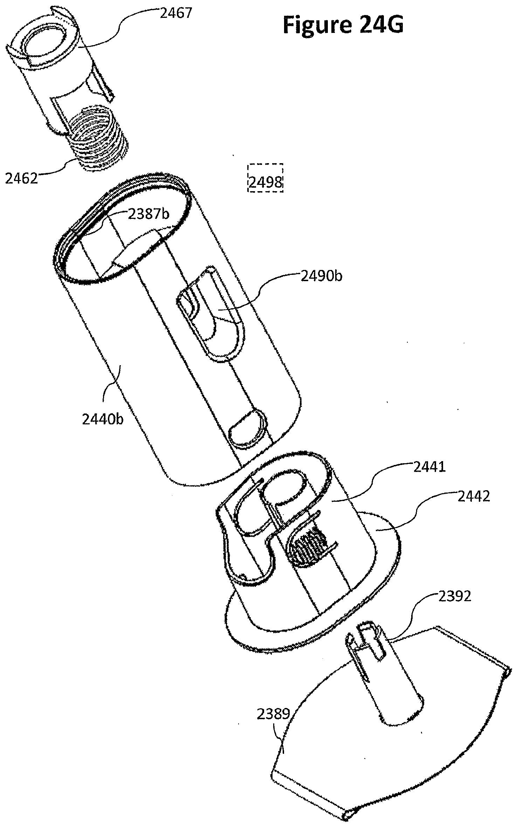

Figs. 24A-G illustrate a modular preloaded stabilized drug delivery device, for example an autoinjector **2400** in accordance with an embodiment of the current invention. Optionally, a drug distributer may load injector **2400** with a drug using standard components and procedures. Optionally, injector **2400** may be loaded with a standard form syringe and/or assembled by simple assembly not requiring special tools, skilled workers and/or long assembly time. For example, the drug delivery device may be delivered to a medicine distributer with a small number of separate assemblies. For example, the drug delivery device may be delivered to the medicine distributor with two separate preassembled assemblies, a distal assembly **2498** and a proximal assembly **2499.** The medicine distributor may optionally supply the medicine in a standard sealed preloaded syringe with a needle cover protecting the sterility of a needle at the distal end and/or a stopper sealing the syringe barrel at the proximal end. Finally assembly of the drug delivery device optionally includes inserting the syringe into a channel in distal assembly **2498** and then assembling the drug delivery device for example by placing proximal assembly **2499** over the proximal end of distal assembly **2498** and/or snapping the assemblies together.

Fig. 24A illustrates an exterior view of exemplary injector **2400** in accordance with an embodiment of the current invention. Optionally proximal assembly **2499** is packed into a proximal housing **2440a** and/or distal assembly **2498** is packed into a distal housing **2440b.** Optionally, a needle shield **2441** is slidably attached to the distal end of distal housing **2440b.** Needle shield **2441** optionally includes a skin contact surface, for example an adhesive base **2442.** The adhesive of base **2442** may be protected for example by a adhesive protector **2389.** An optional pull tab handle **2494.** Handle **2494** serves for example for enabling injector **2400** and/or for peeling adhesive protector **2389** and/or for removing a needle cover. A proximal cover **2421** optionally closes the proximal end of proximal housing **2440a.** Windows **2490a** and/or **2490b** are optionally supplied for monitoring needle retraction and/or medicine discharge respectively. The height **2429** of injector **2400** may range between half the length of the long axis of base **2442** to the length of long axis **2442** and/or between the length of the long axis of base **2442** to twice the length of long axis **2442** and/or greater than twice the length of the long axis of base **2442.** The large height of the injector may give leverage for pivoting base **2442** off a skin of a patient. An adhesive strength of said base may be adjusted to pivot off a skin of the patient without ripping the skin, for example when the injector is pivoted around an edge of base **2442.** Optionally, the adhesive strength may be non-uniform. For example adhesive strength may be less far from a pivoting axis and more near the pivoting axis.

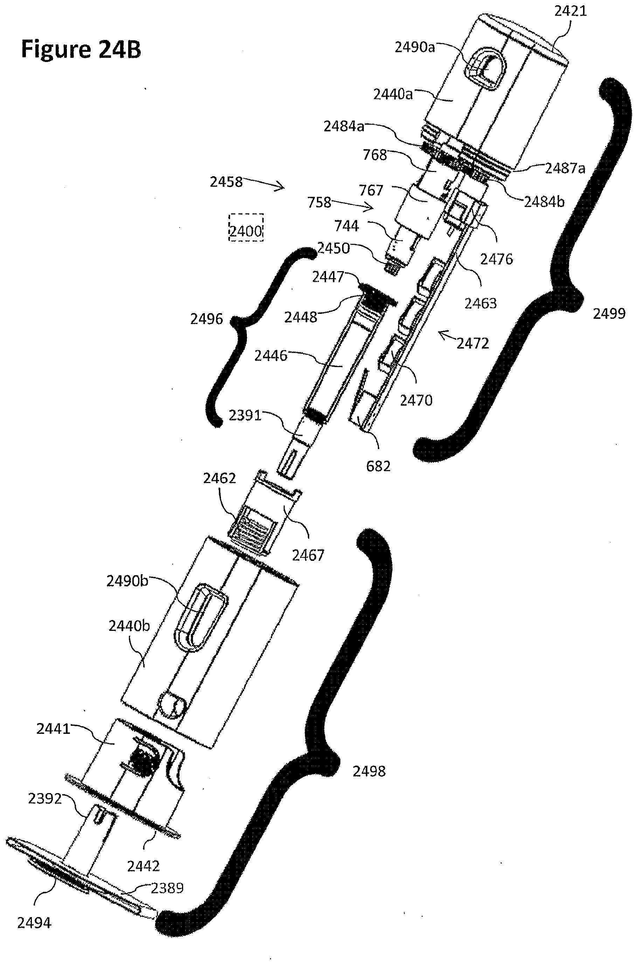

Fig 24B is an exploded view of the system of injector **2400** in accordance with and embodiment of the current invention. Injector **2400** may for example include three modular assemblies: lower distal assembly **2498,** upper proximal assembly **2499** and/or a preloaded syringe **2496.**

In some embodiments, proximal assembly **2499** may include a drive system. For example, proximal assembly **2499** includes a power module **2472,** a transmission **2458** including a rotary screw driver for a plunger and/or a retraction mechanism (for example as illustrated in Figs. 7A-B and 7I-K).

In some embodiments, distal assembly **2498** may include a patient interface and/or a syringe fitting.

In some embodiments, transmission **2458** of proximal assembly **2499** optionally includes a needle retraction mechanism **758** and/or a telescoping assembly including a plunger adaptor **2450** for driving a plunger **2448** for discharging a drug and or a coupling element **2484a** (for example a gear) for connecting to a motor (for example motor **2476**).

In some embodiments, power module **2472** may include a modular component. For example the entire power module **2472** may be installed as a whole into injector **2400.** For example power module **2472** may be tested as a unit (for example testing a motor **2476** and/or a battery **2470**). Optionally the power module **2472** may be supported by a base for example a PCB board **2463.** For example the power module **2472** may include a syringe position sensor, for example a switch **682** and/or a power source for example one or more batteries **2470** and/or a motor **2476.** For example, power module **2472** may include a coupling element **2484b.** For example coupling element **2484b** may transfer power from power module **2472** to coupling element **2484a** and/or transmission **2458.** For example, coupling element **2484b** may include a gear.

In some embodiments, the patient interface of distal assembly **2498** includes an adhesive base **2442** for stabilizing the drug delivery device on the skin of the patient and/or handle **2494** and/or needle shield **2441.** For example, handle **2494** may be used for enabling the drug delivery device and/or for removing a needle protector **2392** and/or for removing a needle cover **2391** and/or for peeling off an adhesive protector **2389.** For example, once injector **2400** is enabled, needle shield **2441** may collapse when pressed against the skin of the patient thereby activating injector **2400** and/or exposing a needle.

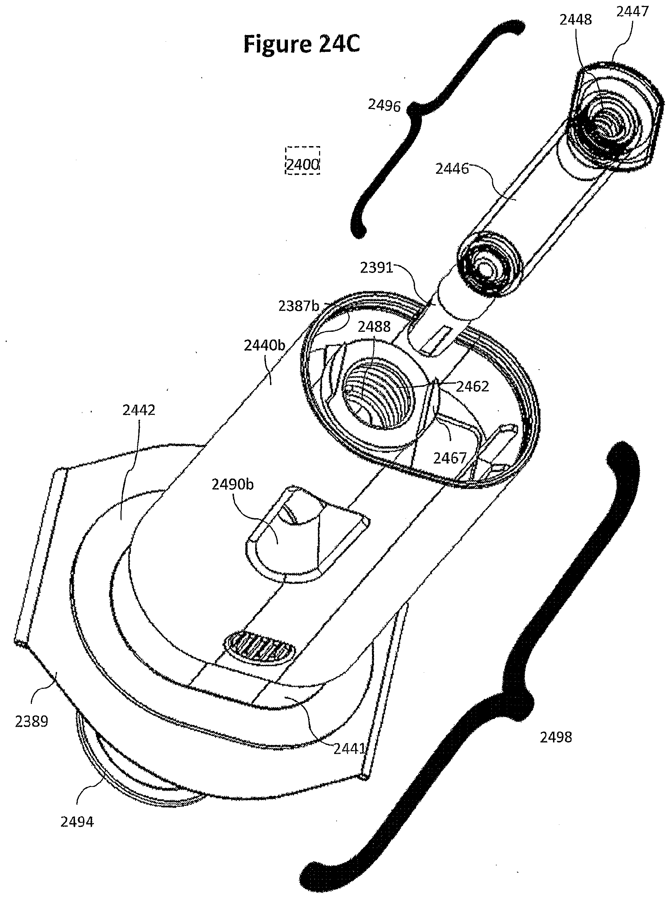

In some embodiments, the syringe fitting of distal assembly **2498** may include a channel **2488** fitting the syringe (for example as illustrated in Fig. 24C) and/or a syringe retainer **2467** and/or a biasing element (for example a return spring **2462**) for retracting the needle.

In some embodiments, preloaded syringe **2496** may include a barrel **2446** filled with a aseptic and/or sterile drug. Barrel **2446** is optionally in fluid communication and/or rigidly attached to a hollow needle on the distal end of barrel **2446.** Some and/or all of the needle may be covered and/or protected and/or kept sterile by needle cover **2391.** An open proximal end of barrel **2446** is optionally sealed by a plunger **2448.**

Figs. 24C-E illustrate final assembly of a modular stabilized drug delivery device in accordance with an embodiment of the current invention. Optionally, proximal assembly **2499** and distal assembly **2498** are each supplied to a drug distributor, in an assembled state. For example, as illustrated in Fig. 24C, a drug distributor (for example a drug company and/or a hospital and/or a pharmacist inserts a preloaded syringe **2496** into channel **2488** in distal assembly **2498.** When syringe **2496** is inserted into channel **2488** optionally needle cover **2391** is snapped into needle protector **2392** and/or a flange **2447** on syringe **2496** fits into and/or is snapped into retainer **2467.**

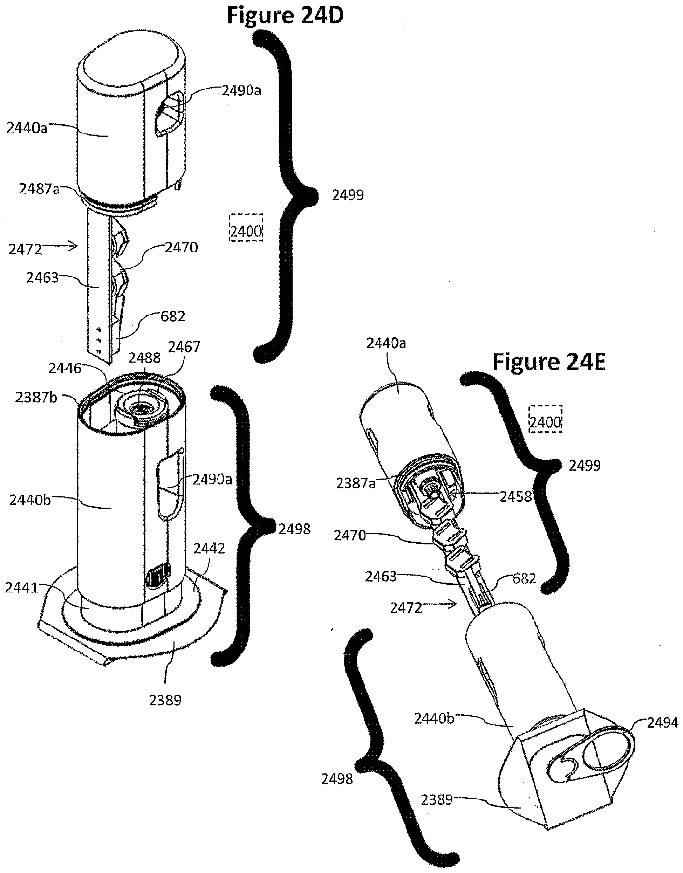

In some embodiments, after inserting syringe **2496** into distal assembly **2498,** the distributor then joins distal assembly **2498** to proximal assembly **2499** for example as illustrated in Figs. 24D and/or 24E. For example the distributor joins distal assembly **2498** to proximal assembly **2499** in an axial motion. For example switch **682** slides into position inside of distal assembly **2498** and/or adaptor **2450** is joined to plunger **2448** and/or a snap fitting **2487a** on proximal housing **2440a** connects to a snap fitting **2487b** on distal housing **2440b** for example as illustrated in Fig. 24A.

In some embodiments, the assembled injector **2400** may be distributed to a patient. The patient may optionally pull handle **2494** distally away from injector **2400** thereby peeling adhesive protector **2389** from base **2442** and/or pulling needle protector **2492** and/or needle cover **2391** out a needle aperture in base **2442** and/or enabling injector **2400.** Optionally the patient places adhesive base **2442** of the enabled injector **2400** against his skin and pushes distally on proximal cover **2421** collapsing needle shield **2441** and or activating injector **2400.** For example, a needle may inserted through the needle aperture in base **2442** into the skin of the patient and/or switch **682** may be triggered activating motor **2476** and/or rotating couplings **2484a,b** and/or extending a telescoping transmission **2458** and/or pushing plunger **2448** into barrel **2446** and/or discharging a medicine out the needle into the patient. Optionally, when plunger **2448** is blocked (for example when it reaches the end of barrel **2446** and/or due to a malfunction) the torque and/or linear force unlocks retraction mechanism **758.** When retraction mechanism **758** is unlocked return spring optionally pushes syringe **2496** into the retracted position and/or releases switch **682** stopping motor **2476** and/or drug delivery. The patient optionally sees through window **2490b** whether the entire dose has been discharged and/or through window **2490a** whether the needle has been retracted (for example inner sleeve may be colored red and be visible in window **2490a** before retraction and/or outer sleeve **767** may be colored green and/or may become visible in window **2490a** after retraction). Once the needle has retracted, the patient optionally pivots injector **2400** off his skin (for example by twisting and/or inclining injector **2400**).

In some embodiments, the form of the injector is designed to aid removal. For example the adhesive zone may have a short axis and/or a long axis. The injector is optionally tipped around a pivoting axis parallel to the long axis, such that the short axis is the lever for pulling the adhesive off the skin. Alternatively or additionally, the adhesive may have a reduced flexible skirt at the location from which it is to be removed from the skin.

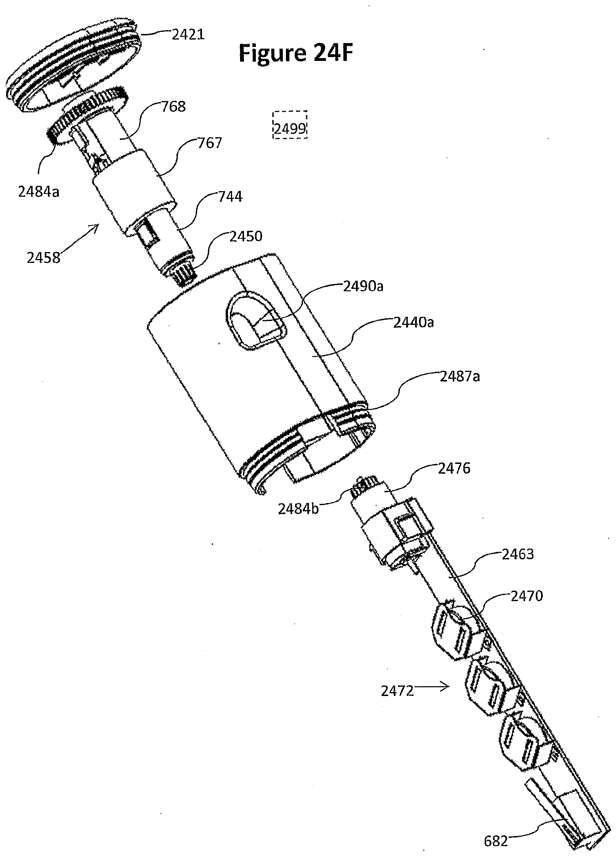

Fig. 24F illustrates assembly of proximal assembly **2499** in accordance with an embodiment of the current invention. For example, the assembled transmission **2558** module may be inserted into the proximal end of housing **2440a** and/or the assembled power module **2472** may be inserted into the distal end of housing **2440a** until coupling **2484a** in engaged to coupling **2484b.** Optionally proximal cover **2421** is placed over the proximal end of housing **2440a.**

Fig. 24F illustrates assembly of distal assembly **2498** in accordance with an embodiment of the current invention. For example, needle shield **2441** is partially inserted into the distal end of housing **2440b.** Optionally needle protector **2392** is inserted into the distal end of channel **2488** and/or retainer **2467** and/or spring **2462** are inserted into the proximal end of channel **2488.** Optionally adhesive protector **2389** is placed over the distal side of adhesive base **2442.** Optionally, adhesive protector **2389** is connected to handle **2494** by extenders (for example portions **785b,c** as illustrated in Fig. 7C-H)

### 25 Modular injector

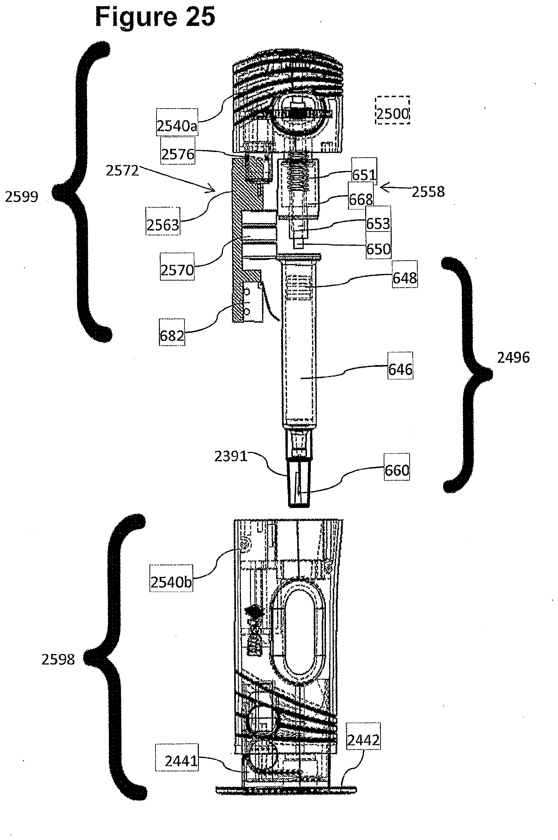

Figs. 25 illustrates an alternative modular preloaded stabilized drug delivery device **2500** in accordance with an embodiment of the current invention. Device **2500** is optionally designed for simple final assembly by a drug distributor. For example a prefilled syringe **2496** is inserted into a channel in a distal assembly **2598** and/or a proximal assembly **2599** is snapped onto distal assembly **2598** via an axial motion. Device **2500** includes a transmission **2558** with a rotary needle retraction mechanism (for example as illustrated in Figs. 6A-F). Power module **2572** includes a motor **2576** and/or batteries **2570** and/or a switch **682** rigidly mounted on a base **2563.** Base **2563** may be made for example of molded plastic. Optionally distal assembly is assembled into a distal housing **2540b.** Optionally proximal assembly is assembled into a proximal housing **2540a.** Distal assembly **2598** may optionally not include a biasing element for needle return.

### 26 Method of manufacture of a stabilized injector

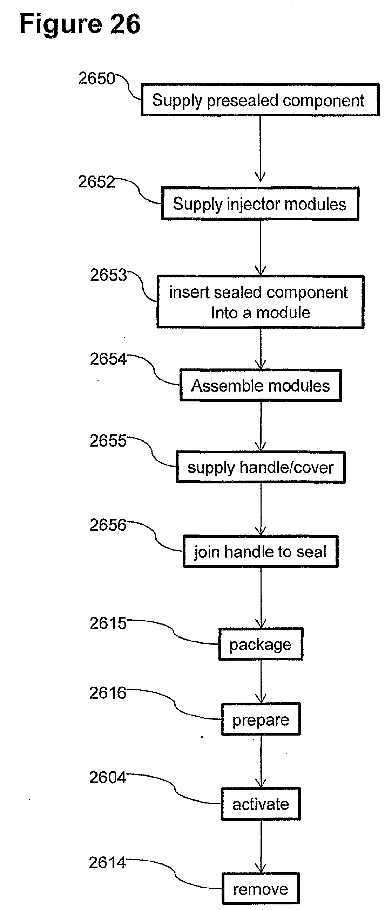

Fig. 26 illustrates a method of manufacture of a modular preloaded stabilized drug delivery device in accordance with an embodiment of the current invention.

In some embodiments, a presealed and/or preloaded component is optionally supplied **2650.** For example, a preloaded component may include a preloaded syringe with a sealed and/or covered sterile needle.

In some embodiments, preassembled modules of the injector are optionally supplied **2652.** For example the preassembled modules may be designed for easy assembly.

In some embodiments, the presealed component may be inserted **2653** into one or more of the modules. During insertion **2653,** a part of the presealed component may be connected to an enabling mechanism of the injector. For example, inserting a preloaded syringe into a channel of an injector may cause a needle protector to snap onto a sterile needle cover such that when the needle protector and/or an adhesive protector is removed from the device, the needle cover is also removed.

In some embodiments, the preassembled modules and/or the presealed component may be assembled **2654** together. For example the modular components may include a quick connection (for example a snap and/or a friction fit and/or a interference element) for simple assembly.

In some embodiments, the drug delivery device may include a stabilizer. A cover and/or activation mechanism and/or handle is optionally supplied **2655** for the stabilizer. For example, the stabilizer may include an adhesive surface which is activated by peeling off a surface cover. For example, a handle may be supplied **2655** for peeling the cover. Alternatively or additionally an adaptor may include the stabilizer and/or the adhesive cover and/or a safety cap. Optionally, various components of the activator and/or cover may be connected for synchronized removal and/or activation.

In some embodiments, the activation mechanism and/or surface cover of the adapter may be joined **2656** to the presealed component. For example, a coupler may connect between the handle and the seal of the presealed component and/or a safety cap. For example the coupler may be fit through an aperture (for example a needle aperture) in the adhesive surface to connect between the handle and the needle cover. For example an extender may connect the handle to the adhesive cover, for example for peeling the adhesive cover.

In some embodiments the entire assembled system including the presealed component and the preassembled modules may be packaged **2615** for shipping and/or sent to a dealer (for example a hospital and/or a pharmacist) and/or a patient. Optionally the injector may be shipped in a packed state. For example, components may be packaged in a folded and/or stacked manner, for example saving space and/or packaging materials.

In some embodiments, the injector may be prepared **2616** by the dealer and/or the patient. For example, the injector may be removed from packaging and/or unfolded and/or assembled. Preparing an injector may include enabling the injector. For example, enabling may include removing an adhesive protector and/or a needle protector and/or a sterility protector (for example a needle cover).

In some embodiments, the injector may be activated **2604.** Activation **2604** may include fastening the injector to the patient and/or inserting a needle and/or activating a switch, for example a button.

After use the injector may be removed **2614** from the patient. For example the injector may include a needle protection system that guards the needle to avoid a sharp point hazard. In some embodiments, the injector or part thereof may be disposed of in domestic waste.

### 27 Method of stabilizing a pen injector

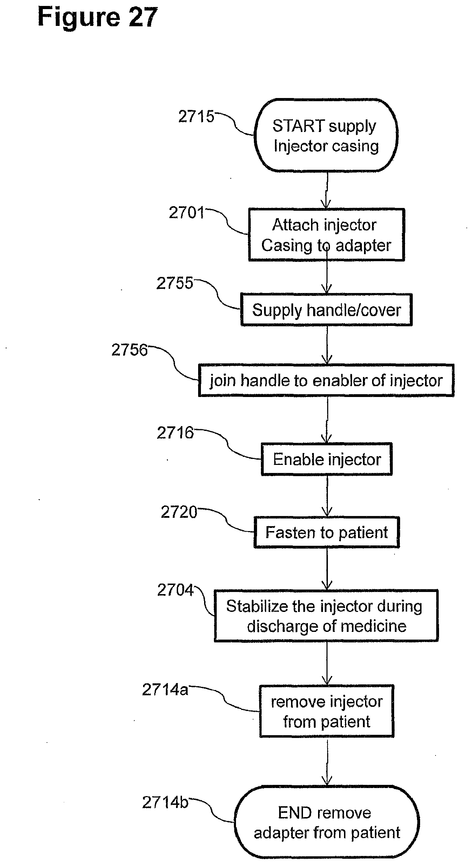

Fig. 27 illustrates a method of stabilizing a pen injector in accordance with an embodiment of the current invention. For example the method may be used to retrofit an existing pen injector.

In some embodiments a pen injector may be supplied **2715.** For example the pen injector may be capable of use in an autonomous mode (without stabilization).

In some embodiments a stabilizer (for example a stabilizing adapter) may be attached **2701** to the housing of the injector. For example the stabilizer may include a relatively large skin contact surface and/or an adhesive surface to hold the injector onto an injection site during injection. Optionally, the injector may be supplied to a patient with the stabilizing adaptor already attached. For example the stabilizer may be attached to the injector by a drug supplier and/or a drug distributer and/or a retailer and/or a dealer (for example a pharmacist and/or a health worker). Alternatively or additionally the patient may attach the adaptor to the injector before enabling the injector and/or before uncovering the adhesive and/or before fastening the adaptor to his skin and/or before activating the injector).

In some embodiments, the stabilizer may be adapted to allow the injector to function according to its autonomous mode for example:
1. In some embodiments an injector may have a sleeve that extends out of the distal end of the injector at the end of injection. A stabilizing adapter may optionally include a large needle aperture. For example the needle aperture may be large enough to allow the sleeve to protrude through the needle aperture after injection. Alternatively or additionally, the adapter may be designed so that the injector is axially releasably connected to the adapter. For example after injection, the injector is removed by an axial movement from the adapter while the adapter remains on the skin of the patient. Removing the injector from the adapter may allow the sleeve to extend while removing the injector. After removing the injector, then the adapter is optionally removed from the patient. For example, the injector may be connected to the adaptor with a friction fitting and/or a reversible snap and/or interference snap fit and/or with an adhesive and/or with a breakable joint that allows the injector to be removed axially from the adapter. Alternatively or additionally, the adapter may be designed so that at the end of injection, an attachment between the injector and the adaptor is released automatically and/or by a manual action.
2. In some embodiments an injector may have a skin contact sensor. An adapter may be designed with a hole that allows the skin contact sensor to protrude from the adapter. Alternatively or additionally, the injector may be supplied to a patient with the injector partially inserted. The patient may adhere the adapter to his skin and then push the injector distally against the adapter. Pressure of the distal end of the injector due to the pressure by the patient may serve to activate the injector as if it were held against the patient's skin.
3. In some embodiments an injector may have a retractable needle. Optionally the adapter may have a large and/or a small needle aperture allowing insertion and/or retraction of the needle.

In some embodiments a cover and/or activation mechanism and/or handle may be supplied **2755** for the stabilizing adaptor. For example, the stabilizing adapter may include an adhesive surface which is activated by peeling off a surface cover. For example, a handle may be supplied **2755** for peeling the cover. Alternatively or additionally adaptor may include a safety cap. Optionally, various components of the activator and/or cover may be connected for synchronized removal and/or activation.

In some embodiments, the activation mechanism and/or surface cover of the adapter may be joined **2756** to an enabling mechanism of the injector. For example, a coupler may connect between the handle and the seal of a protected component and/or a safety cap. For example the coupler may be fit through an aperture (for example a needle aperture) in the adapter to connect between the handle and an enabling mechanism of the injector.

In some embodiments, before use the stabilized injector system may be enabled **2716.** For example, enabling the injector may include manipulating the handle of the adapter to activate the adapter and/or enable the injector.

In some embodiments the combined injector adaptor system may be fastened **2720** to a patient before injection. The injector may inject a drug while stabilized **2704** by and/or attached to the stabilizing adaptor.

In some embodiments, the injector may be removed **2714a** from the patient after the drug has been discharged and/or in the case of a malfunction. Removal **2714a** may be adapted to the injector design and/or status. For example, for an injector which retracts the needle at the end of injection, the injector and stabilizer may be peeled and/or pivoted off the patient together, for example by twisting and/or inclining the injector adaptor system. Alternatively or additionally, the injector may for example be removed **2714a** from the adapter and the patient simultaneously by an axial movement. For example in removing **2714a** the injector by axial movement may be useful where the needle does not retract (for example for injector that does not have a retraction mechanism and/or in the case of a malfunction when the needle failed to retract). Optionally, the adaptor and injector may sometimes be removed **2714a,b** by inclining the injector and/or pivoting the adaptor. Alternatively or additionally the adaptor may have a safety release that allows the injector to be removed **2714a** from the adaptor when desired and/or under specified circumstances. Alternatively or additionally the injector may be attached to the adaptor in a way that allows axial removal **2714a** of the injector from the adaptor using a force that is less than the expected axial force necessary to remove the adhesive from the skin. For example, the injector may be removed from the adapter by a linear force ranging between 0.1 to 0.2 N and/or between 0.2 to 0.4 N and/or between 0.4 to 0.8 N and/or between 0.8 to 1.6 N and/or between 1.6 to 3.2 N. Alternatively or additionally, the geometry of the adapter may allow pealing the adapter off the patient while pulling the needle more or less axially from the skin (for example, the needle aperture of the adaptor may be located closer to an edge that is lifted from the skin and further from an edge that is used as a fulcrum for pivoting the injector).

In some embodiments, after injection, the adapter and/or the adhesive may be removed **2714b** from the skin of the patient. For example removal **2714b** of the adaptor may be after removal **2714a** of the injector and/or simultaneous to removal **2714a** of the injector and/or prior to removal **2714a** of the injector.

### 28 Removing a stabilized pen injector

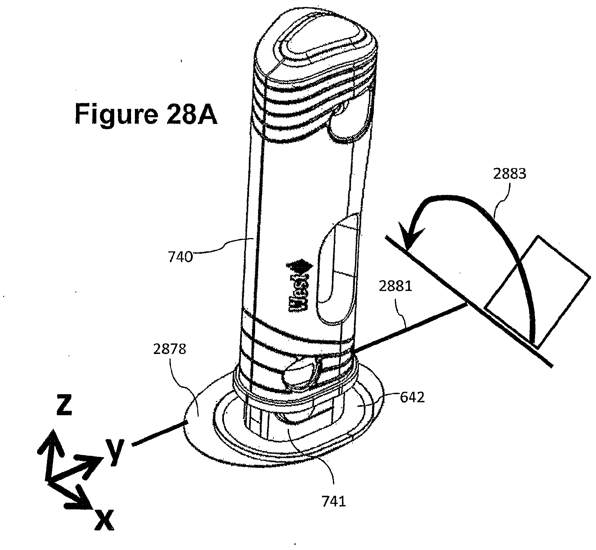

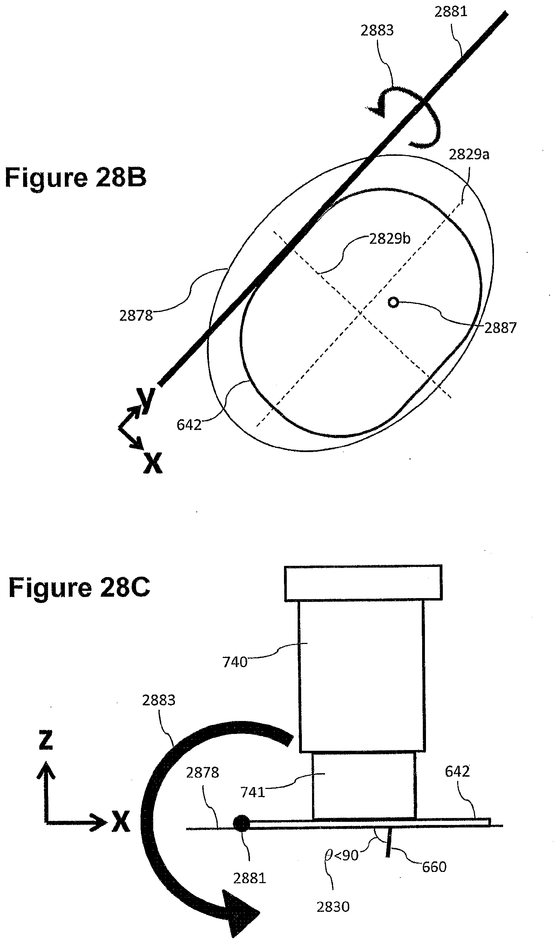

Figs. 28A-C illustrate removing a stabilized pen injector in accordance with an embodiment of the current invention. Optionally, the injector is removed by pivoting off the skin. Optionally, an adhesive base **642** of the injector has a non-symmetric shape for example including a long axis **2829a** and/or a short axis **2829b.** Pivoting may be for example around an axis **2881** of pivoting that is approximately perpendicular to the short axis **2829b** of base **642** and/or approximately parallel to the long axis **2829a** of the base **642.** An adhesive skirt **2878** may extend past the edge of the base on at least one side. The adhesive skirt may extend further along an edge close to axis **2881** of pivoting than along an edge opposite axis **2881** of rotation. A needle aperture **2887** may be distanced from the axis **2881** of pivoting.

In some embodiments, axis **2881of** pivoting may be along an edge of base **642.** For example, base **642** may include a linear edge at the location intended for axis **2881** of pivoting. An exemplary direction of pivoting is illustrated by arrow **2883.** Optionally, adhesive skirt **2878** may extend further from the edge of base near the axis **2881** of pivoting than from the edges far from and/or opposite axis **2881** of pivoting. For example, skirt **2878** may extend between 1 to 2 mm and/or between 2 to 3 mm and/or between 3 to 4 mm and/or between 4 to 10 mm from an edge of base **642** near axis **2881** of pivoting. For example, skirt **2878** may extend between 0 to 1 mm and/or between 2 to 3 mm and/or between 3 to 4 mm from an edge of base **642** far from and/or opposite axis **2881** of pivoting.

In some embodiments, a needle location and/or a needle aperture (for example an location and/or an aperture **2887** from which a needle **660** extends from the injector into the skin of the patient [for example see Fig. 28C]) may be located to reduce a torque on needle **660** when pivoting the injector from the skin of the patient. For example the needle aperture **2887** and/or needle location may be located opposite and/or distanced from axis **2878** of pivoting. For example, the needle location and/or aperture **2887** may be located closer to the side of base **642** opposite the axis **2881** of pivoting than to the side of base **642** near axis **2881** of pivoting. For example the position of needle location and/or aperture **2887** may range between 51 to 60% of the distance along a line from axis **2881** of pivoting to an opposite edge of adhesive base **642** and/or between 60 to 70% of the distance along a line from axis **2881** of pivoting to an opposite edge of adhesive base **642** and/or between 70 to 90% of the distance along a line from axis **2881** of pivoting to an opposite edge of adhesive base **642** and/or between 90 to 100% of the distance along a line from axis **2881** of pivoting to an opposite edge of adhesive base **642.** For example, the needle location and/or aperture **2887** may be located at a distance ranging between 5 mm to 15 mm from axis **2881** of pivoting and/or between 15 mm to 30 mm from axis **2881** of pivoting and/or between 30 mm to 60 mm from axis **2881** of pivoting and/or more than 60 mm from axis **2881** of pivoting. For example, the needle location and/or aperture **2887** may be located at a distance ranging between 0 mm to 5 mm from the side of base **642** opposite axis **2881** of pivoting and/or between 5 mm to 15 mm the side of base **642** opposite axis **2881** of pivoting and/or between 15 mm to 60 mm from the side of base **642** opposite axis **2881** of pivoting. Alternatively or additionally, needle **660** may be oriented to reduce torque during pivoting of the injector. For example, needle **660** may be pointed inward with respect to axis **2881** of pivoting. For example the needle **660** may be pointed inward with respect axis **2881** of pivoting at an angle **2830** to base **642** ranging for example between 99.9 and 99 degrees and/or between 99 and 98 degrees and/or between 98 and 95 degrees and/or between 95 and 90 degrees.

### 29 Stabilizer with large needle aperture

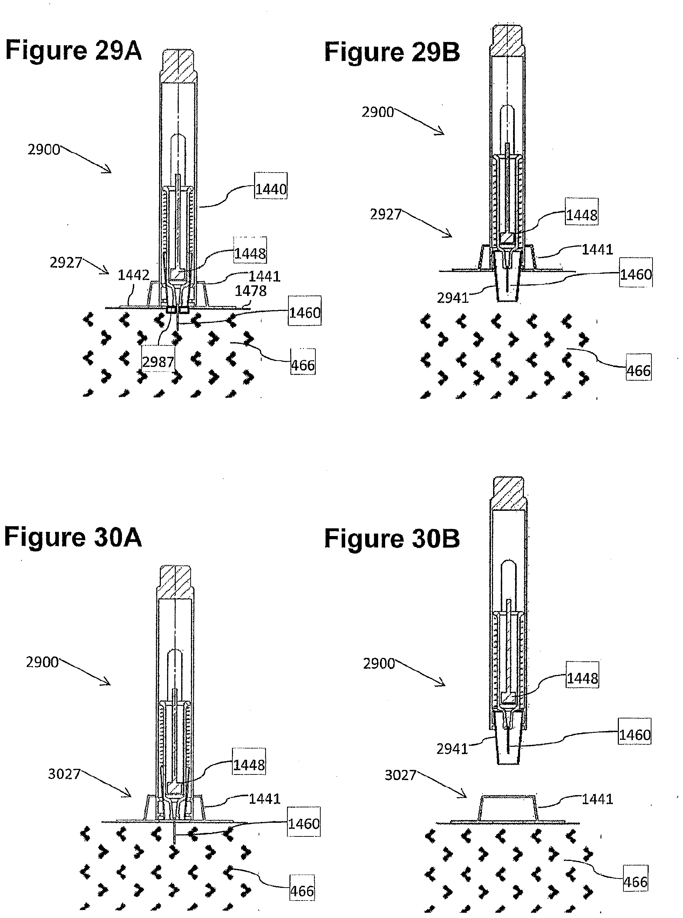

Figs. 29A-B illustrate a stabilizing adaptor **2927** having a large needle aperture **2987** in accordance with an embodiment of the current invention. Optionally stabilizing adaptor **2927** may be in the form of an open ring (optionally the ring may have an arbitrary cross section [circular and/or non-circular]) with an adhesive base **642** surrounding the cavity of the ring. Alternatively, needle aperture **2987** may be smaller than the cavity of adapter **2827.**

In some embodiments, stabilizing adapter **2927** with large needle aperture **2987** may be used with an injector **2900** having a protruding component. Aperture **2987** may be large enough for the protruding component to protrude through the aperture. For example, injector **2900** includes a needle shield **2941** that pops out to surround and/or protect needle **1460.** For example, shield **2941** may extend out of housing **1440** of injector **2900** and/or through needle aperture **2987** of adapter **2927** before injection of medicine. For example, shield **2941** collapse into of housing **1440** of injector **2900** and/or through needle aperture **2987** of adapter **2927** when injector **2900** is activated exposing needle **1460** (for example as illustrated with respect to injector **600** in Figs. 6A,B). Alternatively or additionally, shield **2941** may extend out of housing **1440** of injector **2900** and/or through needle aperture **2987** of adapter after injection of medicine, for example for protecting needle **1460** after injection (for example when injector **2900** and/or adapter **2927** are removed from skin **466** of the patient.

In some embodiments a width of the needle aperture along its smallest axis may range between 1 mm and 2 mm and/or between 2 mm and 4mm and/or between 4mm and 8mm and/or between 8mm and 16mm. Alternatively or additionally the width of the needle aperture along its smallest axis may range between 10% and 20% a width of the injector along its smallest axis and/or between 20 and 40% and/or between 40%mm and 80% and/or between 80 and 100% the width of the injector along its smallest axis.

### 30 Stabilizer axially reversible attachment to an injector

Figs. 30A-B illustrate a stabilizing adaptor **3027** which allows an injector **2900** to be removed from the adapter axially in accordance with an embodiment of the current invention. Optionally stabilizing adaptor **3027** may be in the form of an open ring (optionally the ring may have an arbitrary cross section [circular and/or non-circular]) with an adhesive base **642** surrounding the cavity of the ring. Alternatively, needle aperture **2987** may be smaller than the cavity of adapter **2827.**

In some embodiments, stabilizing adapter **3027** may be used with an injector **2900** having a protruding component. For example shield **2941** may extend out of housing **1440** of injector **2900** after injection of medicine protecting needle **1460** after injection (for example when adapter **3027** is removed from adapter **3027** and/or skin **466** of the patient).

### 31 Distributing a stabilized injector

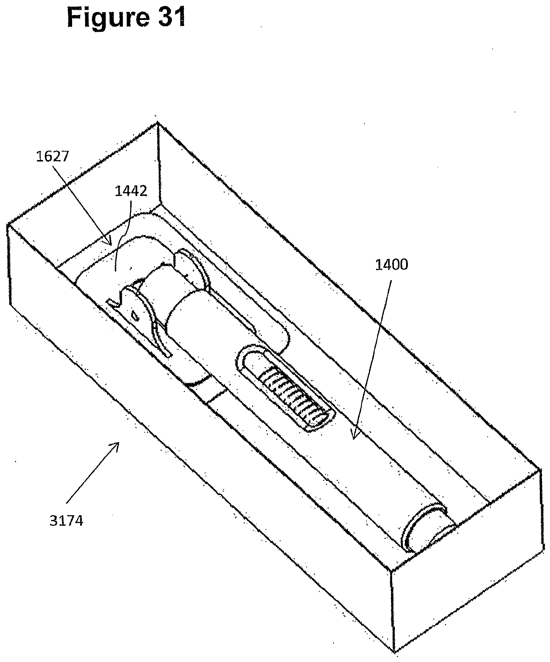

Fig. 31 illustrates pen injector **1400** retrofit with stabilizing adaptor **1627** packaged for distribution in accordance with an embodiment of the current invention. Optionally, stabilizing adaptor **1627** has a packed configuration (where injector **1400** is, for example, oriented parallel at 0 degrees to the base **1442** of adaptor **1627**) that takes up less space when packed in a packaging (for example a box **3174**) than in the active configuration (where injector **1400** is, for example, oriented at between 60 to 120 degrees to the base **1442** of adaptor **1627**). Optionally, adapter **1627** may automatically revert to the active configuration (for example by a biasing element e.g. a spring) when removed from packaging **3174.**

As used herein the term "about" refers to ± 5%. As used herein the term "approximately" when used in reference to an angle refers to ± 10 degrees.

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims. In addition, citation or identification of any reference in this application shall not be construed as an admission that such reference is available as prior art to the present invention. To the extent that section headings are used, they should not be construed as necessarily limiting.

## Claims

1. A drug delivery device comprising:
a preloaded syringe (2496) including:
a sterile hollow needle,
a barrel (2446) in fluid communication with said sterile hollow needle, a distal end of said barrel (2446) rigidly attached to said sterile hollow needle,
a plunger (2448) slidingly sealing a proximal end of said barrel (2446), said plunger (2448) sliding distally thereby discharging a contents of said barrel (2446) out of said hollow needle,
a flange (2447) at a proximal end of said barrel (2446), and
a needle cover preserving sterility of said sterile hollow needle;
a proximal housing (2440a) portion including:
a motor assembly (2476); and
a transmission (2458) converting a rotation motion of said motor assembly (2476) to a linear motion; and
a distal housing (2440b) portion including:
a channel (2488) fitting said preloaded syringe (2496), said channel (2488) including a proximal opening large enough for inserting said preloaded syringe (2496) into said channel (2488), a distal opening large enough to allow said sterile hollow needle to protrude from a distal end of said distal housing (2440b) portion, wherein the drug delivery device is adapted that, when said syringe is inserted into said channel (2488), said transmission (2458) is operationally connected to said plunger (2448) when a distal end of said proximal housing (2440a) is joined to a proximal end of said distal housing , enabling discharging of said contents of said barrel (2446) by activating said motor,
**characterized in that**, the distal housing includes an adhesive base (2442) for fastening said distal housing portion to a skin of a patient, the adhesive base (2442) including a needle aperture,
**in that** an activation device is provided including:
a handle (2394) accessible from outside of said channel (2488),
an attachment mechanism exposed to said channel (2488), such that when said preloading syringe is fully inserted into said channel (2488), said attachment mechanism attaches to said needle cover,
a coupler (1871, 1971) passing through the needle aperture in said adhesive base (2442), said coupler (1871, 1971) connecting said handle (2394) to said attachment mechanism, and
an adhesive cover (2389) for said adhesive base (2442) connected to said handle (2394) by an extender for peeling said adhesive cover (2389) from said adhesive base (2442).

2. The drug delivery device of claim 1, further comprising a mechanical connector for fastening said distal housing portion to said proximal housing (2440a) portion.

3. The drug delivery device of claim 1, wherein said motor assembly (2476) includes:
a support structure connectable to said proximal housing (2440a) portion,
a motor rigidly attached to said support structure,
a power source rigidly attached to said support structure, and
a syringe position sensor rigidly attached to said support structure, said syringe position sensor controlling an electrical connection between said power source and said motor.

4. The drug delivery device of claim 1, wherein said adhesive base (2442) is located distal to all of said needle cover.

5. The drug delivery device of claim 1, wherein said distal housing portion further includes a biasing mechanism pushing said preloaded syringe (2496) distally to a disengaged position.

6. The drug delivery device of claim 1, wherein said adhesive base (2442) has an aspect ratio of a long axis to a short axis of at least 1.5 to 1.

7. The drug delivery device of claim 1, wherein said sterile hollow needle has a principle longitudinal axis substantially parallel to an axis of said barrel (2446).

8. The drug delivery device of claim 7, further comprising a flexible adhesive skirt extending beyond at least one edge of said adhesive base (2442), said flexible adhesive skirt extending in a direction of a short axis of said adhesive base (2442) further from said at least one edge of said adhesive base (2442) than from a second edge of said adhesive base (2442).

9. The drug delivery device of claim 8, further comprising a needle aperture in said adhesive base (2442) offset from said at least one edge toward said second edge.

10. The drug delivery device of claim 8, wherein said at least one edge and said second edge are substantially perpendicular to said short axis.

11. The drug delivery device of claim 8, wherein said sterile hollow needle is oriented toward said at least one edge at an angle between 85 and 99 degrees of said adhesive base (2442).

12. A method of manufacturing an autoinjector comprising;
supplying a preloaded syringe (2496) including a sterile needle and a needle cover, a preassembled distal housing portion including an adhesive base (2442) on a distal end thereof and a channel (2488) for insertion of said preloaded syringe (2496), and a proximal housing (2440a) portion including a motor and a transmission (2458);
supplying an activation device including:
a handle (2394) accessible from outside of said channel (2488),
an attachment mechanism exposed to said channel (2488), such that when said preloading syringe is fully inserted into said channel (2488), said attachment mechanism attaches to said needle cover,
an adhesive cover (2389) for said adhesive base (2442) connected to said handle (2394) by an extender for peeling said adhesive cover (2389) from said adhesive base (2442), and
a coupler (1871, 1971) passing through a needle aperture in said adhesive base (2442) and connecting said handle (2394) to said attachment mechanism;
attaching a cover remover to said needle cover through the needle aperture in said adhesive base (2442);
connecting said cover remover to said adhesive cover (2389) such that removing said needle cover also peels said adhesive cover (2389) from said adhesive base (2442);
sliding said preloaded syringe (2496) longitudinally through an opening in a proximal end of said distal housing portion into said channel (2488);
joining, by an axial motion, a distal end of said proximal housing (2440a) portion to a proximal end of said distal housing portion thereby connecting said transmission (2458) to a plunger (2448) of said preloaded syringe (2496).

13. The method claim 12, wherein said needle cover is located entirely on one side of said adhesive base (2442).

## Patentansprüche

1. Vorrichtung zur Abgabe von Arzneimitteln, umfassend:
- eine gefüllte Spritze (2496) mit:
-- einer sterilen Hohlnadel,
-- einem Zylinder (2446) in Fluidverbindung mit der sterilen Hohlnadel, wobei das distale Ende des Zylinders (2446) an der sterilen Hohlnadel starr befestigt ist,
-- einem Kolben (2448), der das proximale Ende des Zylinders (2446) gleitfähig verschließt, wobei der Kolben (2448) beim Gleiten nach distal den Inhalt des Zylinders (2446) aus der Hohlnadel ausstößt,
-- einem Flansch (2447) am proximalen Ende des Zylinders (2446) und
-- einer die Sterilität der sterilen Hohlnadel bewahrende Nadelkappe,
- einen proximalen Gehäuseabschnitt (2440a) mit:
-- einer Motoranordnung (2476) und
-- einem Getriebe (2458), welches die Drehbewegung der Motoranordnung (2476) in eine Linearbewegung umwandelt, und
- einen distalen Gehäuseabschnitt (2440b) mit:
--einem zur gefüllten Spritze (2496) passenden Kanal (2488), wobei der Kanal (2488) eine proximale Öffnung, die zum Einsetzen der gefüllten Spritze (2496) in den Kanal (2488) groß genug ist, eine distale Öffnung, welche groß genug ist, um die sterile Hohlnadel aus dem distalen Ende des distalen Gehäuseabschnitts (2440b) hervor stehen zu lassen, umfasst, wobei die Vorrichtung zur Abgabe von Arzneimitteln so eingerichtet ist, dass das Getriebe (2458) bei in den Kanal (2488) eingesetzter Spritze betriebsfähig mit dem Kolben (2448) verbunden ist, wenn das distale Ende des proximalen Gehäuses (2440a) mit dem proximalen Ende des distalen Gehäuses verbunden ist, wodurch der Inhalt des Zylinders (2446) durch Aktivieren des Motors ausgestoßen werden kann,
- **dadurch gekennzeichnet, dass** das distale Gehäuse zum Befestigen des distalen Gehäuseabschnitts an der Haut eines Patienten eine selbstklebende Grundplatte (2442) umfasst, wobei die selbstklebende Grundplatte (2442) eine Öffnung für die Nadel umfasst,
- dass eine Aktivierungsvorrichtung vorgesehen ist, umfassend:
-- einen von außerhalb des Kanals (2488) zugänglichen Griff (2394),
-- einen zum Kanal (2488) offenen Haftmechanismus, der Art, dass der Haftmechanismus an der Nadelkappe haftet, wenn die gefüllte Spritze vollständig in den Kanal (2488) eingesetzt ist,
-- eine durch die Nadelöffnung in der selbstklebenden Grundplatte (2442) hindurchtretende Kupplung (1871, 1971), wobei diese Kupplung (1871, 1971) den Griff (2394) mit dem Haftmechanismus verbindet und
-- eine mit dem Griff (2394) durch eine Verlängerung verbundene selbstklebende Abdeckung (2389) für die selbstklebende Grundplatte (2442) zum Abziehen der selbstklebenden Abdeckung (2389) von der selbstklebenden Grundplatte (2442).

2. Vorrichtung zur Abgabe von Arzneimitteln nach Anspruch 1, ferner einen mechanischen Verbinder zum Befestigen des distalen Gehäuseabschnitts an dem proximalen Gehäuseabschnitt (2440a) umfassend.

3. Vorrichtung zur Abgabe von Arzneimitteln nach Anspruch 1, wobei die Motoranordnung (2476) umfasst:
- eine mit dem proximalen Gehäuseabschnitt (2440a) verbindbare Halterung,
- einen starr an der Halterung befestigten Motor,
- eine starr an der Halterung befestigte Stromquelle und
- einen starr an der Halterung befestigten Spritzenlagesensor, wobei der Spritzenlagesensor die elektrische Verbindung zwischen der Stromquelle und dem Motor steuert.

4. Vorrichtung zur Abgabe von Arzneimitteln nach Anspruch 1, wobei die selbstklebende Grundplatte (2442) distal zur gesamten Nadelkappe angebracht ist.

5. Vorrichtung zur Abgabe von Arzneimitteln nach Anspruch 1, wobei der distale Gehäuseabschnitt ferner einen Federmechanismus umfasst, welcher die gefüllte Spritze (2496) distal in eine abgekoppelte Stellung drückt.

6. Vorrichtung zur Abgabe von Arzneimitteln nach Anspruch 1, wobei die selbstklebende Grundplatte (2442) ein Aspektverhältnis der langen Achse zur kurzen Achse von mindestens 1,5 zu 1 aufweist.

7. Vorrichtung zur Abgabe von Arzneimitteln nach Anspruch 1, wobei die sterile Hohlnadel eine im Wesentlichen zur Achse des Zylinders (2446) parallele Hauptlängsachse aufweist.

8. Vorrichtung zur Abgabe von Arzneimitteln nach Anspruch 7, ferner eine über mindestens einen Rand der selbstklebenden Grundplatte (2442) hinausgehende flexible selbstklebende Schürze aufweisend, wobei die flexible selbstklebende Schürze sich in Richtung der kurzen Achse der selbstklebenden Grundplatte (2442) weiter weg vom wenigstens einen Rand der selbstklebenden Grundplatte (2442) erstreckt als vom zweiten Rand der selbstklebenden Grundplatte (2442).

9. Vorrichtung zur Abgabe von Arzneimitteln nach Anspruch 8, ferner eine vom mindestens einen Rand in Richtung auf den zweiten Rand versetzte Nadelöffnung in der selbstklebenden Grundplatte (2442) aufweisend.,

10. Vorrichtung zur Abgabe von Arzneimitteln nach Anspruch 8 wobei der mindestens eine Rand und der zweite Rand im Wesentlichen senkrecht zur kurzen Achse verlaufen.

11. Vorrichtung zur Abgabe von Arzneimitteln nach Anspruch 8, wobei die sterile Hohlnadel gegen den mindestens einen Rand der selbstklebenden Grundplatte (2442) in einem Winkel zwischen 85 und 99° ausgerichtet ist.

12. Verfahren zur Herstellung eines Autoinjektors, umfassend
- Bereitstellen einer gefüllten Spritze (2496) mit einer sterilen Nadel und einer Nadelkappe, einem vormontierten distalen Gehäuseabschnitt mit einer selbstklebenden Grundplatte (2442) an seinem distalen Ende und einem Kanal (2488) zum Einsetzen der gefüllten Spritze (2496), und mit einem proximalen Gehäuseabschnitt (2440a) mit einem Motor und einem Getriebe (2458),
- Bereitstellen einer Aktivierungsvorrichtung, umfassend:
-- einen von außerhalb des Kanals (2488) zugänglichen Griff (2394),
-- einen zum Kanal (2488) offenen Haftmechanismus, der Art, dass der Haftmechanismus an der Nadelkappe haftet, wenn die gefüllte Spritze vollständig in den Kanal (2488) eingesetzt ist,
-- eine mit dem Griff (2394) durch eine Verlängerung verbundene selbstklebende Abdeckung (2389) für die selbstklebende Grundplatte (2442) zum Abziehen der selbstklebenden Abdeckung (2389) von der selbstklebenden Grundplatte (2442) und
-- eine durch die Nadelöffnung in der selbstklebenden Grundplatte (2442) hindurchtretende Kupplung (1871, 1971) wobei diese Kupplung (1871, 1971) den Griff (2394) mit dem Haftmechanismus verbindet,
- Anheften eines Kappenabziehers an die Nadelkappe durch die Nadelöffnung in der selbstklebenden Grundplatte (2442),
- Verbinden des Kappenabziehers mit der selbstklebenden Abdeckung (2389), sodass das Abziehen der Nadelkappe auch die selbstklebende Abdeckung (2389) von der selbstklebenden Grundplatte (2442) abzieht,
- Schieben der gefüllten Spritze (2496) längs durch die Öffnung im proximalen Ende des distalen Gehäuseabschnitts in den Kanal (2488),
- Verbinden des distalen Endes des proximalen Gehäuseabschnitts (2440a) mit dem proximalen Ende des distalen Gehäuseabschnitts durch eine axiale Bewegung, wobei das Getriebe (2458) mit dem Kolben (2448) der gefüllten Spritze (2496) verbunden wird.

13. Verfahren nach Anspruch 12, wobei sich die Nadelkappe vollständig auf einer Seite der selbstklebenden Grundplatte befindet (2442).

## Revendications

1. Dispositif d'administration de médicament comprenant:
une seringue préchargée (2496) comprenant:
une aiguille creuse stérile,
un cylindre (2446) en communication fluidique avec ladite aiguille creuse stérile, une extrémité distale dudit cylindre (2446) fixée rigidement à ladite aiguille creuse stérile,
un piston (2448) scellant de manière coulissante une extrémité proximale dudit cylindre (2446), ledit piston (2448) coulissant distalement, déchargeant ainsi un contenu dudit cylindre (2446) hors de ladite aiguille creuse,
une bride (2447) à une extrémité proximale dudit cylindre (2446), et
un couvre-aiguille préservant la stérilité de ladite aiguille creuse stérile; une partie proximale de logement (2440a) comprenant:
un ensemble moteur (2476); et
une transmission (2458) convertissant un mouvement de rotation dudit ensemble moteur (2476) en un mouvement linéaire; et
une partie de logement distal (2440b) comprenant:
un canal (2488) adapté à ladite seringue préchargée (2496), ledit canal (2488) comprenant une ouverture proximale suffisamment grande pour insérer ladite seringue préchargée (2496) dans ledit canal (2488), une ouverture distale suffisamment grande pour permettre à ladite aiguille creuse stérile de faire saillie depuis une extrémité distale de ladite partie de logement distal (2440b), le dispositif d'administration de médicament étant adapté de telle sorte que, lorsque ladite seringue est insérée dans ledit canal (2488), ladite transmission (2458) est connectée de manière opérationnelle audit piston (2448) lorsqu'une extrémité distale dudit logement proximal (2440a) est reliée à une extrémité proximale dudit logement distal, permettant la décharge dudit contenu dudit cylindre (2446) par activation dudit moteur,
**caractérisé en ce que** le logement distal comprend une base adhésive (2442) pour fixer ladite partie de logement distal à une peau d'un patient, la base adhésive (2442) comprenant une ouverture pour aiguille,
**en ce qu'**un dispositif d'activation est prévu comprenant:
une poignée (2394) accessible de l'extérieur dudit canal (2488), un mécanisme de fixation exposé audit canal (2488), de telle sorte que lorsque ladite seringue de précharge est complètement insérée dans ledit canal (2488), ledit mécanisme de fixation se fixe sur ledit couvre-aiguille,
un coupleur (1871, 1971) traversant l'ouverture de l'aiguille dans ladite base adhésive (2442), ledit coupleur (1871, 1971) reliant ladite poignée (2394) audit mécanisme de fixation, et
un couvercle adhésif (2389) pour ladite base adhésive (2442) relié à ladite poignée (2394) par un extenseur pour décoller ledit couvercle adhésif (2389) de ladite base adhésive (2442).

2. Dispositif d'administration de médicament selon la revendication 1, comprenant en outre un connecteur mécanique pour fixer ladite partie distale de logement à ladite partie proximale de logement (2440a).

3. Dispositif d'administration de médicament selon la revendication 1, dans lequel ledit ensemble moteur (2476) comprend:
une structure de support pouvant être connectée à ladite partie de logement proximal (2440a),
un moteur fixé rigidement à ladite structure de support,
une source d'énergie fixée rigidement à ladite structure de support, et
un capteur de position de seringue fixé rigidement à ladite structure de support, ledit capteur de position de seringue commandant une connexion électrique entre ladite source d'énergie et ledit moteur.

4. Dispositif d'administration de médicament selon la revendication 1, dans lequel ladite base adhésive (2442) est située en distal par rapport à tout ledit couvre-aiguille.

5. Dispositif d'administration de médicament selon la revendication 1, dans lequel ladite partie de logement distale comprend en outre un mécanisme de sollicitation poussant ladite seringue préchargée (2496) distalement dans une position désengagée.

6. Dispositif d'administration de médicament selon la revendication 1, dans lequel ladite base adhésive (2442) a un rapport d'aspect d'un axe long à un axe court d'au moins 1,5 à 1.

7. Dispositif d'administration de médicament selon la revendication 1, dans lequel ladite aiguille creuse stérile a un axe longitudinal principal sensiblement parallèle à un axe dudit cylindre (2446).

8. Dispositif d'administration de médicament selon la revendication 7, comprenant en outre une jupe adhésive flexible s'étendant au-delà d'au moins un bord de ladite base adhésive (2442), ladite jupe adhésive flexible s'étendant dans une direction d'un axe court de ladite base adhésive (2442) plus éloignée dudit au moins un bord de ladite base adhésive (2442) que du second bord de ladite base adhésive (2442).

9. Dispositif d'administration de médicament selon la revendication 8, comprenant en outre une ouverture d'aiguille dans ladite base adhésive (2442) décalée dudit au moins un bord vers ledit second bord.

10. Dispositif d'administration de médicament selon la revendication 8, dans lequel ledit au moins un bord et ledit second bord sont sensiblement parallèles audit axe court.

11. Dispositif d'administration de médicament selon la revendication 8, dans lequel ladite aiguille creuse stérile est orientée vers ledit au moins un bord selon un angle compris entre 85 et 99 degrés de ladite base adhésive (2442).

12. Procédé de fabrication d'un auto-injecteur comprenant :
fournir une seringue préchargée (2496) comprenant une aiguille stérile et un protecteur d'aiguille, une partie de logement distal préassemblée comprenant une base adhésive (2442) sur une extrémité distale de celle-ci et un canal (2488) pour insérer ladite seringue préchargée (2496), et une partie de logement proximal (2440a) comprenant un moteur et une transmission (2458);
fournir un dispositif d'activation comprenant:
une poignée (2394) accessible de l'extérieur dudit canal (2488),
un mécanisme de fixation exposé audit canal (2488), tel que lorsque ladite seringue de précharge est complètement insérée dans ledit canal (2488),
ledit mécanisme de fixation se fixe sur ledit protecteur d'aiguille,
un couvercle adhésif (2389) pour ladite base adhésive (2442) relié à ladite poignée (2394) par un extenseur pour décoller ledit couvercle adhésif (2389) de ladite base adhésive (2442), et
un coupleur (1871, 1971) traversant une ouverture d'aiguille dans ladite base adhésive (2442) et reliant ladite poignée (2394) audit mécanisme de fixation;
fixer un enlève-couvercle sur ledit protecteur d'aiguille à travers l'ouverture d'aiguille dans ladite base adhésive (2442) ;
connecter ledit enlève-couvercle audit couvercle adhésif (2389) de telle sorte que le retrait dudit protecteur d'aiguille décolle également ledit couvercle adhésif (2389) de ladite base adhésive (2442);
glisser ladite seringue préchargée (2496) longitudinalement à travers une ouverture dans une extrémité proximale de ladite partie de logement distal dans ledit canal (2488) ;
joindre par un mouvement axial, une extrémité distale de ladite partie de logement proximal (2440a) à une extrémité proximale de ladite partie de logement distal reliant ainsi ladite transmission (2458) à un piston (2448) de ladite seringue préchargée (2496).

13. Procédé selon la revendication 12, dans lequel ledit protecteur d'aiguille est situé entièrement sur un côté de ladite base adhésive (2442).
